# FASCICULE DE BREVET EUROPEEN

(11) **EP 1 972 327 B1**
(45) Date de publication et mention de la délivrance du brevet: **07.04.2010**
(21) Numéro de dépôt: 08152085.0
(22) Date de dépôt: 28.02.2008
(51) Int. Cl.: A61K 8/49, A61Q 5/10, A61Q 5/06

(54) **Utilisation pour la coloration des fibres kératiniques d'une composition comprenant un composé halochromique et / ou le colorant correspondant à ce composé**
Verwendung einer Zusammensetzung für die Färbung von Keratinfasern, die einen halochromen Bestandteil und/oder den Farbstoff enthält, der diesem Bestandteil entspricht
Use of a composition for dyeing keratinous fibres comprising a halochromic compound and/or the dye corresponding to this compound

(30) Priorité: 20.03.2007 FR 0753923
(43) Date de publication de la demande: 24.09.2008
(73) Titulaire: L'Oréal, 75008 Paris (FR)
(72) Inventeur: Guerin, Frédéric, 75010 Paris (FR); Lagrange, Alain, 77700 Coupvray (FR)
(74) Mandataire: Prevel, Estelle Nicole

(56) Documents cités:
- WO-A-00/76466
- FR-A- 2 862 531
- DATABASE CA [Online] CHEMICAL ABSTRACTS SERVICE, COLUMBUS, OHIO, US; 16 décembre 2001 (2001-12-16), SEN, RAJENDRA N. ET AL: "The condensation of esters with resorcinol, dimethylaniline and diethyl-m-aminophenol" XP002459317 extrait de STN Database accession no. 24:9972 -& J. INDIAN CHEM. SOC. , 6, 557-63, 1929, XP009092395
- DATABASE CA [Online] CHEMICAL ABSTRACTS SERVICE, COLUMBUS, OHIO, US; 16 septembre 1989 (1989-09-16), KUROSAWA, KUNISAKU: "Thermosensitively color-changing nail lacquers" XP002459318 extrait de STN Database accession no. 111:102542 & JP 63 301806 A (MORIMURA BROS., INC., JAPAN; THREETECK DAVIS K. K.) 8 décembre 1988 (1988-12-08)
- GUNZENHAUSER, SIGMUND ET AL: "Halochromic molecules. Substituted 6,11- dihydrospiro[[1]benzopyrano[4,3-b]indole-6 ,9'-(9'H)-xanthene]-2',6'- diamines and their aza analogs: new chromogens for black images" HELVETICA CHIMICA ACTA , 73(2), 359-79 CODEN: HCACAV; ISSN: 0018-019X, 1990, XP002459316

## Description

La présente invention a pour objet l'utilisation pour la coloration des fibres kératiniques, et en particulier des fibres kératiniques humaines telles que les cheveux, d'une composition comprenant au moins un composé halochromique convenablement sélectionné et / ou le colorant correspondant à ce composé.

Il est connu de teindre les fibres kératiniques, et en particulier les fibres kératiniques humaines telles que les cheveux, avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques tels que des dérivés de diaminopyrazole. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est aussi connu de teindre les fibres kératiniques, et en particulier les fibres kératiniques humaines telles que les cheveux, avec des compositions tinctoriales contenant des colorants directs. Ces colorants sont des molécules colorées et colorantes ayant une affinité pour les fibres kératiniques. Ils sont appliqués sur les fibres kératiniques pendant un temps nécessaire à l'obtention de la coloration désirée, puis rincés.

Les colorants classiques qui sont utilisés sont en particulier des colorants du type nitré benzénique, anthraquinonique, nitropyridinique, azoïque, azoïque cationique, xanthénique, acridinique azinique, triarylméthane, benzénique nitré ou des colorants naturels.

L'utilisation des colorants directs est très répandue car ils présentent certains avantages par rapport aux précurseurs de colorants d'oxydation et, notamment, une diminution des risques potentiels d'allergie, l'absence de sensibilisation du cheveu due au processus oxydatif et des temps de pose moins longs.

Les colorations obtenues sont cependant temporaires ou semi-permanentes, car la nature des interactions qui lient les colorants directs à la fibre kératinique, et leur désorption de la surface et / ou du coeur de la fibre sont responsables de leur faible puissance tinctoriale et de leur mauvaise tenue aux lavages, aux intempéries, ou à la transpiration. Ces colorants directs sont en outre généralement sensibles à la lumière du fait de la faible résistance du chromophore vis-à-vis des attaques photochimiques et conduisent dans le temps à un affadissement de la coloration des cheveux.

L'utilisateur peut choisir le mode de coloration lui permettant d'obtenir des nuances adaptées à ses besoins en terme de reflets et de ténacité. Cependant, pour effacer les couleurs ainsi obtenues, il doit utiliser des compositions de décoloration comprenant un ou plusieurs agents oxydants ou des réducteurs de type réductone, thiol ou sulfite. Parmi les agents oxydants classiquement utilisés, on peut citer le peroxyde d'hydrogène ou des composés susceptibles de produire du peroxyde d'hydrogène par hydrolyse, comme par exemple le peroxyde d'urée ou les persels tels que les perborates, les persulfates, les percarbonates. L'utilisation de ces agents oxydants présente l'inconvénient d'entraîner une dégradation non négligeable des fibres kératiniques et d'altérer leurs propriétés cosmétiques. Les cheveux ont tendance à devenir rèches, plus difficilement démêlables et plus fragiles. Les réducteurs de type réductone, thiol ou sulfite présentent quant à eux l'inconvénient de ne pas convenir à tous les types de colorants. Si ils permettent d'effacer efficacement et sans endommager la fibre kératinique les colorants azoïques, ils sont inopérants avec les colorants dérivés des anthraquinones.

Par ailleurs, l'utilisation pour la coloration des fibres kératiniques de composés halochromiques particuliers est connue de l'art antérieur, notamment de la demande FR 2 862 530 qui décrit des composés comportant un cycle pouvant subir une ouverture en formant un groupement acide, de la demande FR 2 862 531 qui décrit des composés comportant un cycle lactone, et de la demande FR 2 862 532 qui décrit des dimères de composés comportant un cycle lactone. Ces composés permettent d'obtenir des compositions pour la coloration des fibres kératiniques qui permettent de pallier partiellement les inconvénients indiqués ci-dessus mais qui sont encore insuffisamment performantes.

Le but de la présente invention est de fournir de nouveaux composés halochromiques pour la coloration des fibres kératiniques qui permettent d'obtenir des compositions améliorées pour la coloration des fibres kératiniques notamment vis-à-vis des inconvénients mentionnés ci-dessus. En particulier, le but de la présente invention est de fournir de nouveaux composés halochromiques pour la coloration des fibres kératiniques permettant d'obtenir rapidement des colorations avec des reflets intenses et tenaces qui peuvent s'effacer grâce à un agent extérieur qui n'altère pas les fibres kératiniques comme un agent de pH ou la chaleur par exemple.

Ce but est atteint avec la présente invention qui a pour objet l'utilisation pour la coloration des fibres kératiniques, et en particulier des fibres kératiniques humaines telles que les cheveux, d'une composition comprenant, dans un milieu approprié pour la teinture, au moins un composé choisi parmi les composés de formule (I) comportant un groupement cyclique G incluant un cycle H susceptible de s'ouvrir, les colorants correspondant aux composés de formule (I) dont le cycle H est ouvert et leurs sels d'addition : 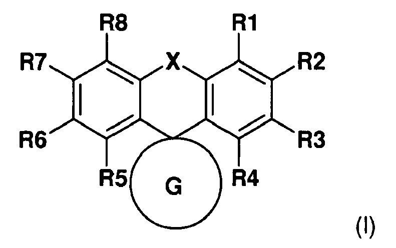 dans laquelle :
- **R₁, R₂, R₃, R₄, R₅, R₆, R₇** et **R₈** représentent, indépendamment l'un de l'autre :
   - un atome d'hydrogène ;
   - un radical halogéno ;
   - un radical hydroxyle ;
   - un radical nitro ;
   - un radical amino ;
   - un radical carboxy ;
   - un radical aminocarbonyle ;
   - un radical cyano ;
   - un radical issu d'une chaîne hydrocarbonée comportant de 1 à 100 atomes de carbone, de préférence de 1 à 50, linéaire ou ramifiée, acyclique ou mono ou polycyclique, condensée ou non condensée, saturée ou insaturée, aromatique ou non aromatique, pouvant être interrompue par un ou plusieurs hétéroatomes choisis parmi les atomes d'azote, d'oxygène et de soufre ou par un ou plusieurs groupements carbonyle, pouvant être terminée par un groupement hydrogénocarbonyle ou par un groupement contenant un ou plusieurs hétéroatomes choisis parmi les atomes d'azote, d'oxygène et de soufre, pouvant commencer par un groupement carbonyle ou par un groupement contenant un ou plusieurs hétéroatomes choisis parmi les atomes d'azote, d'oxygène et de soufre, et pouvant être substituée par un ou plusieurs groupements choisis parmi les radicaux hydroxyle, halogéno, carboxy, carboxyalkyle en C₁-C₉, cyano, amino, alcoxy en C₁-C₆ ;
- deux des radicaux **R₁, R₂, R₃, R₄, R₅, R₆, R₇** et **R₈** portés par deux atomes de carbone adjacents pouvant former ensemble et avec les atomes de carbone auxquels ils sont attachés un groupement mono ou polycarbocyclique aromatique, condensé ou non condensé, comprenant de 5 à 20 chaînons, un ou plusieurs atomes de carbone pouvant être remplacés par un atome d'oxygène, d'azote, de soufre ou de phosphore, le groupement mono ou polycarbocyclique aromatique étant non substitué ou substitué par un ou plusieurs radicaux choisis parmi un radical halogéno, hydroxyle, amino, carboxy, aryle en C₆-C₁₈, cyano ;
   les radicaux amino étant non substitués ou substitués par un ou deux radicaux, identiques ou différents, choisis parmi un radical alkyle en C₁-C₉ ; un radical hydroxyalkyle en C₁-C₉ ; un radical alcényle en C₂-C₉ ; un radical cycloalkyle en C₆-C₁₂; un radical aryle en C₆-C₁₈ lui-même éventuellement substitué par un ou plusieurs radicaux choisis parmi un radical halogéno, alkyle en C₁-C₉ ; un radical aryl(C₆-C₁₈)carbonyle ; un radical cycloalkyl(C₆-C₁₂)alkyle en C₁-C₉ ; un radical aryl(C₆-C₁₈)alkyl(C₁-C₉) ; un radical alkyl(C₁-C₉)carbonyle ; un radical alcoxy(C₁-C₉)carbonylalkyl(C₁-C₉) ; un radical α-naphtylalkyle ; un radical halogénoalkyle en C₁-C₉ ; un radical cyanoalkyle en C₁-C₉ ; un radical acyle en C₂-C₁₅ ; un radical alcoxy(C₁-C₉)carbonyle ; un radical aryloxy(C₆-C₁₈)carbonyle ; un radical aryloxy(C₆-C₁₈)alkyl(C₁-C₉)carbonyle ; un radical aryl(C₆-C₁₈)alcoxy(C₁-C₉)carbonyle ; un radical alcoxy(C₁-C₉)aryl(C₆-C₁₈)carbonyle ; un radical alcoxy(C₁-C₉)alkyl(C₁-C₉)carbonyle ; un radical dialkyl(C₁-C₉)aminocarbonyle ; un radical dialkyl(C₁-C₉)aminosulfonyle ; un radical alkyl(C₁-C₉)aryl(C₆-C₁₈)sulfonyle ; un radical alkyl(C₁-C₉)sulfonyle ; un radical dialkyl(C₁-C₉)aminoalkyle en C₁-C₉ ; un radical alcoxy(C₁-C₉)alkyle en C₁-C₉ ;
   lorsque les radicaux amino sont substitués par deux radicaux, ceux-ci peuvent former avec l'atome d'azote du radical amino un hétérocycle à 5 ou 6 chaînons comportant éventuellement un ou plusieurs hétéroatomes additionnels ;
- **X** représente une liaison directe ou un atome divalent tel qu'un atome de soufre ou d'oxygène ou un groupement sulfone SO₂ ou C(R₁₃)₂ ou NR₁₃ ;
- **G** représente un radical divalent ayant l'une des formules suivantes : 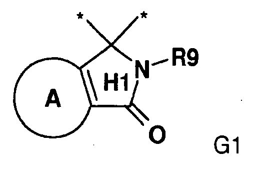 ou 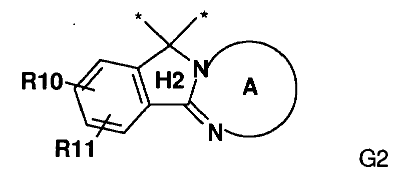 ou 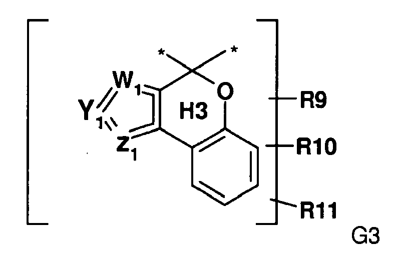 ou 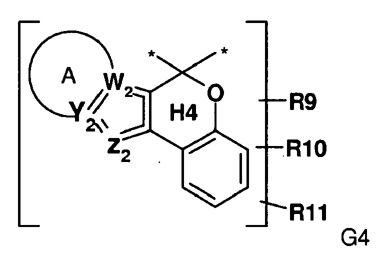 ou 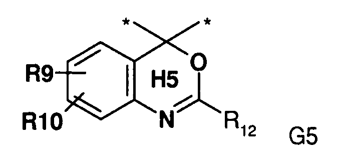 ou 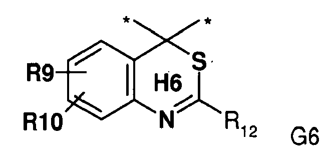 ou 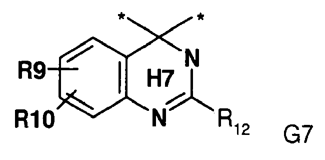
dans lesquelles :
**Y₁, W₁** et **Z₁** d'une part, et **Y₂, W₂** et **Z₂** d'autre part, représentent, indépendamment les uns des autres, un atome de carbone, un atome d'azote, un atome de soufre ou un groupement divalent CR₁₃ ou NR₁₃ ;
**R₉, R₁₀**, **R₁₁** et **R₁₃** ont les mêmes définitions que **R₁**;
**R₁₂** représente :
   - un atome d'hydrogène ;
   - un radical alkyle en C₁-C₉ ;
   - un radical amino ;
   - un radical alcoxy en C₁-C₉;
   - un radical aryle en C₆-C₁₈ non substitué ou substitué par un ou plusieurs groupements choisis parmi un atome d'hydrogène, un radical hydroxyle, alkyle en C₁-C₉, aryle en C₆-C₁₈, aryloxy en C₆-C₁₈, alcoxy en C₁-C₉, halogéno, carboxy, cyano, amino substitué ou non ;
   - un radical furanyle ;
   - un radical alkyl(C₁-C₉)thio ;
   - un radical thiényle ;
   - un radical phénylcarbonyle ;
   - un radical trifluoroalkyle ;
   - un radical diaryl(C₆-C₁₈)alkyle en C₁-C₉ ;
**A** représente un groupement aryle en C₆-C₁₈ ou un groupement hétérocyclique insaturé ou non et substitué ou non comprenant de 5 à 12 chaînons.

De préférence, les substituants de A, identiques ou différents, sont de type R₁₄, R₁₄ pouvant présenter les mêmes significations que R₁.

La présente invention a également pour objet un procédé de traitement des fibres kératiniques, et en particulier des fibres kératiniques humaines telles que les cheveux, mettant en oeuvre la composition conforme à l'invention.

La présente invention permet en particulier d'obtenir rapidement une coloration des fibres kératiniques avec des reflets intenses et tenaces pouvant être effacée puis reformée tout aussi rapidement. Le procédé d'effaçage et de reformation de la couleur peut être reproduit au moins une fois sans perte substantielle de couleur à l'aide d'un agent de pH ou en agissant sur la température.

Les composés de formule (I) comportent un cycle H pouvant subir une ouverture en formant un groupement acide en présence de protons. Ces composés sont incolores ou faiblement colorés et les composés correspondant à l'ouverture du cycle H sont des espèces colorées et colorantes. En milieu aqueux, il s'établit un équilibre entre les espèces colorées et les espèces non colorées qui dépend du pH et de la température.

Lorsque la composition comprend plusieurs composés choisis parmi les composés de formule (I), les colorants correspondant aux composés de formule (I) dont le cycle H est ouvert et leurs sels d'addition, la coloration des fibres kératiniques peut aussi être modifiée à l'aide d'un agent de pH ou en agissant sur la température.

Dans le cadre de la présente invention, les symboles * dans les formules G1 à G7 indiquent les liaisons par lesquelles le radical divalent G se rattache aux noyaux aromatiques substitués par les radicaux R₁ à R₈ dans la formule (I).

Dans le cadre de la présente invention, on entend par radical alkyle (alk) un radical linéaire ou ramifié, par exemple un radical méthyle, éthyle, n-propyle, iso-propyle, n-butyle ou ter-butyle. Un radical alcoxy est un radical alk-O-, un radical alkylthio est un radical alk-S-, un radical mono ou dialkylamino est un radical -N(alk)ₙ avec n = 1 ou 2, un radical alkylcarbonyle est un radical alk-CO-, un radical alcoxycarbonyle est un adical alk-O-CO-, un radical alkylcarbonylalkyle est un radical alk-CO-alk-, un radical alkylcarbonylamino est un radical alk-CO-NH-, dans chacune de ces définitions le radical alkyle étant tel que défini précédemment.

On entend par radical alcényle, un alkyle de 2 à 10 atomes de carbone et comprenant une ou plusieurs doubles et / ou triples liaisons, conjuguées ou non.

On entend par radical cycloalkyle un radical alkyle dans lequel les atomes de carbone forment un cycle, par exemple un radical cyclohexyle. Un radical mono ou dicycloalkylamino est un radical amino substitué par un ou deux radicaux cycloalkyle.

On entend par radical aryle (ar) un radical carboné dérivé des composés benzéniques condensés ou non condensés, par exemple phényle, anthracényle ou naphtyle. Un radical mono ou diarylamino est un radical amino substitué par un ou deux radicaux aryle. Un radical mono ou di(arylalkyl)amino est un radical amino substitué par un ou deux radicaux arylalkyle. Un radical arylalkyle est un radical alkyle substitué par un radical aryle. Un radical arylalcoxy est un radical alcoxy substitué par un radical aryle. Un radical arylcarbonyle est un radical ar-CO-, avec ar étant tel que défini précédemment.

On entend par radical hétéroaryle un radical aryle comportant un ou plusieurs hétéroatomes, par exemple un cycle pyridinique.

Un radical halogéno désigne un atome d'halogène choisi parmi le chlore, le brome, l'iode et le fluor.

Dans le cadre de la présente invention, le terme "condensé" signifie au moins deux cycles accolés présentant au moins deux atomes communs.

Un groupement mono ou polyhétérocyclique, condensé ou non condensé, aromatique ou non aromatique, comprenant de 5 à 50 chaînons, peut être par exemple un cycle thiophène, benzofurane, benzothiophène, indole, bispyridine, benzopyrane, quinoline, pyrazole, pyridine, pyrrole, furane, imidazole, benzimidazole.

Un groupement mono ou polycarbocyclique aromatique, condensé ou non condensé, comprenant de 5 à 50 chaînons, un ou plusieurs atomes de carbone pouvant être remplacés par un atome d'oxygène, d'azote, de soufre ou de phosphore peut être par exemple un cycle benzène, naphtalène, anthracène, pyridine, quinoline, thiofène, pyrimidine.

Un radical imino est un radical HN=C.

Un radical arylimino peut être par exemple un radical phénylimino.

Un radical alcoxycarbonylalkylamino est un radical amino substitué par un radical alcoxycarbonylalkyle.

Un radical α-naphtylalkylamino est un radical amino substitué par un radical α-naphtylalkyle, qui est un radical alkyle substitué par un radical α-naphtyle.

Selon un mode de réalisation particulier de l'invention, le composé de formule (I) est tel que :
- R₁, R₄, R₅ et R₈ désignent un atome d'hydrogène ;
- R₂ et R₇ désignent, indépendamment l'un de l'autre, un atome d'hydrogène ; un radical halogéno ; un radical alkyle en C₁-C₉ ; un radical alcoxy en C₁-C₉ ; un radical amino substitué ou non ;
- R₃ et R₆ désignent, indépendamment l'un de l'autre, un atome d'hydrogène ; un radical halogéno ; un radical alkyle en C₁-C₉ ; un radical amino substitué ou non ;
- X représente une liaison directe ou un atome de soufre ou d'oxygène ou un groupe SO₂ ;
- G représente un radical divalent de formule G1, G2, G3, G4, G5 ou G6 telle que définie ci-dessus.

Selon un mode de réalisation particulier de l'invention, G représente un groupement G1 ; X représente un atome d'oxygène ; R₁, R₃, R₄, R₅, R₆ et R₈ désignent un atome d'hydrogène ; R₂ et R₇ désignent, indépendamment l'un de l'autre, un radical amino substitué ou non ; R₉ désigne un radical aryle substitué ou non, un radical amino substitué ou non ; A désigne un cycle benzénique substitué ou non.

Selon un autre mode de réalisation particulier de l'invention, G représente un groupement G2 ; X représente un atome d'oxygène ; R₁, R₃, R₄, R₅, R₆, R₈, R₁₀ et R₁₁ désignent un atome d'hydrogène ; R₂ et R₇ désignent, indépendamment l'un de l'autre, un radical amino substitué ou non ; A représente un cycle benzénique substitué ou non.

Selon un autre mode de réalisation particulier de l'invention, G représente un groupement G3 ; R₁, R₃, R₄, R₅, R₆, R₈, R₁₀ et R₁₁ désignent un atome d'hydrogène ; R₂ ou R₇ désignent, indépendamment l'un de l'autre, un atome d'hydrogène, un groupement amino substitué ou non ; R₉ désigne un atome d'hydrogène, un groupement alkyle substitué ou non, un groupement alcoxy ; X représente une liaison directe ; W₁ représente un atome de soufre, un atome de carbone substitué par un atome d'hydrogène, un radical alkyle en C₁-C₄, un radical phényle, un groupement amino porteur de deux groupements alkyle en C₁-C₄, les deux radicaux formant éventuellement avec l'atome d'azote les portant, un hétérocycle saturé comprenant 5 chaînons ; Y₁ représente un atome de carbone substitué par un atome d'hydrogène, un radical alkyle en C₁-C₄, un radical phényle, un groupement amino porteur de deux groupements alkyle en C₁-C₄, les deux radicaux formant éventuellement avec l'atome d'azote les portant, un hétérocycle saturé comprenant 5 chaînons, un atome d'azote, un atome d'azote substitué par un atome d'hydrogène, un radical alkyle en C₁-C₂, un radical phényle, un groupement phénylalkyle (C₆H₅-alkyle-) dont le groupement alkyle est en C₁-C₄ ; Z₁ représente un atome d'azote, un atome d'azote substitué par un atome d'hydrogène, un radical alkyle en C₁-C₂, un radical phényle, un groupement phénylalkyle (C₆H₅-alkyle-) dont le groupement alkyle est en C₁-C₄.

Selon un autre mode de réalisation particulier de l'invention, G représente un groupement G4 ; R₁, R₄, R₅, R₈ et R₁₁ désignent un atome d'hydrogène ; R₂ ou R₇ désignent, indépendamment l'un de l'autre, un atome d'hydrogène, un atome d'halogène, un groupement alcoxy, un groupement amino substitué ou non ; R₃ et R₆ désignent, indépendamment l'un de l'autre, un atome d'hydrogène, un atome d'halogène, un groupement amino substitué ou non ; R₉ désigne un atome d'hydrogène, un groupement alkyle substitué ou non, un groupement alcoxy, un groupement aryle, un groupement amino substitué ou non ; R₁₀ désigne un atome d'hydrogène ou un radical alkyle ; A représente un cycle benzénique substitué ou non ou un cycle pyridinique substitué ou non ; X représente un atome d'oxygène ou de soufre, une liaison directe ou un groupe SO₂ ; W₂ représente un atome de carbone, un atome d'azote ; Y₂ représente un atome de carbone, un atome d'azote ; Z₂ représente un groupement CH, un atome de carbone substitué par un atome d'hydrogène, un radical alkyle en C₁-C₄, un radical phényle, un groupement amino porteur de deux groupements alkyle en C₁-C₄, les deux radicaux formant éventuellement avec l'atome d'azote les portant, un hétérocycle saturé comprenant 5 chaînons, un groupement NH, un atome d'azote, un atome d'azote substitué par un atome d'hydrogène, un radical alkyle en C₁-C₂, un radical phényle, un groupement phénylalkyle (C₆H₅-alkyle-) dont le groupement alkyle est en C₁-C₄.

Selon un autre mode de réalisation particulier de l'invention, G représente un groupement G5, X représente un atome d'oxygène ou une liaison directe ; R₁, R₄, R₅, R₈ et R₁₀ désignent un atome d'hydrogène ; R₂ et R₇ désignent, indépendamment l'un de l'autre, un radical alkyle, un radical alcoxy, un radical amino substitué ou non ; R₃ et R₆ désignent, indépendamment l'un de l'autre, un atome d'hydrogène, un radical alkyle, un radical amino substitué ou non ; R₉ désigne un atome d'hydrogène, un radical amino substitué ou non ; R₁₂ désigne un radical alkyle substitué ou non, un radical aryle substitué ou non, un radical thiényle, un radical furanyle.

Selon un autre mode de réalisation particulier de l'invention, G représente un groupement G6 ; X représente une liaison directe ou un atome d'oxygène ; R₁, R₃, R₄, R₅, R₆, R₈ et R₁₀ désignent un atome d'hydrogène, R₂, R₉ et R₇ désignent, indépendamment l'un de l'autre, un groupement amino substitué ou non ; R₁₂ désigne un radical alkyle.

A titre d'exemples de composés de formule (I), on peut citer les composés suivants :

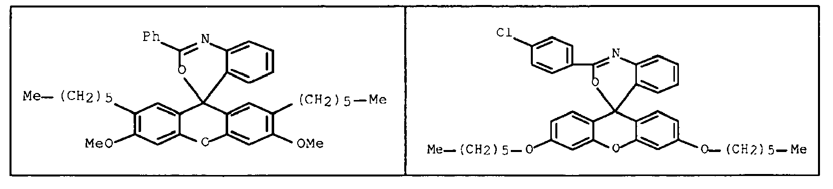

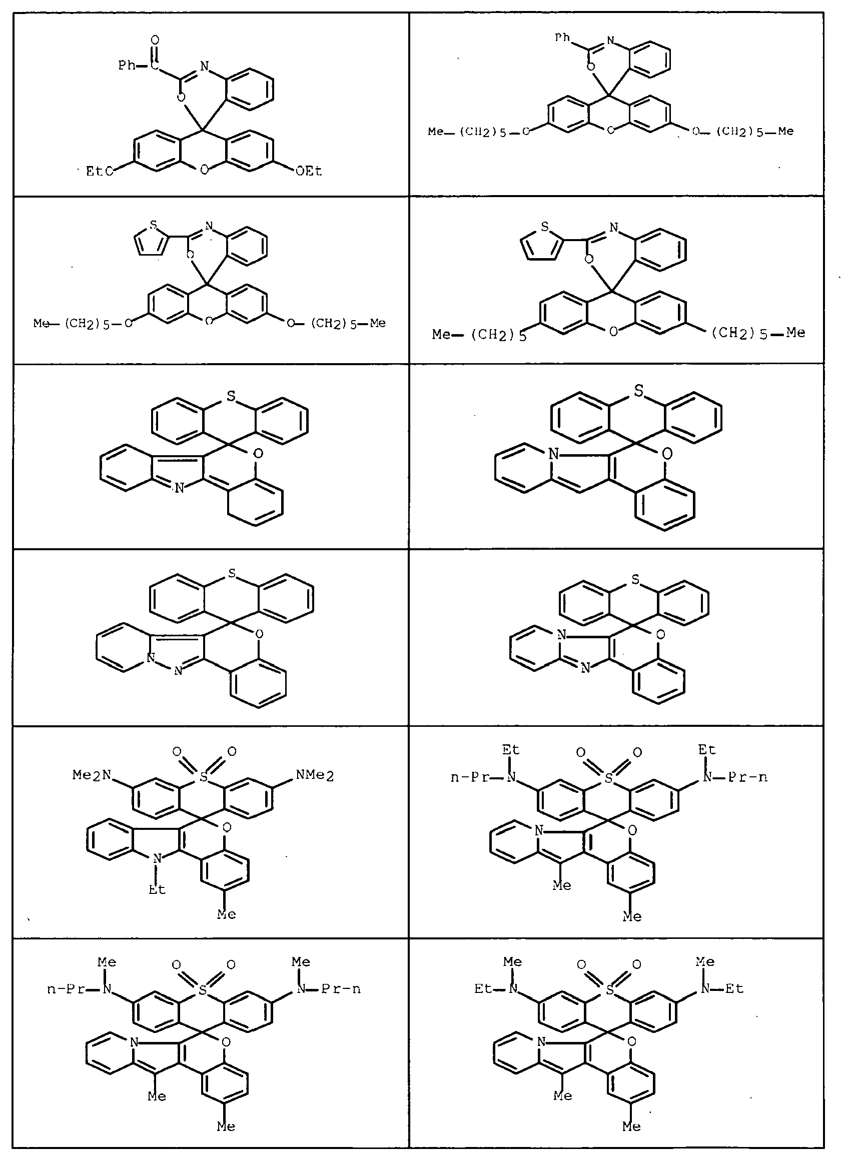

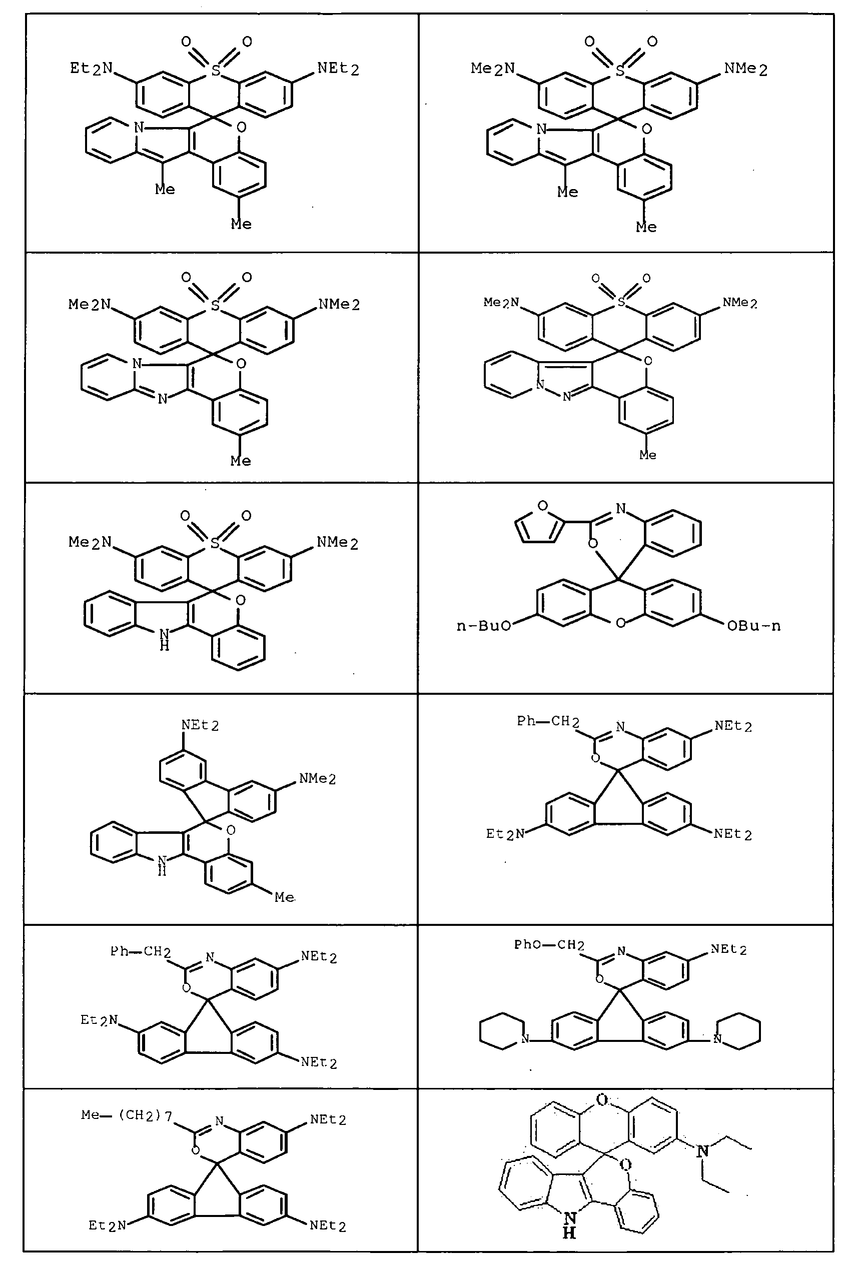

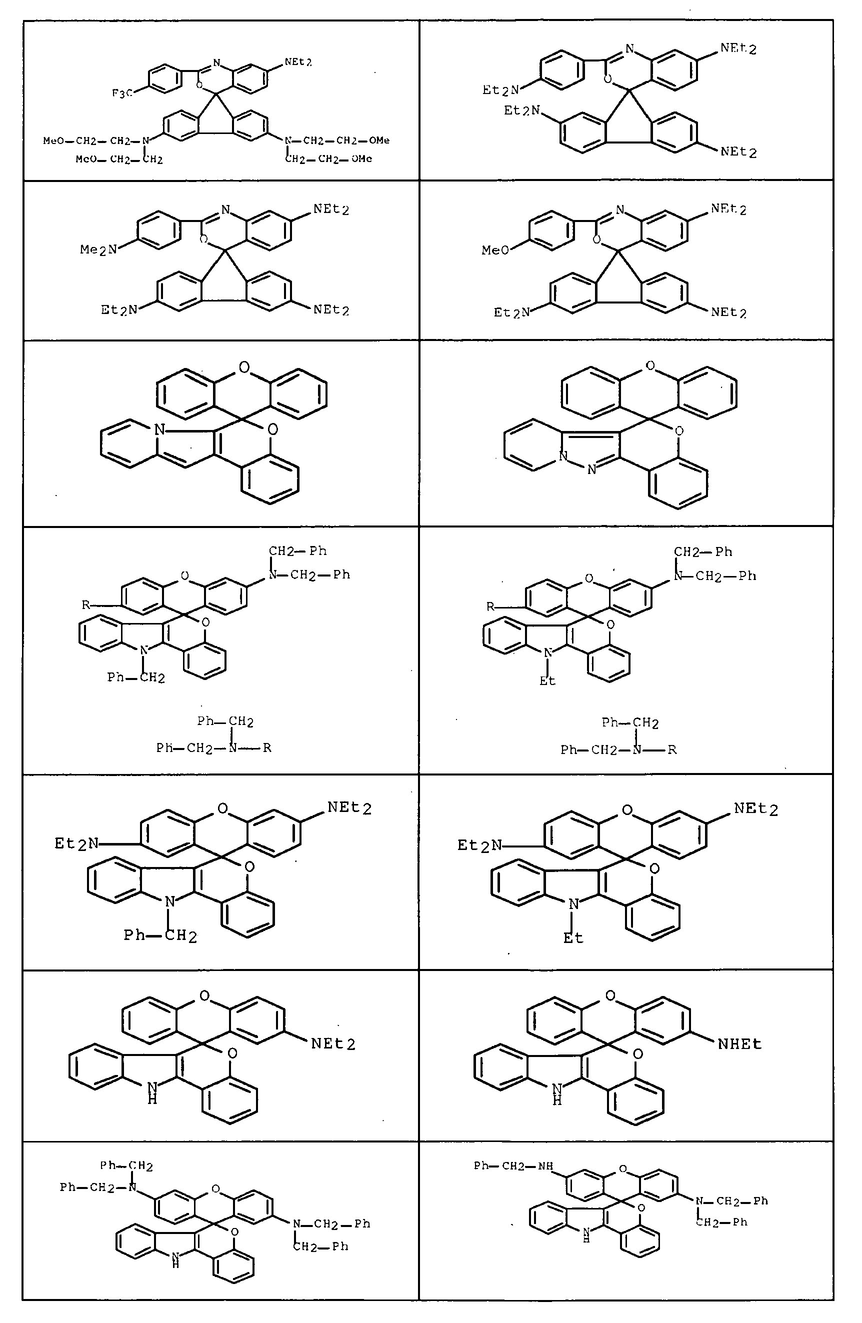

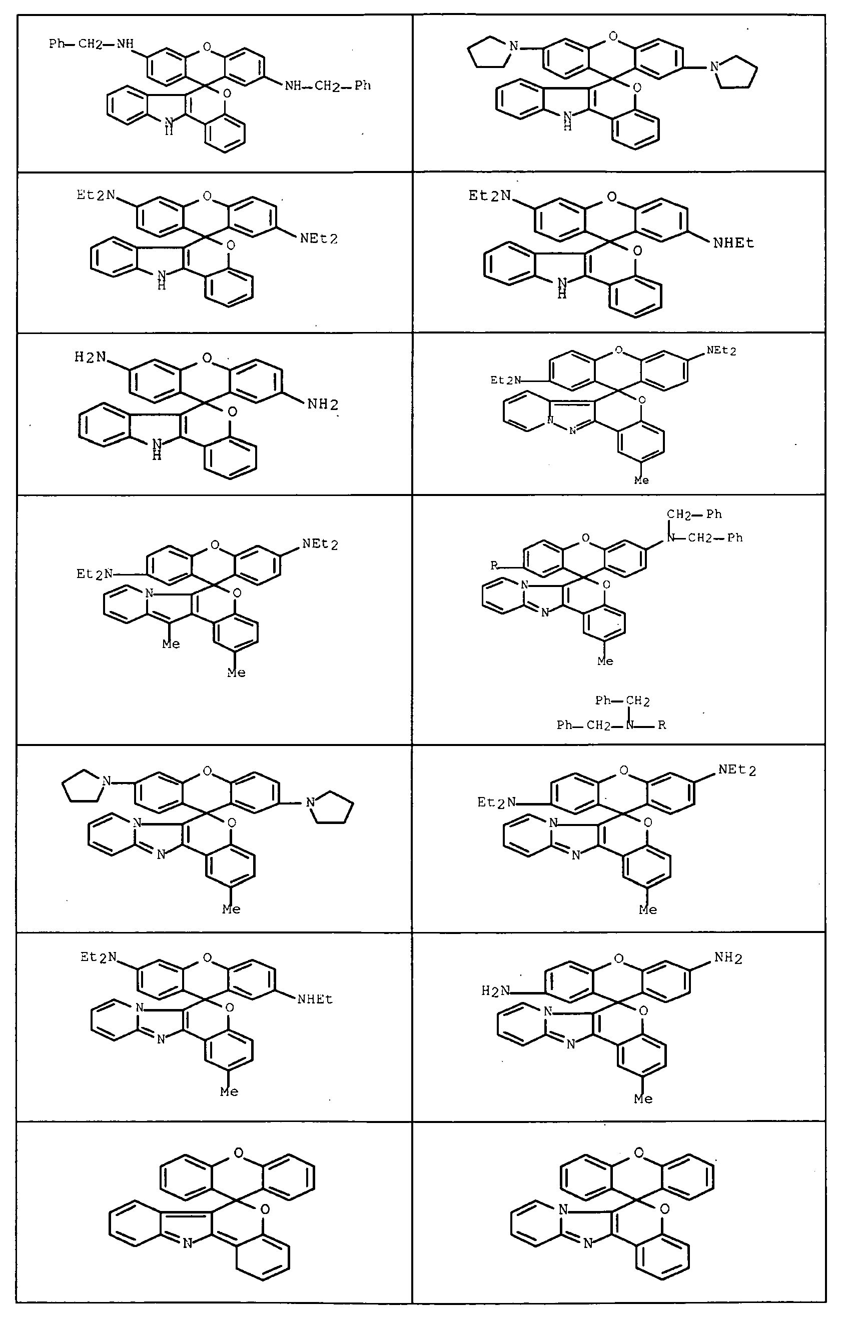

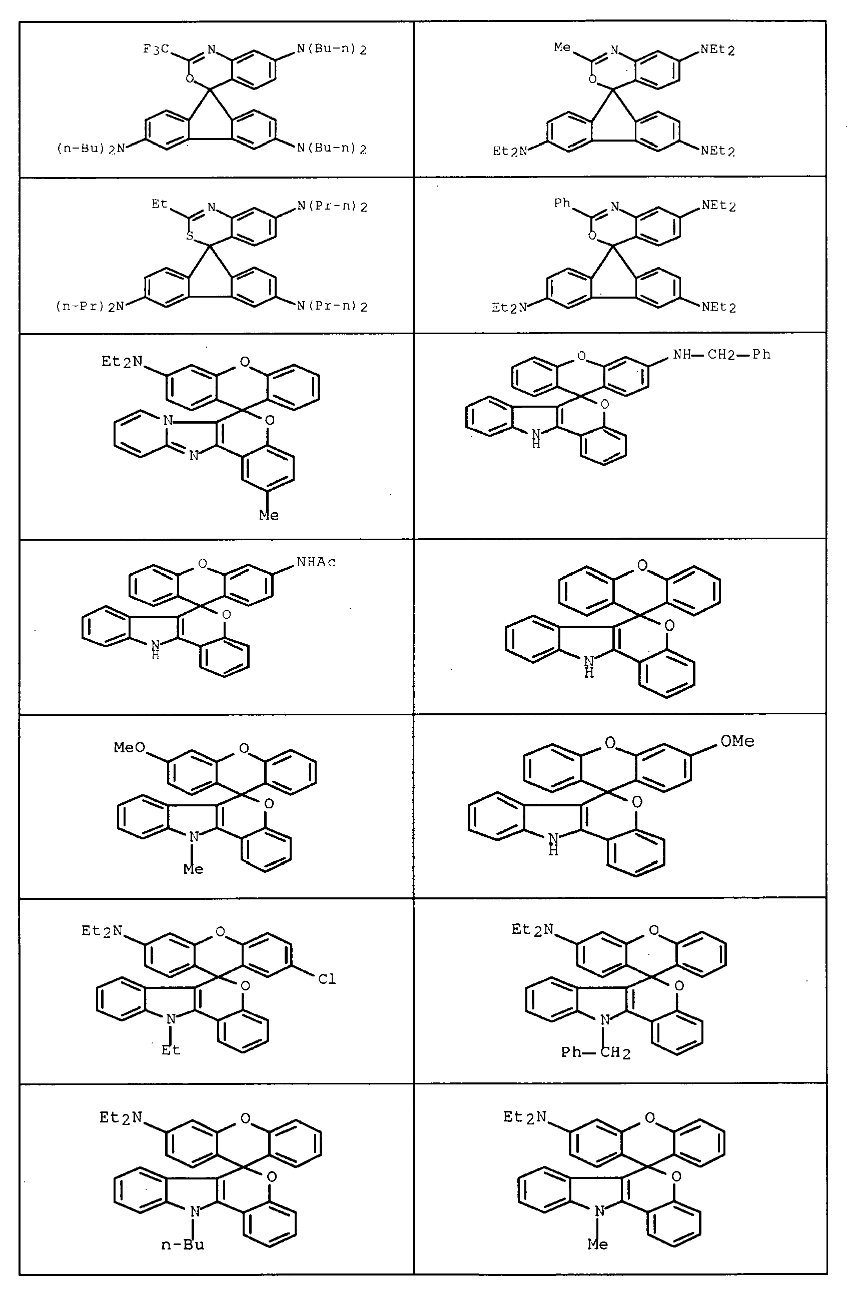

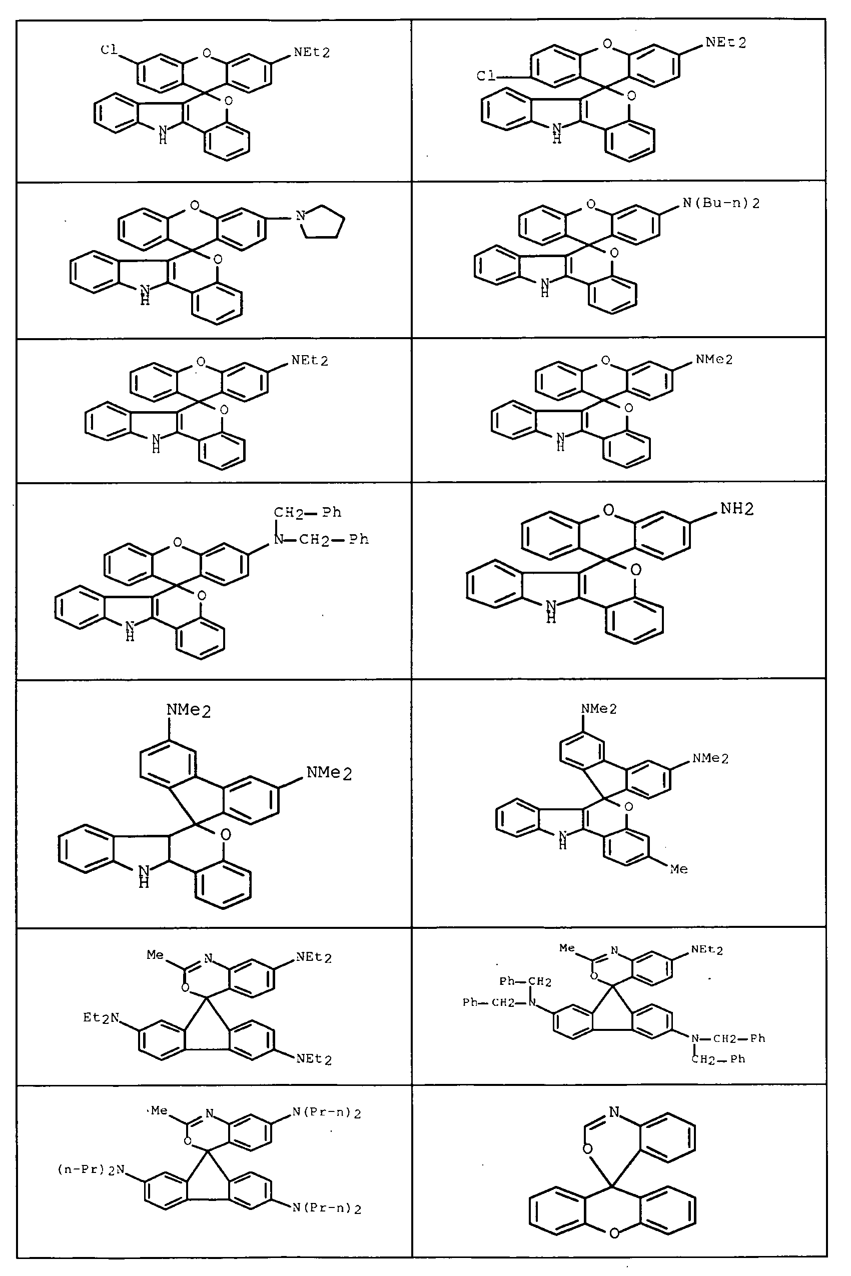

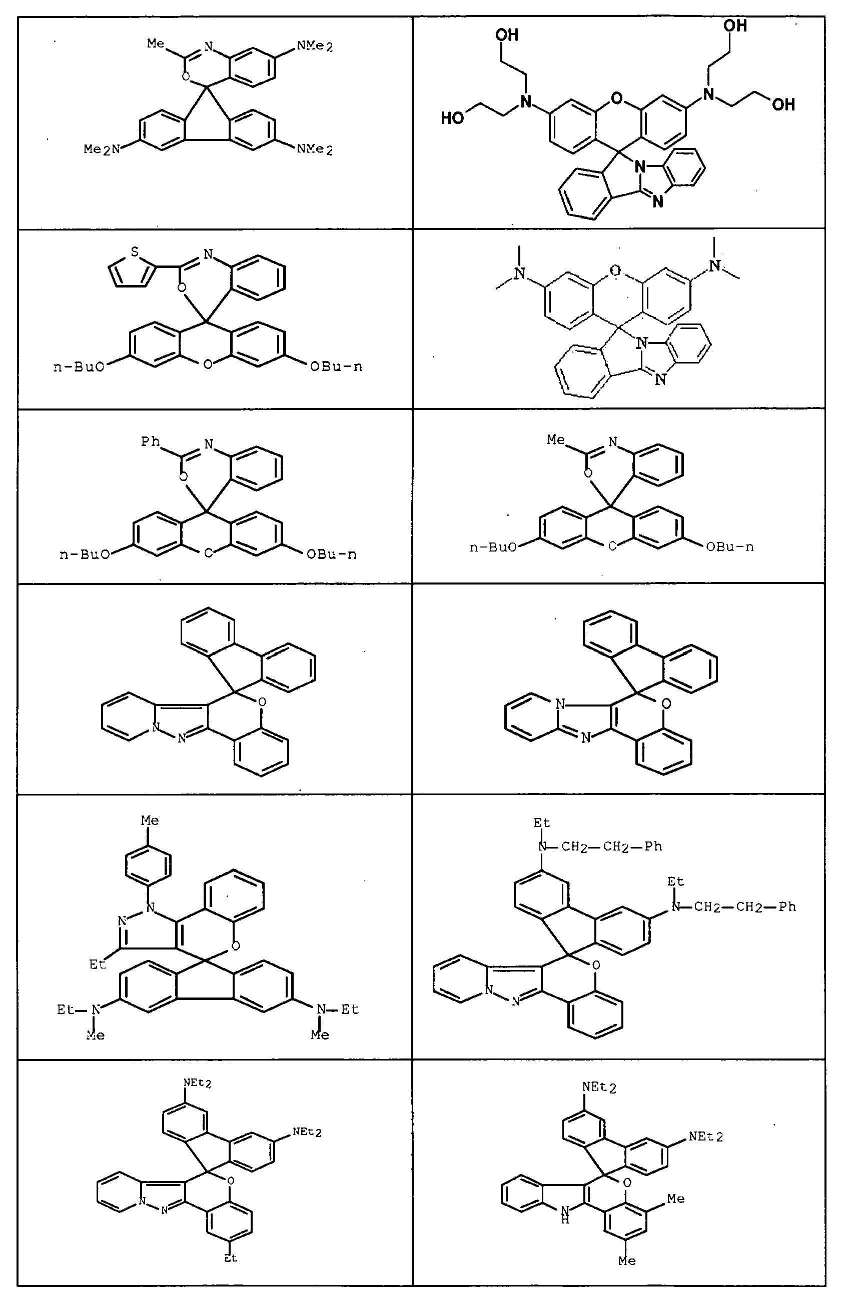

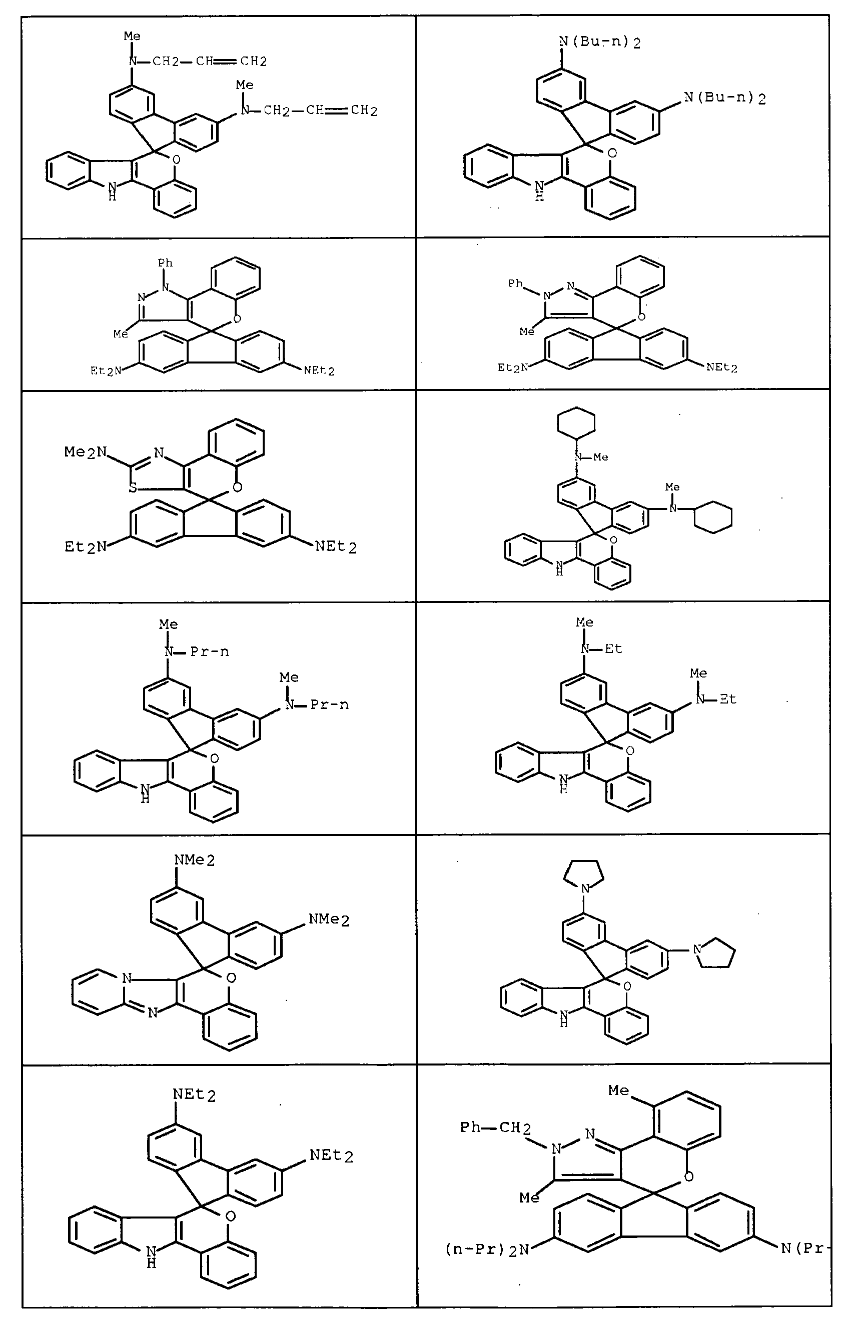

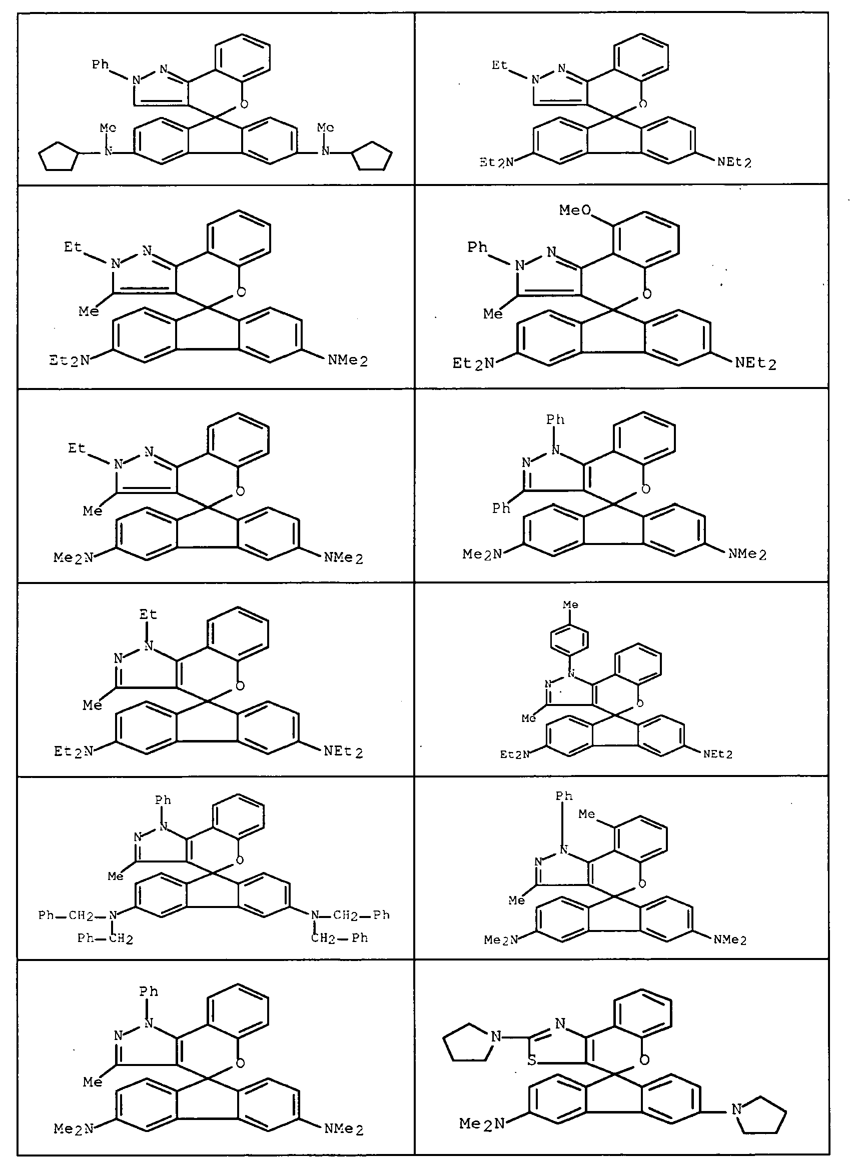

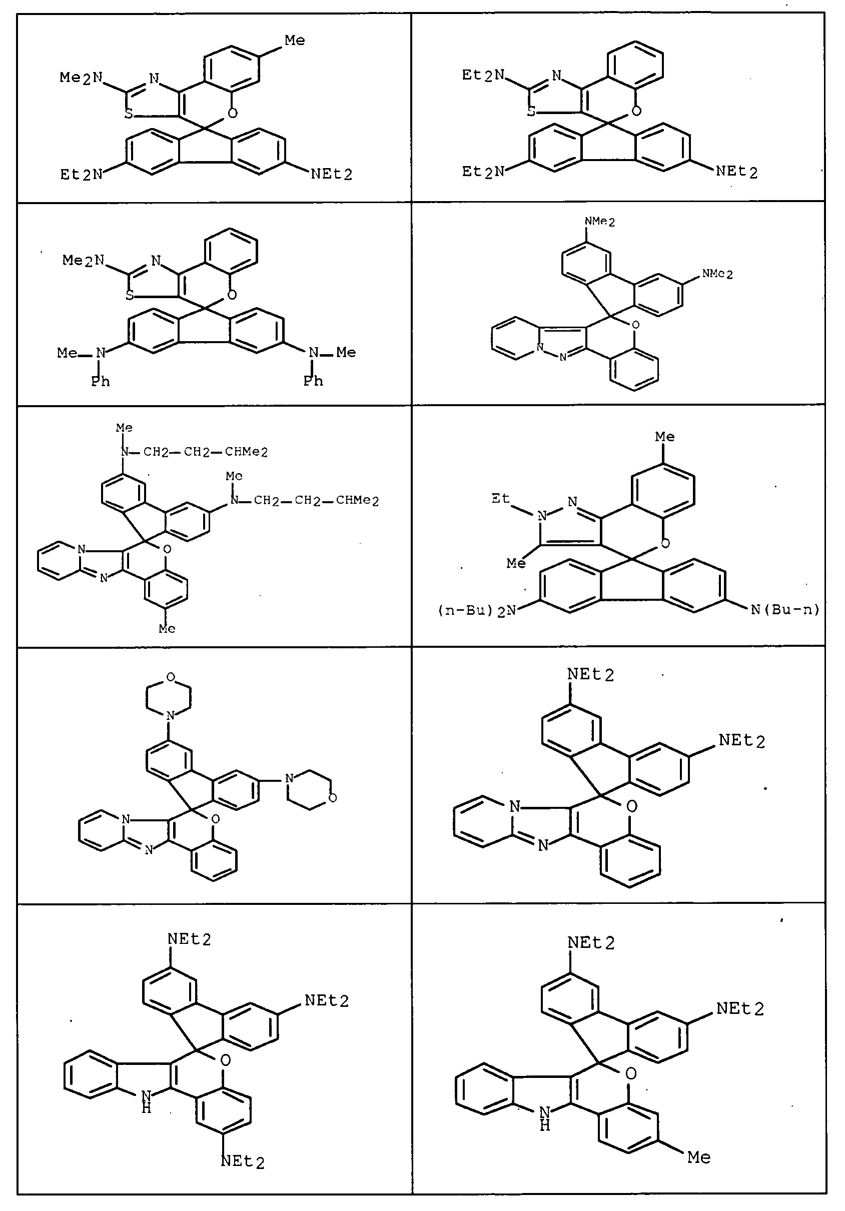

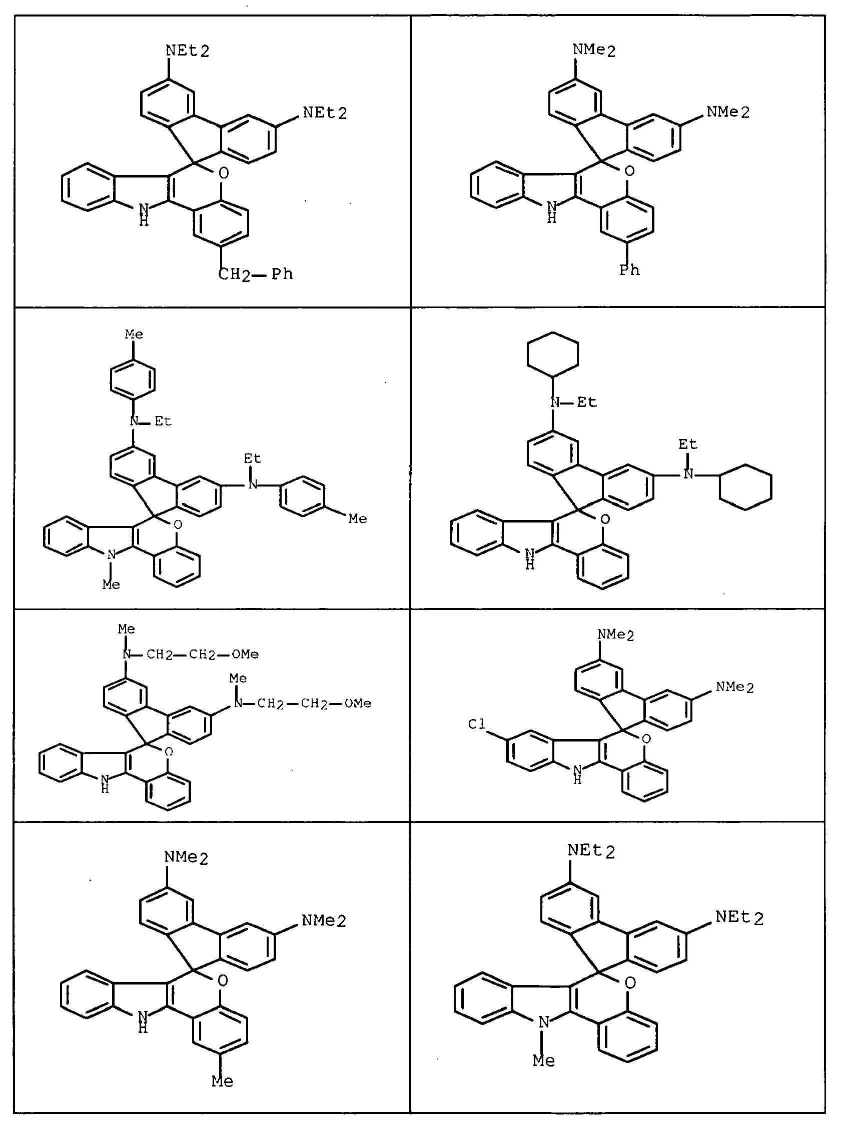

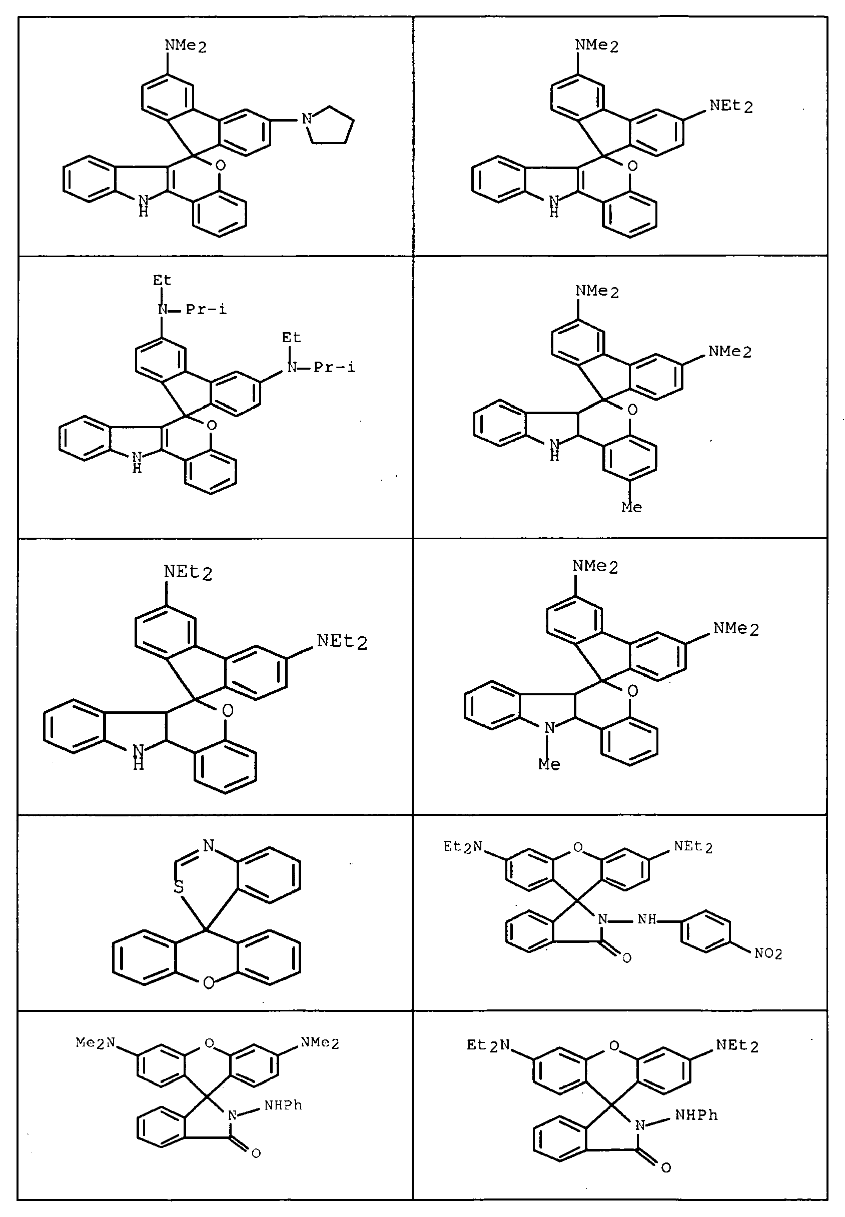

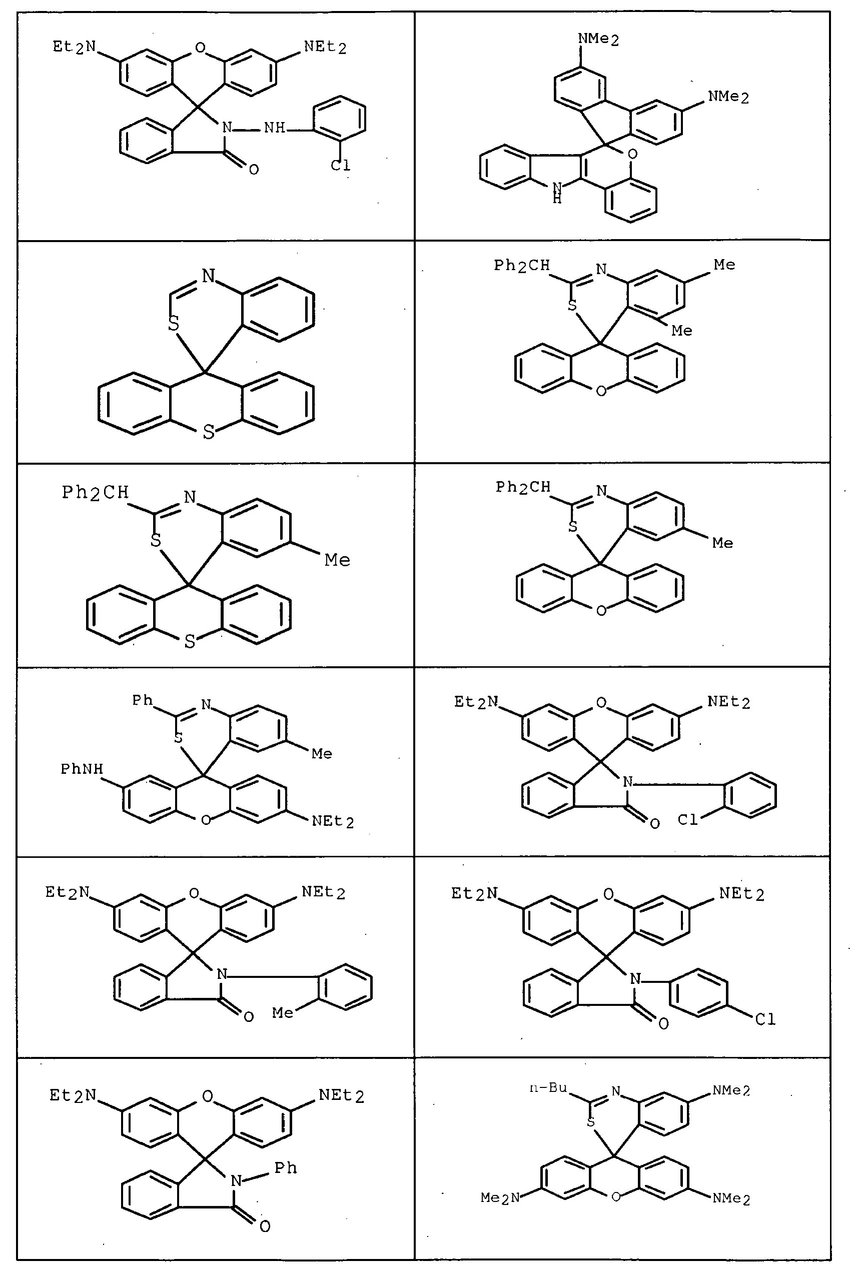

De préférence, les composés de formule (I) sont choisis parmi les composés suivants :

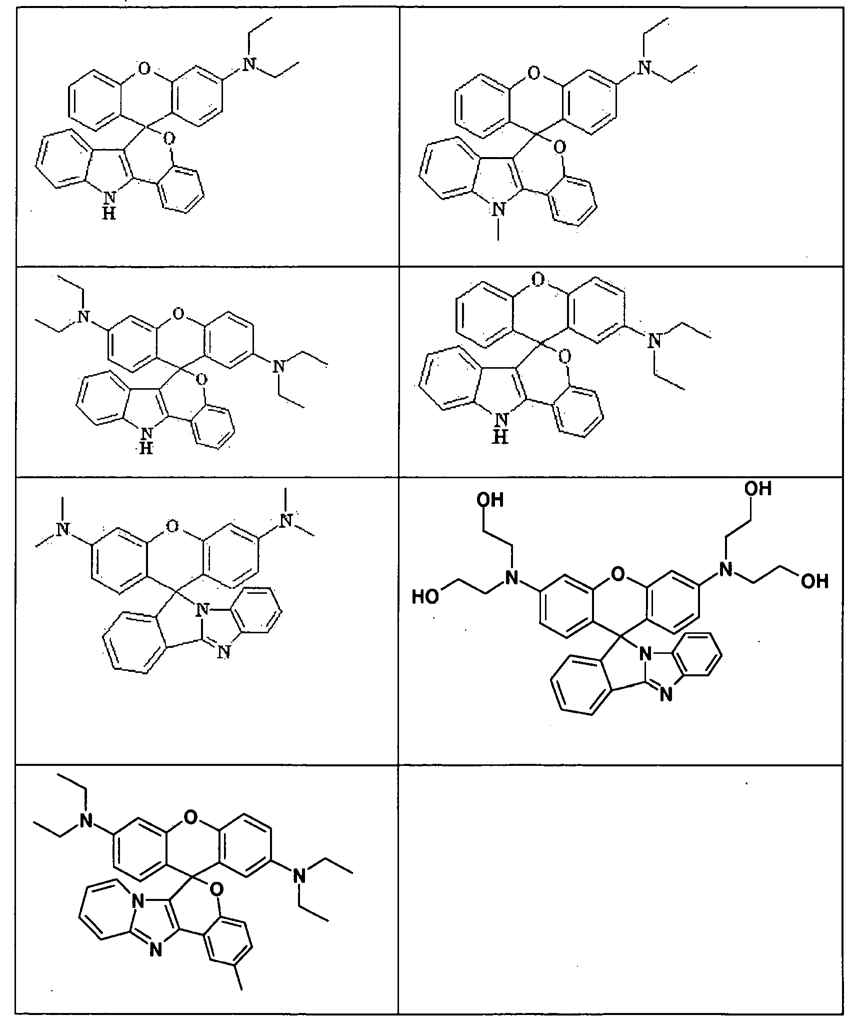

Les composés choisis parmi les composés de formule (I), les colorants correspondant aux composés de formule (I) dont le cycle H est ouvert et leurs sels d'addition représentent en général de 0,001 à 10 % en poids, de préférence de 0,005 à 5 % en poids par rapport au poids total de la composition tinctoriale conforme à l'invention.

D'une manière générale, les sels d'addition des composés de formule (I) et des colorants correspondant aux composés de formule (I) dont le cycle H est ouvert utilisables dans le cadre de l'invention sont notamment choisis parmi les sels d'addition avec un acide tels que les chlorhydrates, les bromhydrates, les sulfates, les méthosulfates, les gluconates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition conforme à l'invention peut en outre contenir un ou plusieurs colorants directs pouvant notamment être choisis parmi les colorants nitrés de la série benzénique, les colorants directs azoïques, les colorants directs méthiniques et les colorants naturels. Ces colorants directs peuvent être de nature non ionique, anionique ou cationique.

En présence de colorants directs additionnels dans la composition conforme à l'invention, ceux-ci représentent en général de 0,001 à 20 % en poids par rapport au poids total de la composition tinctoriale conforme à l'invention, de préférence de 0,01 à 10 %.

La composition de la présente invention peut en outre comprendre un ou plusieurs colorants d'oxydation choisis parmi les bases d'oxydation et les coupleurs classiquement utilisées en coloration d'oxydation.

En présence de colorants d'oxydation dans la composition conforme à l'invention, ceux-ci représentent en général de 0,001 à 20 % en poids par rapport au poids total de la composition tinctoriale conforme à l'invention, de préférence de 0,01 à 10 %.

Le milieu approprié pour la teinture, appelé aussi support de teinture, est généralement constitué par de l'eau ou par au moins un solvant organique ou par un mélange d'eau et d'au moins un solvant organique. A titre de solvant organique, on peut par exemple citer les cétones tels que l'acétone ; les alcanols en C₁-C₄, tels que l'éthanol et l'isopropanol ; les polyols comme le propylène glycol, la glycérine et l'héxylèneglycol ; les éthers de polyols comme le 2-butoxyéthanol, le monométhyléther de propylèneglycol, le monoéthyléther et le monométhyléther du diéthylèneglycol ; et leurs mélanges.

Les solvants sont généralement présents dans des proportions comprises entre 1 et 40 % en poids environ par rapport au poids total de la composition tinctoriale, et encore plus préférentiellement entre 5 et 30 % en poids environ.

Les composés choisis parmi les composés de formule (I), les colorants correspondant aux composés de formule (I) dont le cycle H est ouvert et leurs sels d'addition sont soit solubles, soit en dispersion, dans le support de teinture.

La composition conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des agents dispersants, des agents de conditionnement tels que par exemple des polymères cationiques ou amphotères, des cations, des silicones volatiles ou non volatiles, modifiées ou non modifiées, des chitosanes ou des dérivés de chitosane, des agents filmogènes, des céramides, des agents conservateurs, des agents opacifiants.

Les adjuvants ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Lorsque les solvants sont constitués par de l'eau ou par un mélange d'eau et d'au moins un solvant organique, le pH de la composition tinctoriale conforme à l'invention est généralement compris entre 3 et 12, de préférence entre 4 et 11, encore plus préférentiellement entre 6 et 8,5. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux comme par exemple l'acide chlorhydrique, l'acide nitrique, l'acide sulfurique ou les acides organiques comme par exemple les composés comprenant au moins une fonction acide carboxylique comme l'acide acétique, l'acide tartrique, l'acide citrique, l'acide lactique, une fonction acide sulfonique, une fonction acide phosphonique ou une fonction acide phosphorique.

Parmi les agents alcalinisants on peut citer, à titre d'exemple :
- les acides aminés basiques ;
- les carbonates ou les bicarbonates de métaux alcalins ou alcalino-terreux ;
- les silicates ou les métasilicates ;
- les composés de formule (II) suivante :

   X(OH)ₙ (II)

   dans laquelle :
   - X représente un ion potassium, lithium, sodium, ammonium N⁺R₁₃R₁₄R₁₅R₁₆ avec R₁₃, R₁₄, R₁₅ et R₁₆, identiques ou différents, désignant un radical alkyle en C₂-C₄ lorsque n est égal à 1 ;
   - X représente un atome de magnésium ou de calcium lorsque n est égal à 2 ;
- les composés de formule (III) suivante : 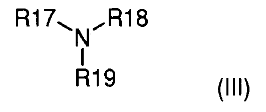 dans laquelle :
   - R₁₇ représente un atome d'hydrogène ; un radical alkyle en C₁-C₆, un radical monohydroxyalkyle en C₁-C₆ ;un radical polyhydroxyalkyle en C₂-C₆ ;
   - R₁₈ et R₁₉, identiques ou différents, représentent un atome d'hydrogène ; un radical alkyle en C₁-C₆ ; un radical monohydroxyalkyle en C₁-C₆ ; un radical polyhydroxyalkyle en C₂-C₆
- les composés de formule (IV) suivante : 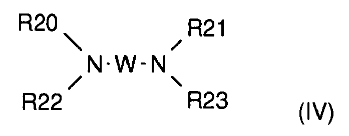 dans laquelle :
   - W est un reste propylène éventuellement substitué par un groupement hydroxyle ou un radical alkyle en C₁-C₄ ;
   - R₂₀, R₂₁, R₂₂ et R₂₃, identiques ou différents, représentent un atome d'hydrogène, un radical alkyle en C₁-C₄ ou hydroxyalkyle en C₁-C₄.

Au sens de la présente invention, on entend par "acide aminé basique", soit (i) un acide aminé présentant en plus de la fonction amine positionnée en α du groupement carboxyle, un groupement cationique (ou basique) supplémentaire; soit (ii) un acide aminé présentant une chaîne latérale (hydrophile) cationique (ou basique); soit (iii) un acide aminé portant une chaîne latérale constituée d'une base azotée. Ces définitions sont généralement connues et publiées dans les ouvrages de biochimie générale tels que J.H. WEIL (1983) pages 5 et suivantes, Lubert STRYER (1995) page 22, A. LEHNINGER (1993) pages 115-116, DE BOECK-WESMAEL (1994) pages 57-59.

Les acides aminés basiques conformes à l'invention sont choisis de préférence parmi ceux répondant à la formule (D) suivante : 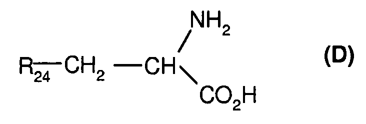 où R₂₄ désigne un groupe choisi parmi : 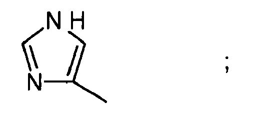 -(CH₂)₃NH₂ ;

-(CH₂)₂NH₂ ;

-(CH₂)₂NHCONH₂ ;

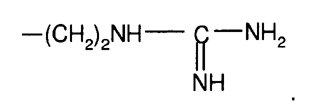

Parmi les composés de formule (D), on peut citer à titre d'exemple l'histidine, la lysine, l'ornithine, la citrulline, l'arginine.

La composition selon l'invention peut se présenter sous des formes diverses, telles que sous forme de liquides, de crèmes, de gels, ou sous toute autre forme appropriée pour réaliser une coloration des fibres kératiniques, et notamment des cheveux humains.

Le procédé de traitement des fibres kératiniques conforme à l'invention permet d'obtenir une coloration intense et tenace dont les reflets peuvent varier en fonction du pH ou de la température et qui peut être effacée et reformée au moins une fois sans perte substantielle de couleur. De préférence, la coloration est modifiée, effacée ou reformée en ajustant le pH à l'aide d'un agent acidifiant ou d'un agent alcalinisant.

Dans le sens de la présente invention, la coloration est effacée lorsque les fibres kératiniques ont retrouvé leur couleur d'origine. La coloration est modifiée lorsque la coloration obtenue est différente de celle obtenue au cours de l'étape précédente. La coloration est reformée lorsque la coloration des fibres kératiniques obtenue est sensiblement la même que celle qui avait été obtenue au cours d'une étape précédente et qui avait ensuite été modifiée.

La coloration obtenue dépend des composés de formule (I) qui sont appliqués sur les fibres kératiniques. Lorsque la totalité de ces composés ont leur cycle H ouvert, la coloration est intense. En jouant sur le pH ou la température, il est possible d'effacer cette coloration en passant de la totalité des composés de formule (I) dont le cycle H est ouvert à la totalité des composés de formule (I) dont le cycle H est fermé, puis de la reformer en passant de la totalité des composés de formule (I) dont le cycle H est fermé à la totalité des composés de formule (I) dont le cycle H est ouvert. Il est également possible de faire varier le rapport entre la concentration en composés de formule (I) dont le cycle H est ouvert et la concentration en composés de formule (I) dont le cycle H est fermé. La coloration est alors modifiée en intensité ou en couleur suivant qu'on applique sur les fibres kératiniques un ou plusieurs composés de formule (I) dont le cycle H est fermé ou un ou plusieurs composés de formule (I) dont le cycle H est ouvert et suivant leur sensibilité relative au pH ou à la température.

Un procédé préféré de traitement des fibres kératiniques, et en particulier des fibres kératiniques humaines telles que les cheveux, est **caractérisé en ce que** les étapes suivantes sont mises en oeuvre :
a) application d'une composition tinctoriale selon l'invention sur les fibres kératiniques pendant un temps de pose suffisant, le pH étant ajusté à l'aide d'un agent acidifiant ou d'un agent alcalinisant en fonction de la coloration souhaitée
b) éventuellement modification de la coloration des fibres kératiniques à l'aide d'un agent acidifiant ou d'un agent alcalinisant appliqué sur les fibres kératiniques.

L'application de la composition tinctoriale selon l'invention dans l'étape a) peut être ou non suivie d'un rinçage.

Selon un mode de réalisation particulier de l'invention, dans la première étape, l'agent acidifiant ou l'agent alcalinisant est mélangée à la composition tinctoriale avant application sur les fibres kératiniques.

Selon un autre mode de réalisation particulier de l'invention, dans la première étape, l'agent acidifiant ou l'agent alcalinisant est appliqué avant ou après la composition tinctoriale. Entre l'application de l'agent acidifiant ou de l'agent alcalinisant et l'application de la composition tinctoriale, on peut avoir un écart de 5 à 30 minutes. L'agent acidifiant ou l'agent alcalinisant est de préférence appliqué après la composition tinctoriale.

Le temps de pose de la composition conforme à l'invention est généralement compris entre 3 et 60 minutes, de préférence entre 5 et 40 minutes, encore plus préférentiellement entre 10 et 20 minutes.

La température d'application est généralement fixée entre la température ambiante et 80 °C, de préférence entre 25 et 55 °C.

Les fibres kératiniques peuvent être ou non rincées après application de l'agent acidifiant ou l'agent alcalinisant.

Les agents acidifiants ou alcalinisants sont choisis parmi ceux qui sont décrits ci-dessus.

La présente invention a également pour objet un dispositif à plusieurs compartiments ou kit permettant de mettre en oeuvre le procédé de coloration des fibres kératiniques décrit ci-dessus.

Le dispositif à plusieurs compartiments de l'invention contient dans un premier compartiment une composition conforme à l'invention et dans un deuxième compartiment un agent acidifiant ou un agent alcalinisant. Dans une autre version, le dispositif à plusieurs compartiments de l'invention contient dans le deuxième compartiment un agent acidifiant et dans un troisième compartiment un agent alcalinisant.

La présente invention a également pour objet une composition pour la coloration des fibres kératiniques, et en particulier des fibres kératiniques humaines telles que les cheveux telle que définie ci-dessus comprenant de plus au moins un tensioactif et au moins un polymère, de préférence un polymère épaississant associatif ou non associatif.

Le ou les tensio-actifs peuvent être choisis parmi les tensio-actifs anioniques, cationiques, non-ioniques, amphotères, zwittérioniques ou leurs mélanges.

Par polymère associatif, on entend au sens de la présente invention tout polymère comportant au moins une chaîne grasse en C₈-C₃₀.

Par polymère épaississant, on entend au sens de la présente invention tout polymère qui, introduit dans la composition sans polymère à 25 °C, permet d'en accroître la viscosité, et en particulier tout polymère qui, introduit à 1 % en matière active dans la composition sans polymère, permet d'en accroître la viscosité d'au moins 100 cps à la température de 25 °C et à un taux de cisaillement de 1 s⁻¹. Cette viscosité est mesurable par exemple à l'aide d'un viscosimètre de type cone / plan.

On peut citer à titre d'exemple de polymère épaississant associatif les dérivés de polyuréthanes associatifs, les dérivés de celluloses associatifs, les dérivés de polyvinyllactames associatifs et les dérivés de polyacides insaturés associatifs.

On peut citer à titre d'exemple de polymère épaississant non associatif les homopolymères d'acide acrylique réticulés ; les homopolymères réticulés d'acide 2-acrylamido-2-méthyl-propane sulfonique et leurs copolymères réticulés d'acrylamide partiellement ou totalement neutralisés ; les homopolymères d'acrylate d'ammonium ou les copolymères d'acrylate d'ammonium et d'acrylamide ; les homopolymères de diméthylaminoéthyl-méthacrylate quaternisé par le chlorure de méthyle ou les copolymères de diméthylamino-éthylméthacrylate quaternisé par le chlorure de méthyle et d'acrylamide ; les gommes de guar non ioniques ; les gommes de biopolysaccharides d'origine microbienne telles que les gommes de scléroglucane ou de xanthane ; les gommes issues d'exudats végétaux telles que les gommes arabiques, gommes Ghatti, gommes Karaya et Tragacanthe ; les hydroxypropyl ou carboxyméthyl celluloses; les pectines ; les alginates.

L'exemple qui suit sert à illustrer l'invention sans toutefois présenter un caractère limitatif.

### EXEMPLE

### Colorant :

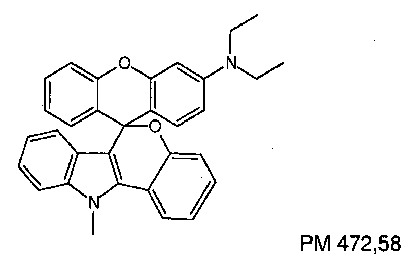

### Composition selon l'invention :

| | **Composition** |
|---|---|
| Colorant | 2 g |
| Alcool benzylique | 4 g |
| Polyéthylèneglycol 6OE | 6 g |
| Acide méthane sulfonique | q.s.p. pH 3 |
| Eau déminéralisée | q.s.p. 100 g |

Cette composition est appliquée sur des mèches de cheveux gris à 90 % de cheveux blancs naturels et permanentés, à raison de 5 g pour 1 g de cheveux, à température ambiante pendant 30 minutes. A l'issue du temps de pose, la mèche est séchée.
La coloration capillaire est évaluée de manière visuelle. La nuance obtenue est un violine.
Les mèches ainsi colorées peuvent être décolorées très rapidement par application d'une solution de soude 0,1 M. Les mèches retrouvent leur nuance d'origine.

## Revendications

1. Utilisation pour la coloration des fibres kératiniques, d'une composition comprenant, dans un milieu approprié pour la teinture, au moins un composé choisi parmi les composés de formule (I) comportant un groupement cyclique G incluant un cycle H susceptible de s'ouvrir, les colorants correspondant aux composés de formule (I) dont le cycle H est ouvert et leurs sels d'addition : 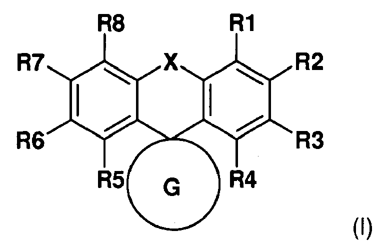 dans laquelle :
• **R₁, R₂, R₃, R₄, R₅, R₆, R₇** et **R₈** représentent, indépendamment l'un de l'autre :
- un atome d'hydrogène ;
- un radical halogéno ;
- un radical hydroxyle ;
- un radical nitro ;
- un radical amino ;
- un radical carboxy ;
- un radical aminocarbonyle ;
- un radical cyano ;
- un radical issu d'une chaîne hydrocarbonée comportant de 1 à 100 atomes de carbone, linéaire ou ramifiée, acyclique ou mono ou polycyclique, condensée ou non condensée, saturée ou insaturée, aromatique ou non aromatique, pouvant être interrompue par un ou plusieurs hétéroatomes choisis parmi les atomes d'azote, d'oxygène et de soufre ou par un ou plusieurs groupements carbonyle, pouvant être terminée par un groupement hydrogénocarbonyle ou par un groupement contenant un ou plusieurs hétéroatomes choisis parmi les atomes d'azote, d'oxygène et de soufre, pouvant commencer par un groupement carbonyle ou par un groupement contenant un ou plusieurs hétéroatomes choisis parmi les atomes d'azote, d'oxygène et de soufre, et pouvant être substituée par un ou plusieurs groupements choisis parmi les radicaux hydroxyle, halogéno, carboxy, carboxyalkyle en C₁-C₉, cyano, amino, alcoxy en C₁-C₆ ;
• deux des radicaux **R₁, R₂, R₃, R₄, R₅, R₆, R₇** et **R₈** portés par deux atomes de carbone adjacents pouvant former ensemble et avec les atomes de carbone auxquels ils sont attachés un groupement mono ou polycarbocyclique aromatique, condensé ou non condensé, comprenant de 5 à 20 chaînons, un ou plusieurs atomes de carbone pouvant être remplacés par un atome d'oxygène, d'azote, de soufre ou de phosphore, le groupement mono ou polycarbocyclique aromatique étant non substitué ou substitué par un ou plusieurs radicaux choisis parmi un radical halogéno, hydroxyle, amino, carboxy, aryle en C₆-C₁₈, cyano ;
les radicaux amino étant non substitués ou substitués par un ou deux radicaux, identiques ou différents, choisis parmi un radical alkyle en C₁-C₉ ; un radical hydroxyalkyle en C₁-C₉ ; un radical alcényle en C₂-C₉ ; un radical cycloalkyle en C₆-C₁₂ ; un radical aryle en C₆-C₁₈ lui-même éventuellement substitué par un ou plusieurs radicaux choisis parmi un radical halogéno, alkyle en C₁-C₉ ; un radical aryl(C₆-C₁₈)carbonyle ; un radical cycloalkyl(C₆-C₁₂)alkyle en C₁-C₉ ; un radical aryl(C₆-C₁₈)alkyl(C₁-C₉) ; un radical alkyl(C₁-C₉)carbonyle ; un radical alcoxy(C₁-C₉)carbonylalkyl(C₁-C₉) ; un radical α-naphtylalkyle ; un radical halogénoalkyle en C₁-C₉ ; un radical cyanoalkyle en C₁-C₉ ; un radical acyle en C₂-C₁₅ ; un radical alcoxy(C₁-C₉)carbonyle ; un radical aryloxy(C₆-C₁₈)carbonyle ; un radical aryloxy(C₆-C₁₈)alkyl(C₁-C₉)carbonyle ; un radical aryl(C₆-C₁₈)alcoxy(C₁-C₉)carbonyle ; un radical alcoxy(C₁-C₉)aryl(C₆-C₁₈)carbonyle ; un radical alcoxy(C₁-C₉)alkyl(C₁-C₉)carbonyle ; un radical dialkyl(C₁-C₉)aminocarbonyle ; un radical dialkyl(C₁-C₉)aminosulfonyle ; un radical alkyl(C₁-C₉)aryl(C₆-C₁₈)sulfonyle ; un radical alkyl(C₁-C₉)sulfonyle ; un radical dialkyl(C₁-C₉)aminoalkyle en C₁-C₉ ; un radical alcoxy(C₁-C₉)alkyle en C₁-C₉ ;
lorsque les radicaux amino sont substitués par deux radicaux, ceux-ci peuvent former avec l'atome d'azote du radical amino un hétérocycle à 5 ou 6 chaînons comportant éventuellement un ou plusieurs hétéroatomes additionnels ;
• **X** représente une liaison directe ou un atome divalent tel qu'un atome de soufre ou d'oxygène ou un groupement sulfone SO₂ ou C(R₁₃)₂ ou NR₁₃ ;
• **G** représente un radical divalent ayant l'un des formules suivantes : 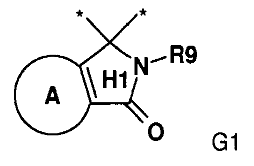 ou 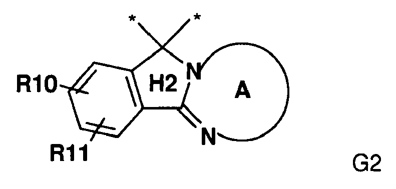 ou 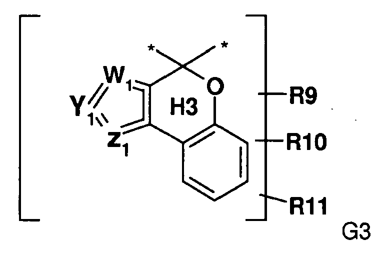 ou 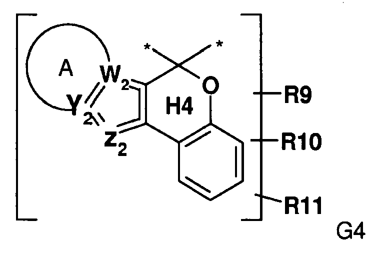 ou 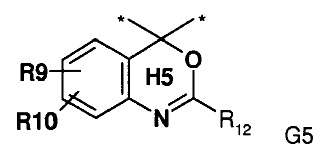 ou 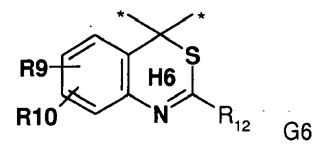 ou 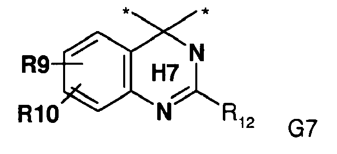
dans lesquelles :
**Y₁**, **W₁** et **Z₁** d'une part, et **Y₂, W₂** et **Z₂** d'autre part, représentent, indépendamment les uns des autres, un atome de carbone, un atome d'azote, un atome de soufre ou un groupement divalent CR₁₃ ou NR₁₃ ;
**R₉, R₁₀, R₁₁** et **R₁₃** ont les mêmes définitions que **R₁ ;**
**R₁₂** représente :
- un atome d'hydrogène ;
- un radical alkyle en C₁-C₉ ;
- un radical amino ;
- un radical alcoxy en C₁-C₉ ;
- un radical aryle en C₆-C₁₈ non substitué ou substitué par un ou plusieurs groupements choisis parmi un atome d'hydrogène, un radical hydroxyle, alkyle en C₁-C₉, aryle en C₆-C₁₈, aryloxy en C₆-C₁₈, alcoxy en C₁-C₉, halogéno, carboxy, cyano, amino substitué ou non ;
- un radical furanyle ;
- un radical alkyl(C₁-C₉)thio ;
- un radical thiényle ;
- un radical phénylcarbonyle ;
- un radical trifluoroalkyle ;
- un radical diaryl(C₆-C₁₈)alkyle en C₁-C₉ ;
**A** représente un groupement aryle en C₆-C₁₈ ou un groupement hétérocyclique insaturé ou non et substitué ou non comprenant de 5 à 12 chaînons.

2. Utilisation selon la revendication 1 dans laquelle le composé de formule (I) est tel que :
• R₁, R₄, R₅ et R₈ désignent un atome d'hydrogène ;
• R₂ et R₇ désignent, indépendamment l'un de l'autre, un atome d'hydrogène ; un radical halogéno ; un radical alkyle en C₁-C₉ ; un radical alcoxy en C₁-C₉ ; un radical amino substitué ou non ;
• R₃ et R₆ désignent, indépendamment l'un de l'autre, un atome d'hydrogène ; un radical halogéno ; un radical alkyle en C₁-C₉ ; un radical amino substitué ou non ;
• X représente une liaison directe ou un atome de soufre ou d'oxygène ou un groupe SO₂ ;
• G représente un radical divalent de formule G1, G2, G3, G4, G5 ou G6.

3. Utilisation selon la revendication 1 ou 2, dans laquelle le composé de formule (I) est tel que G représente un groupement G1 ; X représente un atome d'oxygène ; R₁, R₃, R₄, R₅, R₆ et R₈ désignent un atome d'hydrogène ; R₂ et R₇ désignent, indépendamment l'un de l'autre, un radical amino substitué ou non ; R₉ désigne un radical aryle substitué ou non, un radical amino substitué ou non ; A désigne un cycle benzénique substitué ou non.

4. Utilisation selon la revendication 1 ou 2, dans laquelle le composé de formule (I) est tel que G représente un groupement G2 ; X représente un atome d'oxygène ; R₁, R₃, R₄, R₅, R₆, R₈, R₁₀ et R₁₁ désignent un atome d'hydrogène ; R₂ et R₇ désignent, indépendamment l'un de l'autre, un radical amino substitué ou non ; A représente un cycle benzénique substitué ou non.

5. Utilisation selon la revendication 1 ou 2, dans laquelle le composé de formule (I) est tel que G représente un groupement G3 ; R₁, R₃, R₄, R₅, R₆, R₈, R₁₀ et R₁₁ désignent un atome d'hydrogène ; R₂ ou R₇ désignent, indépendamment l'un de l'autre, un atome d'hydrogène, un groupement amino substitué ou non ; R₉ désigne un atome d'hydrogène, un groupement alkyle substitué ou non, un groupement alcoxy ; X représente une liaison directe ; W₁ représente un atome de soufre, un atome de carbone substitué par un atome d'hydrogène, un radical alkyle en C₁-C₄, un radical phényle, un groupement amino porteur de deux groupements alkyle en C₁-C₄, les deux radicaux formant éventuellement avec l'atome d'azote les portant, un hétérocycle saturé comprenant 5 chaînons ; Y₁ représente un atome de carbone substitué par un atome d'hydrogène, un radical alkyle en C₁-C₄, un radical phényle, un groupement amino porteur de deux groupements alkyle en C₁-C₄, les deux radicaux formant éventuellement avec l'atome d'azote les portant, un hétérocycle saturé comprenant 5 chaînons, un atome d'azote, un atome d'azote substitué par un atome d'hydrogène, un radical alkyle en C₁-C₂, un radical phényle, un groupement phénylalkyle dont le groupement alkyle est en C₁-C₄ ; Z₁ représente un atome d'azote, un groupement NH, un atome d'azote substitué par un atome d'hydrogène, un radical alkyle en C₁-C₂, un radical phényle, un groupement phénylalkyle dont le groupement alkyle est en C₁-C₄.

6. Utilisation selon la revendication 1 ou 2, dans laquelle le composé de formule (I) est tel que G représente un groupement G4 ; R₁, R₄, R₅, R₈ et R₁₁ désignent un atome d'hydrogène ; R₂ ou R₇ désignent, indépendamment l'un de l'autre, un atome d'hydrogène, un atome d'halogène, un groupement alcoxy, un groupement amino substitué ou non ; R₃ et R₆ désignent, indépendamment l'un de l'autre, un atome d'hydrogène, un atome d'halogène, un groupement amino substitué ou non ; R₉ désigne un atome d'hydrogène, un groupement alkyle substitué ou non, un groupement alcoxy, un groupement aryle, un groupement amino substitué ou non ; R₁₀ désigne un atome d'hydrogène ou un radical alkyle ; A représente un cycle benzénique substitué ou non ou un cycle pyridinique substitué ou non ; X représente un atome d'oxygène ou de soufre, une liaison directe ou un groupe SO₂ ; W₂ représente un atome de carbone, un atome d'azote ; Y₂ représente un atome de carbone, un atome d'azote ; Z₂ représente un groupement CH, un atome de carbone substitué par un atome d'hydrogène, un radical alkyle en C₁-C₄, un radical phényle, un groupement amino porteur de deux groupements alkyle en C₁-C₄, les deux radicaux formant éventuellement avec l'atome d'azote les portant, un hétérocycle saturé comprenant 5 chaînons, un atome d'azote, un groupement NH, un atome d'azote substitué par un atome d'hydrogène, un radical alkyle en C₁-C₂, un radical phényle, un groupement phénylalkyle dont le groupement alkyle est en C₁-C₄.

7. Utilisation selon la revendication 1 ou 2, dans laquelle le composé de formule (I) est tel que G représente un groupement G5, X représente un atome d'oxygène ou une liaison directe ; R₁, R₄, R₅, R₈ et R₁₀ désignent un atome d'hydrogène ; R₂ et R₇ désignent, indépendamment l'un de l'autre, un radical alkyle, un radical alcoxy, un radical amino substitué ou non ; R₃ et R₆ désignent, indépendamment l'un de l'autre, un atome d'hydrogène, un radical alkyle, un radical amino substitué ou non ; R₉ désigne un atome d'hydrogène, un radical amino substitué ou non ; R₁₂ désigne un radical alkyle substitué ou non, un radical aryle substitué ou non, un radical thiényle, un radical furanyle.

8. Utilisation selon la revendication 1 ou 2, dans laquelle le composé de formule (I) est tel que G représente un groupement G6 ; X représente une liaison directe ou un atome d'oxygène ; R₁, R₃, R₄, R₅, R₆, R₈ et R₁₀ désignent un atome d'hydrogène, R₂, R₉ et R₇ désignent, indépendamment l'un de l'autre, un groupement amino substitué ou non ; R₁₂ désigne un radical alkyle.

9. Utilisation selon l'une quelconque des revendications précédentes, dans laquelle le composé de formule (I) est choisi parmi les composés suivants :

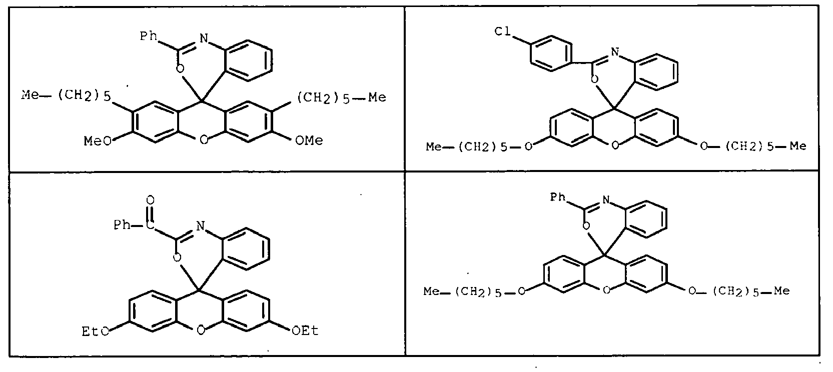

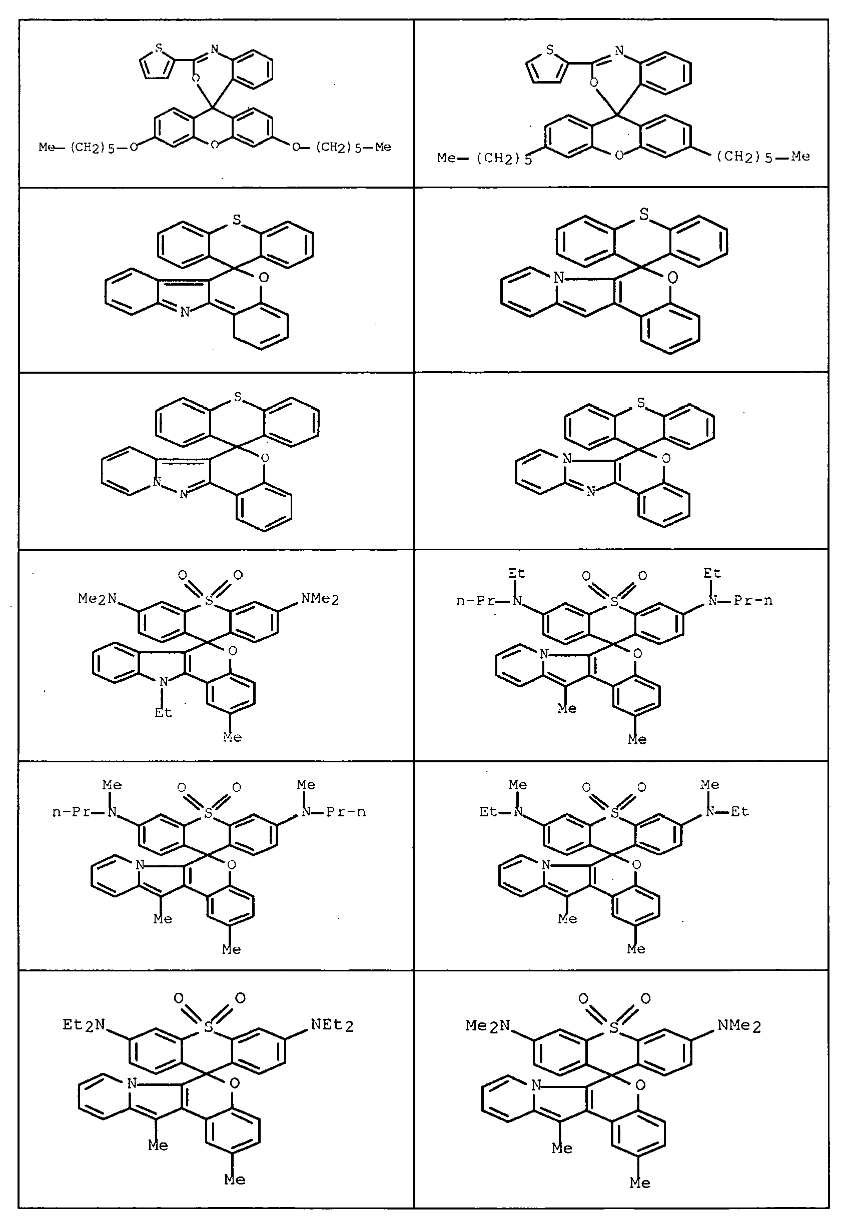

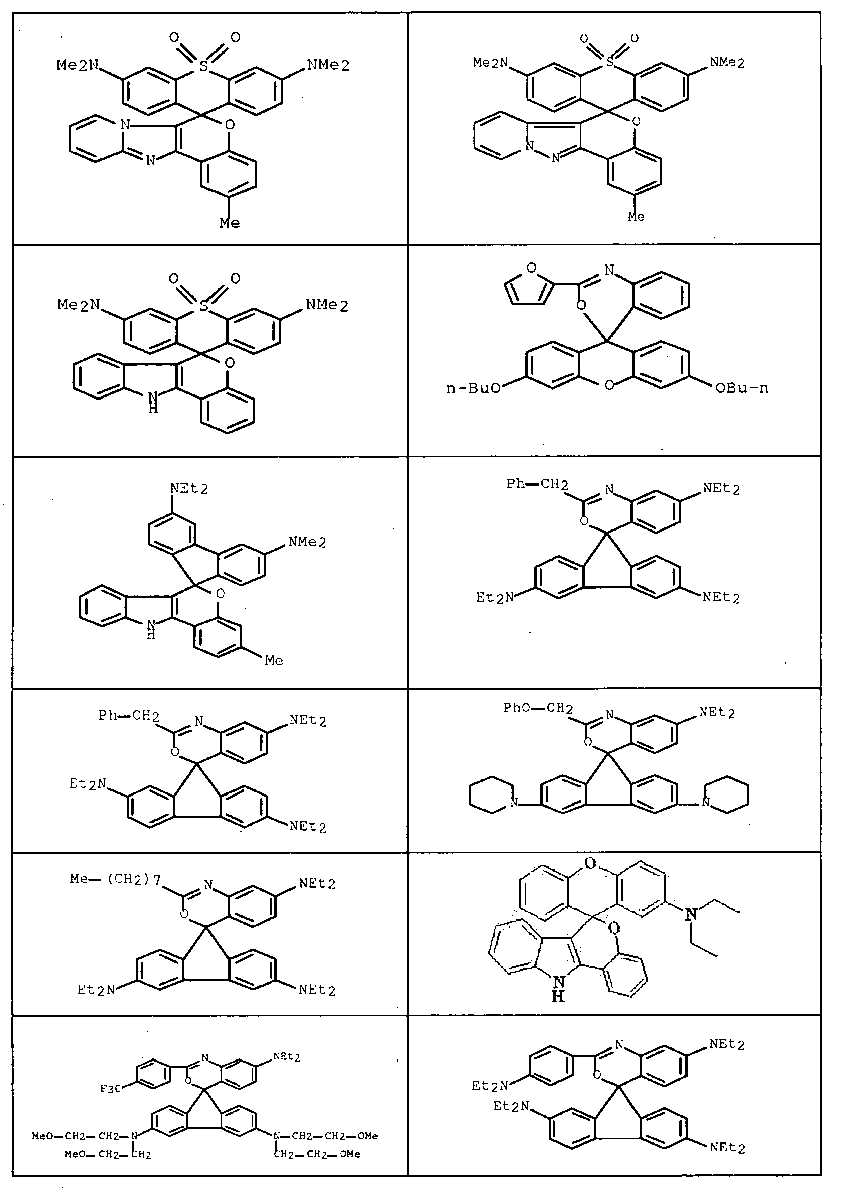

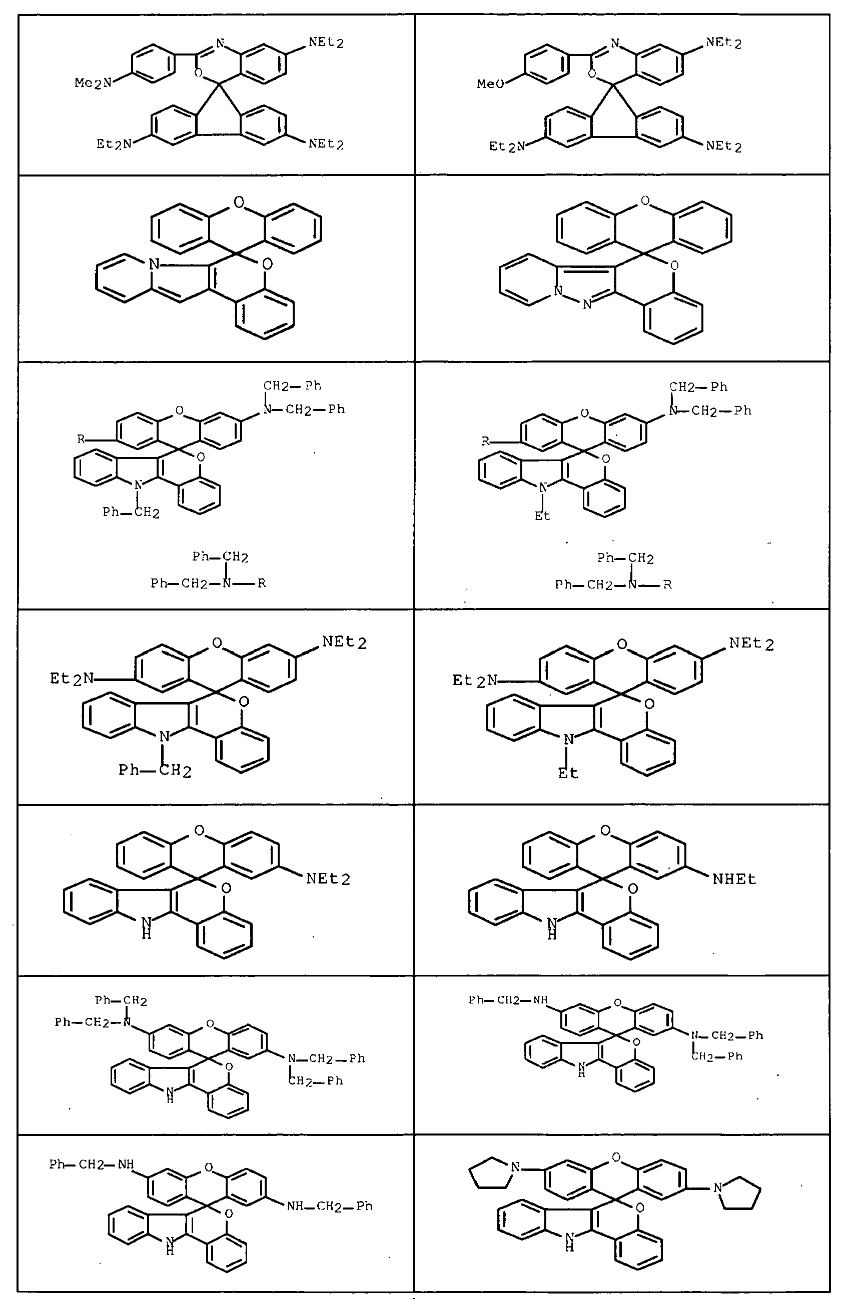

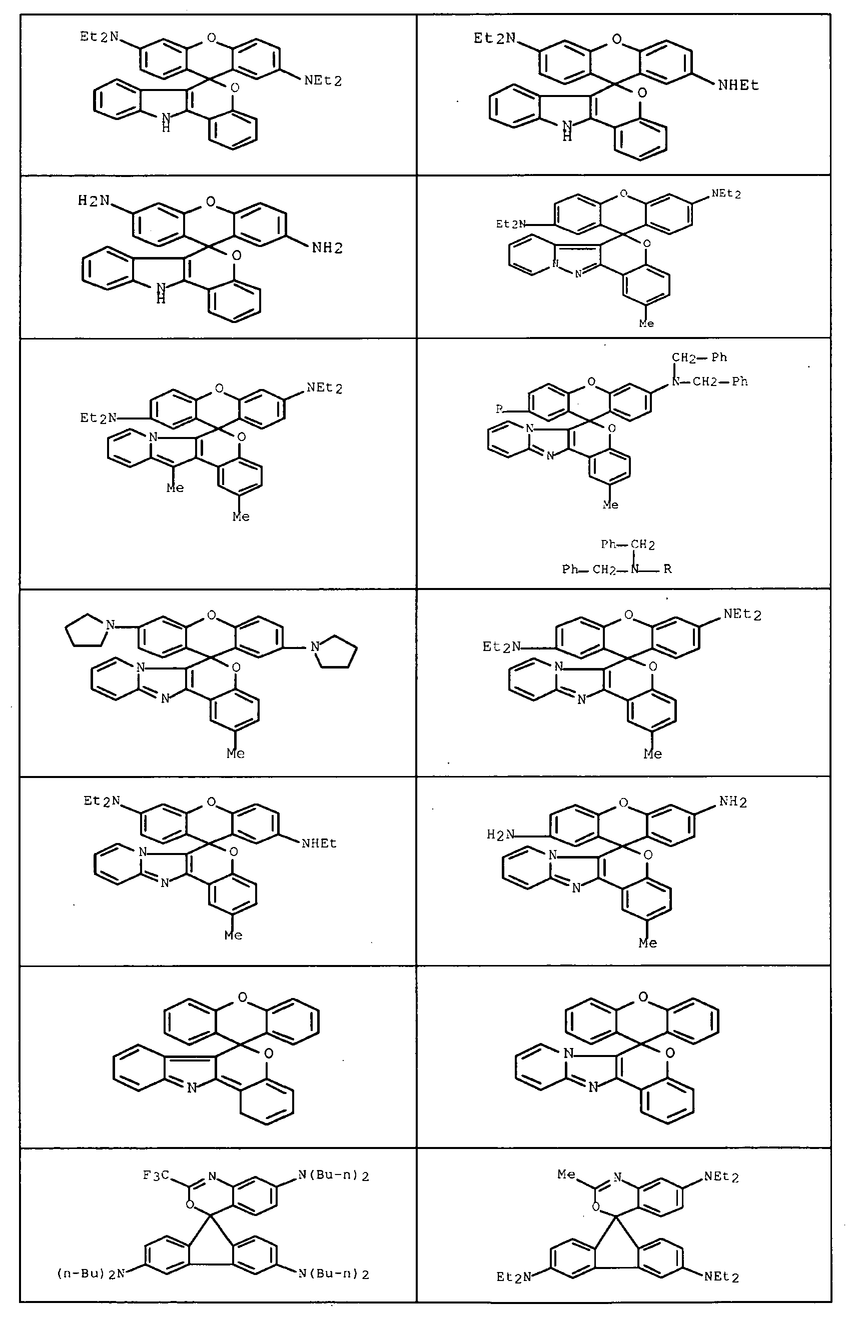

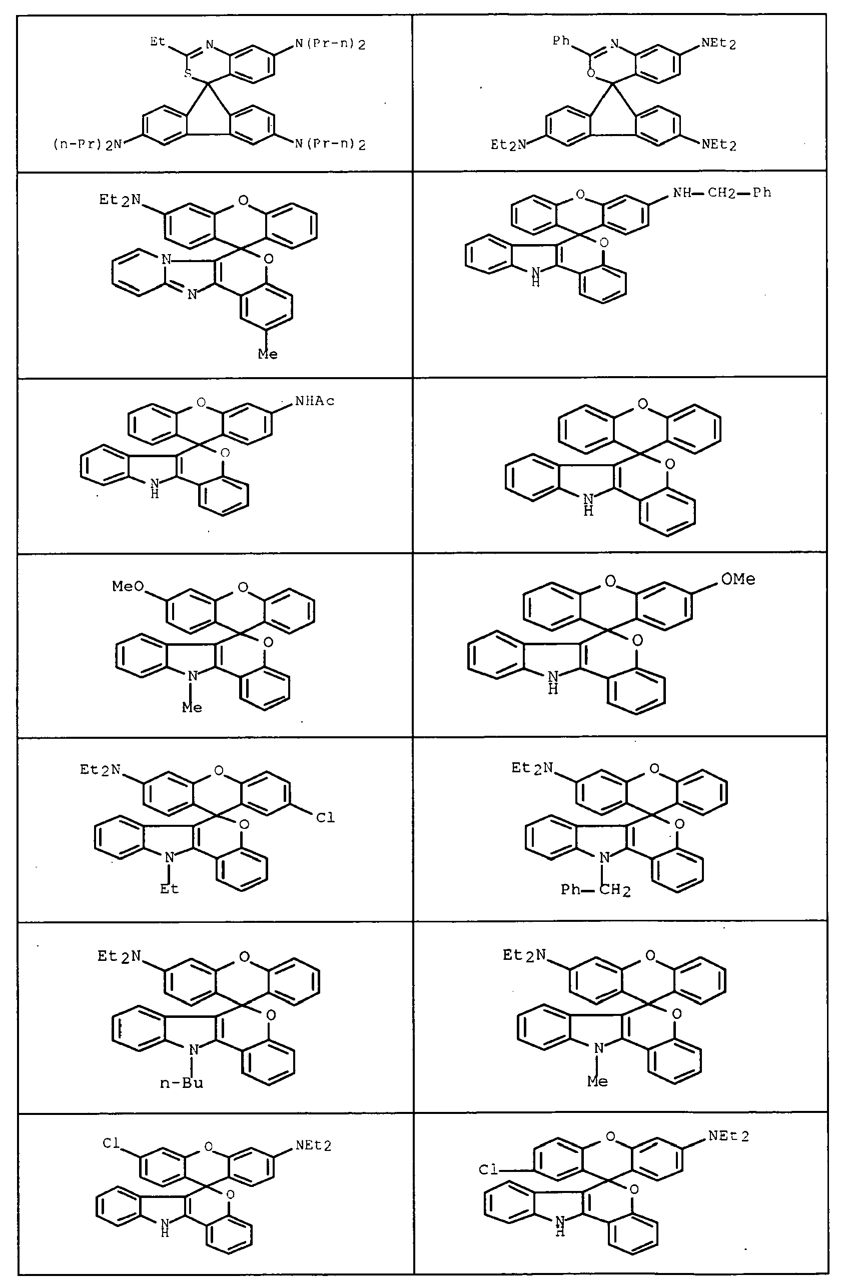

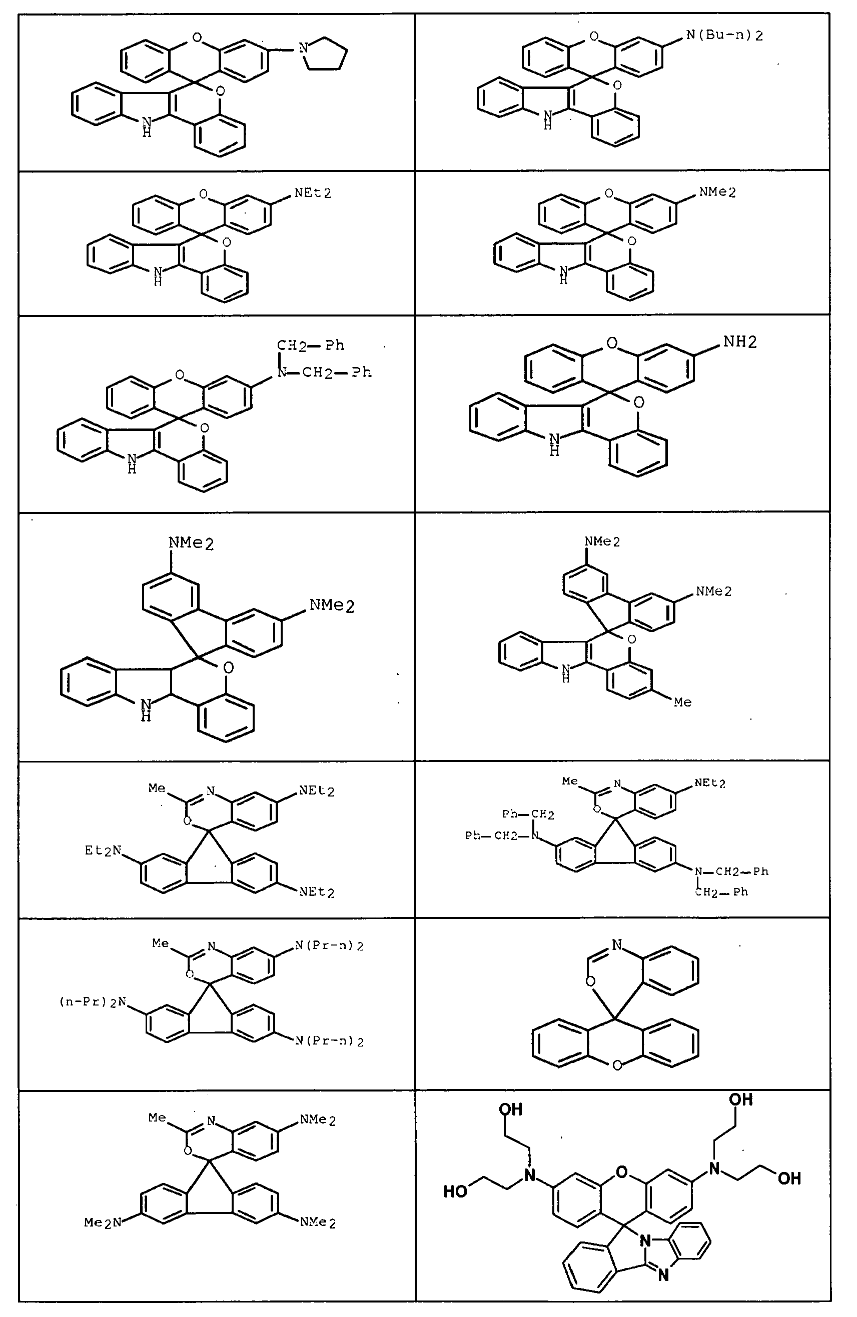

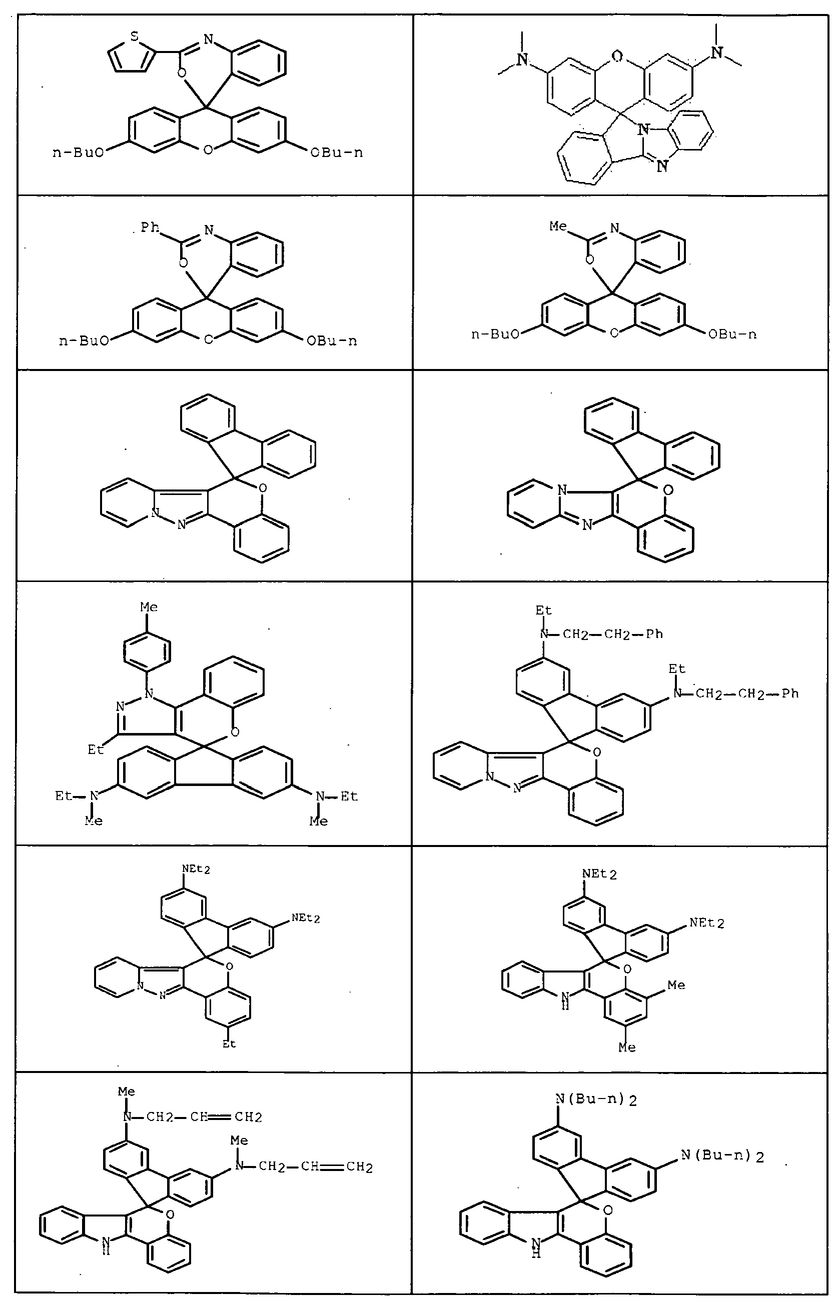

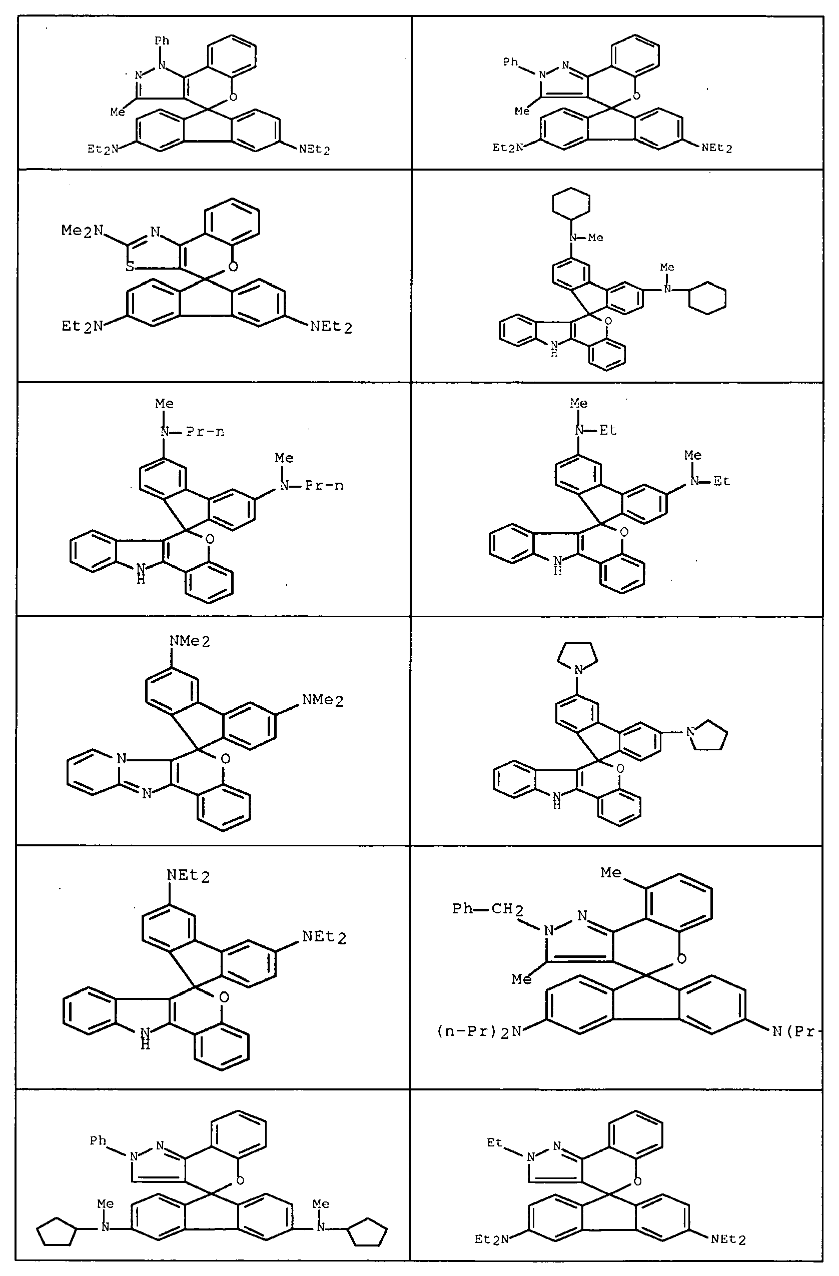

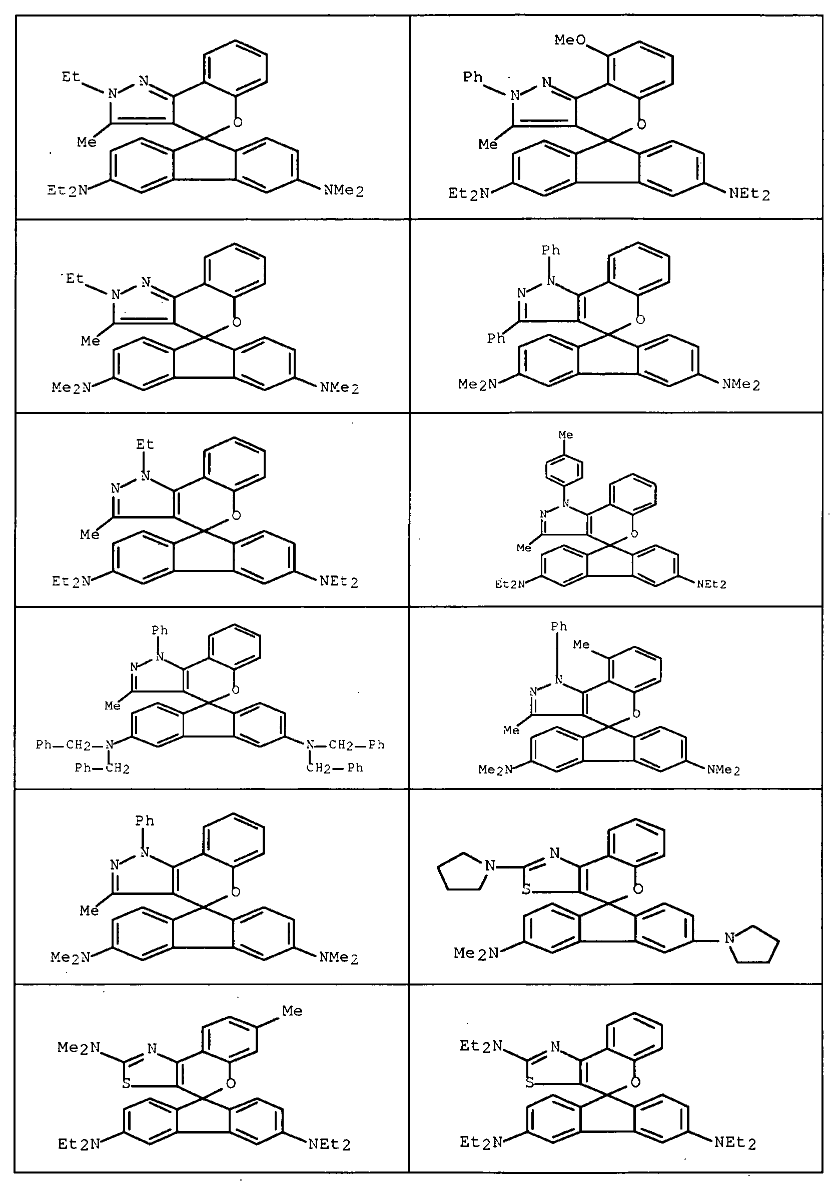

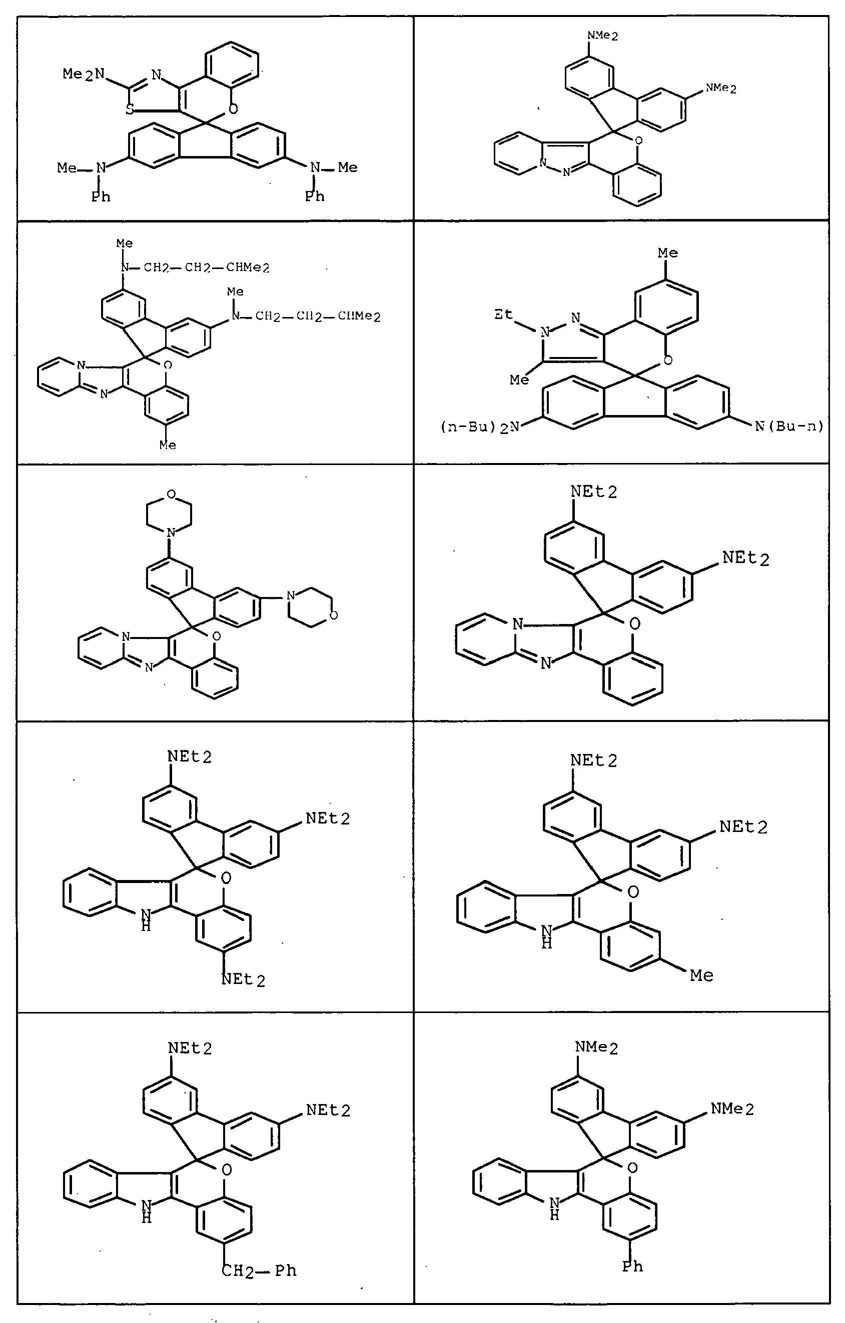

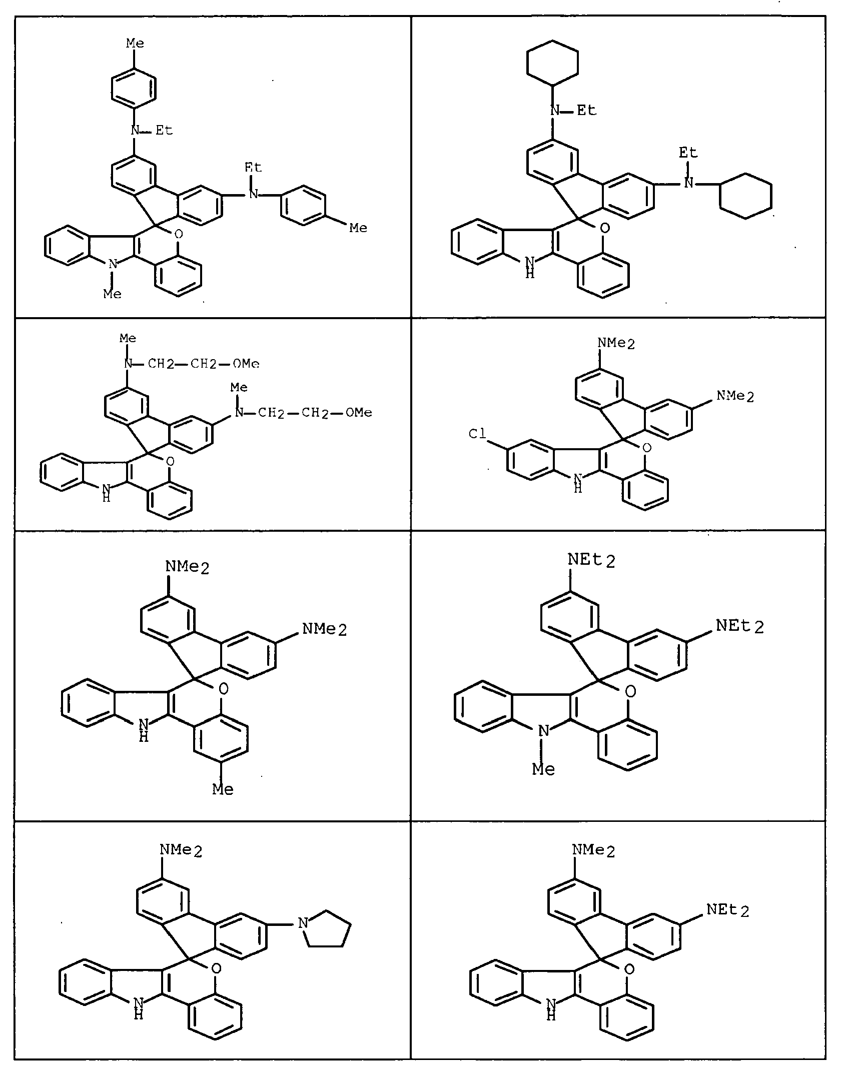

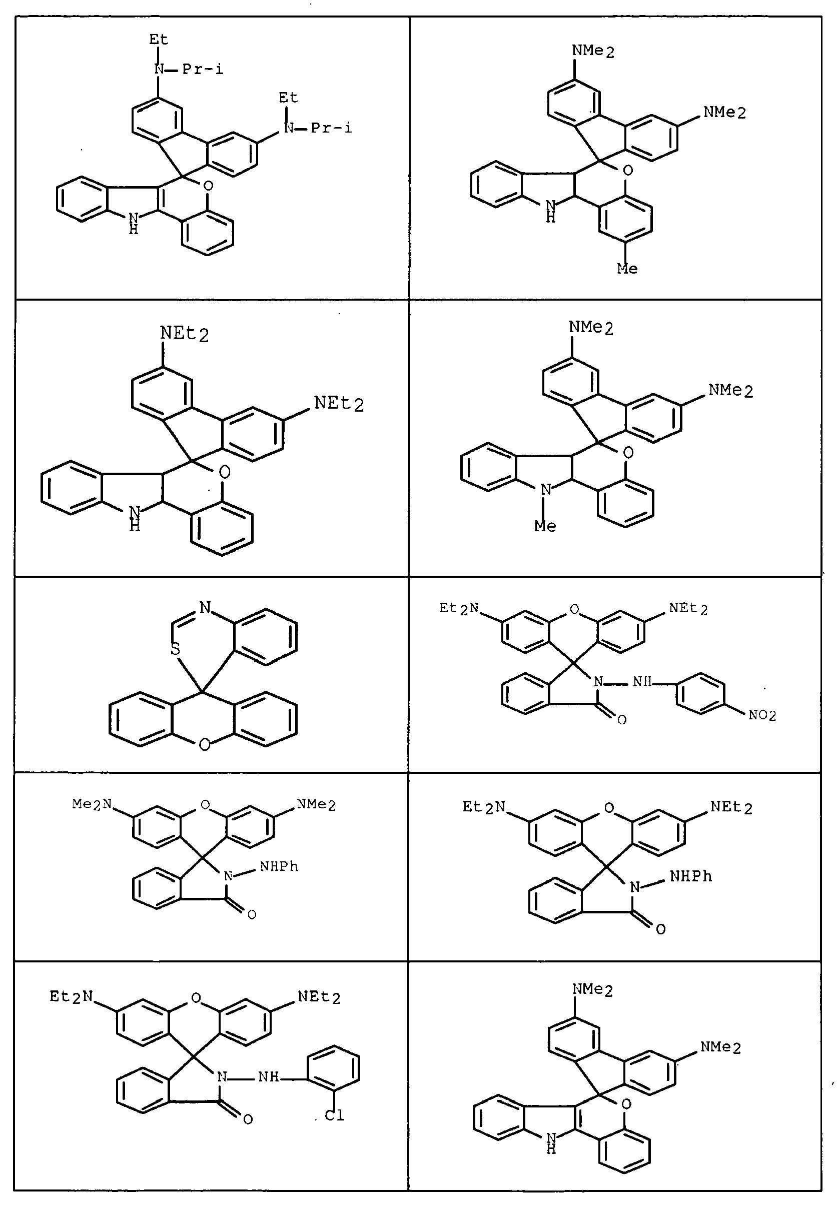

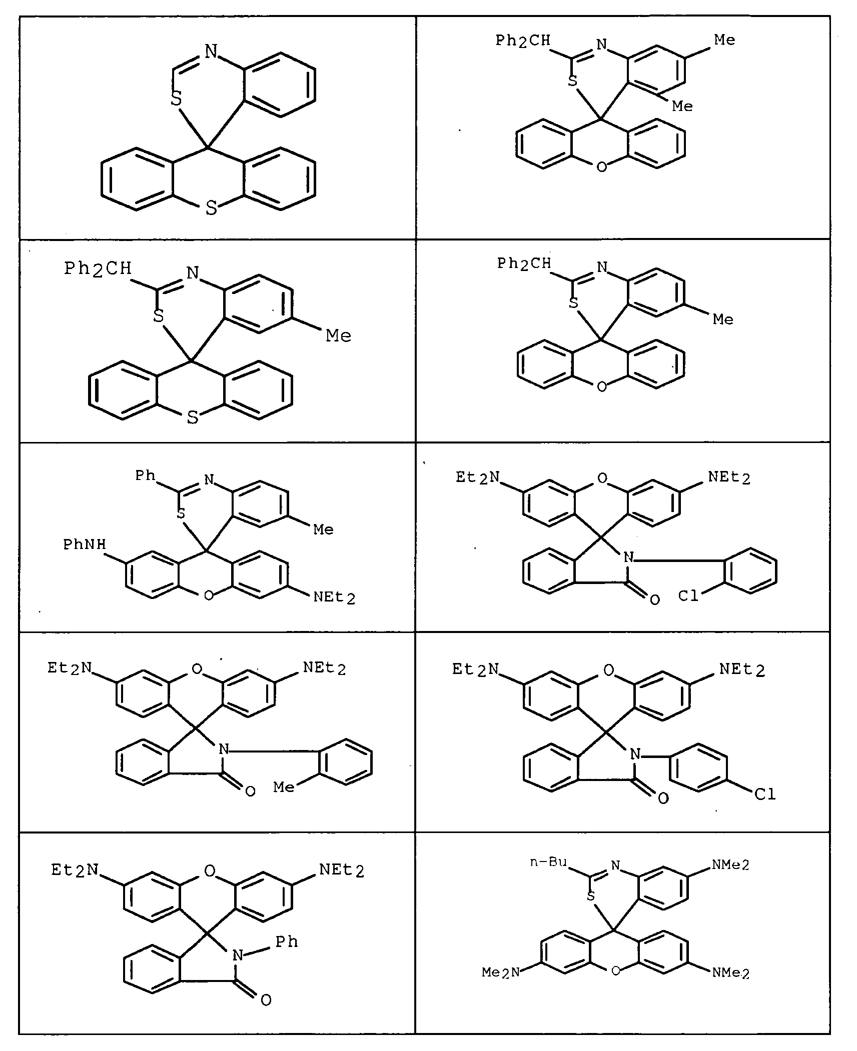

10. Utilisation selon la revendication 9, dans laquelle le composé de formule (I) est choisi parmi les composés suivants :

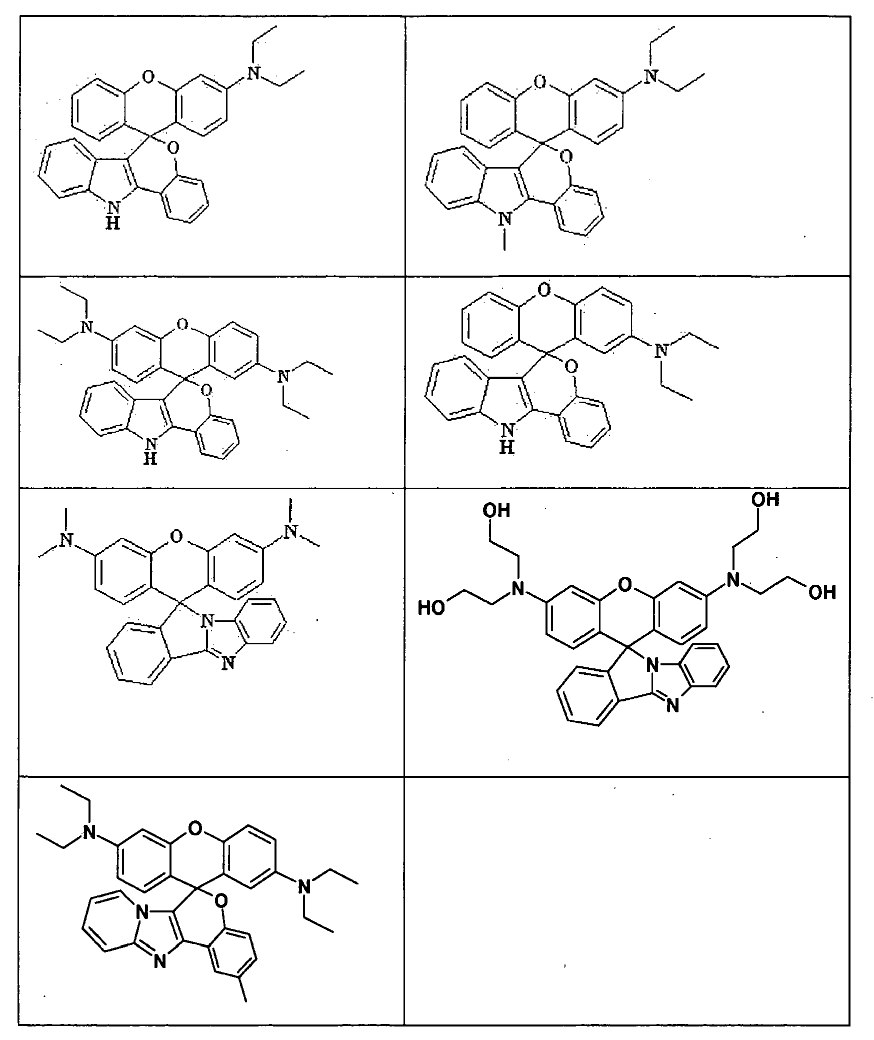

11. Utilisation selon l'une quelconque des revendications précédentes, dans laquelle les composés choisis parmi les composés de formule (I), les colorants correspondant aux composés de formule (I) dont le cycle H est ouvert et leurs sels d'addition représentent de 0,001 à 10 % en poids, rapporté au poids total de la composition.

12. Utilisation selon l'une quelconque des revendications précédentes, dans laquelle le milieu approprié pour la teinture est constitué par de l'eau ou par au moins un solvant organique ou par un mélange d'eau et d'au moins un solvant organique.

13. Utilisation selon la revendication 12, dans laquelle le milieu approprié pour la teinture est constitué par de l'eau ou par un mélange d'eau et d'au moins un solvant organique et le pH est compris entre 3 et 12.

14. Procédé de traitement des fibres kératiniques dans lequel les étapes suivantes sont mises en oeuvre :
a) application d'une composition tinctoriale telle que définie à l'une quelconque des revendications 1 à 13 pendant un temps de pose suffisant, le pH étant ajusté à l'aide d'un agent acidifiant ou d'un agent alcalinisant en fonction de la coloration souhaitée ;
b) éventuellement modification de la coloration des fibres kératiniques à l'aide d'un agent acidifiant ou d'un agent alcalinisant appliqué sur les fibres kératiniques.

15. Procédé selon la revendication 14 dans lequel, dans la première étape, l'agent acidifiant ou l'agent alcalinisant est mélangée à la composition tinctoriale avant application sur les fibres kératiniques.

16. Procédé selon la revendication 14 dans lequel, dans la première étape, l'agent acidifiant ou l'agent alcalinisant est appliqué avant ou après la composition tinctoriale.

17. Procédé selon l'une quelconque des revendications 14 à 16 dans lequel l'agent acidifiant est choisi parmi les acides minéraux tels que l'acide chlorhydrique, l'acide nitrique, l'acide sulfurique ou les acides organiques tels que les composés comprenant au moins une fonction acide carboxylique, une fonction acide sulfonique, une fonction acide phosphonique ou une fonction acide phosphorique.

18. Procédé selon l'une quelconque des revendications 14 à 17 dans lequel l'agent alcalinisant est choisi parmi :
- les acides aminés basiques ;
- les carbonates ou les bicarbonates de métaux alcalins ou alcalino-terreux ;
- les silicates ou les métasilicates ;
- les composés de formule (II) suivante :
X(OH)ₙ (II)
dans laquelle :
• X représente un ion potassium, lithium, sodium, ammonium N⁺R₁₅R₁₆R₁₇R₁₈ avec R₁₅, R₁₆, R₁₇ et R₁₈, identiques ou différents, désignant un radical alkyle en C₂-C₄ lorsque n est égal à 1 ;
• X représente un atome de magnésium ou de calcium lorsque n est égal à 2 ;
- les composés de formule (III) suivante : 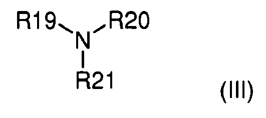 dans laquelle :
• R₁₉ représente un atome d'hydrogène ; une radical alkyle en C₁-C₆, une radical monohydroxyalkyle en C₁-C₆ ; une radical polyhydroxyalkyle en C₂-C₆ ;
• R₂₀ et R₂₁, identiques ou différents, représentent un atome d'hydrogène ; un radical alkyle en C₁-C₆ ; un radical monohydroxyalkyle en C₁-C₆ ; un radical polyhydroxyalkyle en C₂-C₆ ;
- les composés de formule (IV) suivante : 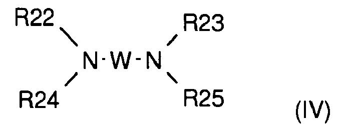 dans laquelle :
• W est un reste propylène éventuellement substitué par un groupement hydroxyle ou un radical alkyle en C₁-C₄ ;
• R₂₂, R₂₃, R₂₄ et R₂₅, identiques ou différents, représentent un atome d'hydrogène, un radical alkyle en C₁-C₄ ou hydroxyalkyle en C₁-C₄.

19. Dispositif à plusieurs compartiments contenant dans un premier compartiment une composition telle que définie à l'une quelconque des revendications 1 à 13 et dans un deuxième compartiment un agent acidifiant ou un agent alcalinisant.

20. Dispositif selon la revendication 19 contenant dans le deuxième compartiment un agent acidifiant et dans un troisième compartiment un agent alcalinisant.

21. Composition telle que définie à l'une quelconque des revendications 1 à 13 comprenant de plus au moins un tensioactif et au moins un polymère.

22. Composition selon la revendication 21, dans laquelle le ou les tensioactifs sont choisis parmi les tensioactifs anioniques, cationiques, non-ioniques, amphotères, zwittérioniques ou leurs mélanges.

23. Composition selon la revendication 21 ou 22, dans laquelle le ou les polymères sont des polymères épaississants associatifs ou non associatifs.

## Claims

1. Use in the colouring of keratinous fibres of a composition comprising, in a medium appropriate for dyeing, at least one compound chosen from the compounds of formula (I) comprising a cyclic group G including a ring H capable of opening, the dyes corresponding to the compounds of formula (I) in which the ring H is open and their addition salts: 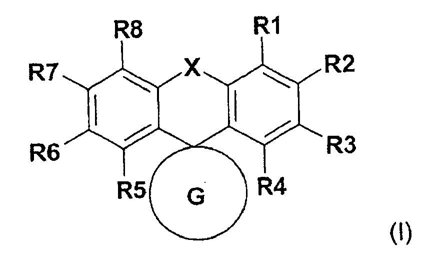 in which:
• **R₁, R₂, R₃, R₄, R₅, R₆, R₇** and **R₈** represent, independently of one another:
- a hydrogen atom;
- a halo radical;
- a hydroxyl radical;
- a nitro radical;
- an amino radical;
- a carboxyl radical;
- an aminocarbonyl radical;
- a cyano radical;
- a radical resulting from a hydrocarbon chain which comprises from 1 to 100 carbon atoms, which is linear or branched, acyclic or mono- or polycyclic, fused or unfused, saturated or unsaturated, aromatic or nonaromatic, which can be interrupted by one or more heteroatoms chosen from nitrogen, oxygen and sulphur atoms or by one or more carbonyl groups, which can be terminated by a hydrocarbonyl group or by a group comprising one or more heteroatoms chosen from nitrogen, oxygen and sulphur atoms, which can begin with a carbonyl group or with a group comprising one or more heteroatoms chosen from nitrogen, oxygen and sulphur atoms, and which can be substituted by one or more groups chosen from the following radicals: hydroxyl, halo, carboxyl, carboxy(C₁-C₉) alkyl, cyano, amino or C₁-C₆ alkoxy;
• it being possible for two of the **R₁, R₂, R₃, R₄, R₅, R₆, R₇** and **R₈** radicals carried by two adjacent carbon atoms to form, together and with the carbon atoms to which they are attached, a fused or unfused, aromatic, mono- or polycarbocyclic group comprising from 5 to 20 ring members, it being possible for one or more carbon atoms to be replaced by an oxygen, nitrogen, sulphur or phosphorus atom, the aromatic mono- or polycarbocyclic group being unsubstituted or substituted by one or more radicals chosen from a halo, hydroxyl, amino, carboxyl or C₆-C₁₈ aryl or cyano radical;
the amino radicals being unsubstituted or substituted by one or two identical or different radicals chosen from a C₁-C₉ alkyl radical; a C₁-C₉ hydroxyalkyl radical; a C₂-C₉ alkenyl radical; a C₆-C₁₂ cycloalkyl radical; a C₆-C₁₈ aryl radical itself optionally substituted by one or more radicals chosen from a halo or C₁-C₉ alkyl radical; a (C₆-C₁₈)arylcarbonyl radical; a cycle (C₆-C₁₂) alkyl (C₁-C₉) alkyl radical; a (C₆-C₁₈) aryl-(C₁-C₉)alkyl radical; a (C₁-C₉)alkylcarbonyl radical; a (C₁-C₉) alkoxycarbonyl (C₁-C₉) alkyl radical; an α-naphthylalkyl radical; a C₁-C₉ haloalkyl radical; a C₁-C₉ cyanoalkyl radical; a C₂-C₁₅ acyl radical; a (C₁-C₉) alkoxycarbonyl radical; a (C₆-C₁₈) aryloxycarbonyl radical; a (C₆-C₁₈)aryloxy(C₁-C₉)alkylcarbonyl radical; a (C₆-C₁₈) aryl (C₁-C₉) alkoxycarbonyl radical; a (C₁-C₉) - alkoxy (C₆-C₁₈) arylcarbonyl radical; a (C₁-C₉)alkoxy-(C₁-C₉)alkylcarbonyl radical; a di (C₁-C₉) alkylaminocarbonyl radical; a di (C₁-C₉) alkylaminosulphonyl radical; a (C₁-C₉) alkyl (C₆-C₁₈) arylsulphonyl radical; a (C₁-C₉) alkylsulphonyl radical; a di(C₁-C₉)alkylamino-(C₁-C₉) alkyl radical; and a (C₁-C₉) alkoxy (C₁-C₉) alkyl radical;
it being possible, when the amino radicals are substituted by two radicals, for the latter to form, with the nitrogen atom of the amino radical, a 5- or 6- membered heterocycle optionally comprising one or more additional heteroatoms;
• **X** represents a direct bond or a divalent atom, such as a sulphur or oxygen atom, or a sulphone SO₂ or C (R₁₃)₂ or NR₁₃ group;
• **G** represents a divalent radical having one of the following formulae: 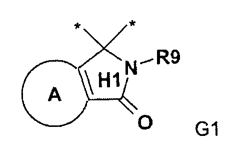 or 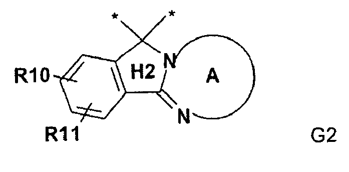 or 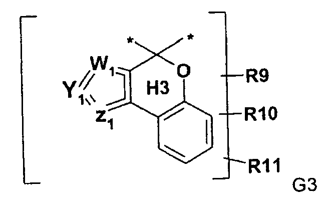 or 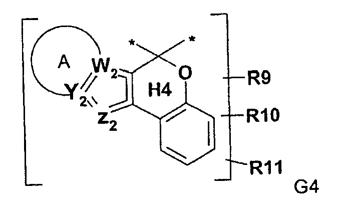 or 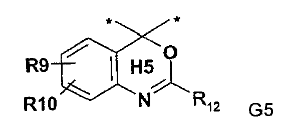 or 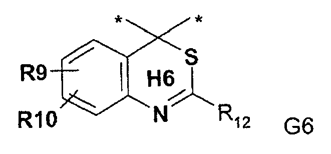 or 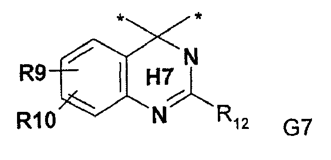
in which:
**Y₁, W₁** and **Z₁,** on the one hand, and **Y₂, W₂** and **Z₂,** on the other hand, represent, independently of one another, a carbon atom, a nitrogen atom, a sulphur atom or a divalent group CR₁₃ or NR₁₃;
**R₉, R₁₀, R₁₁** and **R₁₃** have the same definitions as **R₁;**
**R₁₂** represents:
- a hydrogen atom;
- a C₁-C₉ alkyl radical;
- an amino radical;
- a C₁-C₉ alkoxy radical;
- a C₆-C₁₈ aryl radical which is unsubstituted or substituted by one or more groups chosen from a hydrogen atom, a hydroxyl radical, a C₁-C₉ alkyl radical, a C₆-C₁₈ aryl radical, a C₆-C₁₈ aryloxy radical, a C₁-C₉ alkoxy radical, a halo radical, a carboxyl radical, a cyano radical or an amino radical which are substituted or unsubstituted;
- a furanyl radical;
- a (C₁-C₉)alkylthio radical;
- a thienyl radical;
- a phenylcarbonyl radical;
- a trifluoroalkyl radical;
- a di(C₆-C₁₈)aryl(C₁-C₉)alkyl radical;
**A** represents a C₆-C₁₈ aryl group or a heterocyclic group which is saturated or unsaturated and substituted or unsubstituted, comprising from 5 to 12 ring members.

2. Use according to Claim 1, in which the compound of formula (I) is such that:
• R₁, R₄, R₅ and R₈ denote a hydrogen atom;
• R₂ and R₇ denote, independently of one another, a hydrogen atom; a halo radical; a C₁-C₉ alkyl radical; a C₁-C₉ alkoxy radical; a substituted or unsubstituted amino radical;
• R₃ and R₆ denote, independently of one another, a hydrogen atom; a halo radical; a C₁-C₉ alkyl radical; a substituted or unsubstituted amino radical;
• X represents a direct bond or a sulphur or oxygen atom or an SO₂ group;
• G represents a divalent radical of formula G1, G2, G3, G4, G5 or G6.

3. Use according to Claim 1 or 2, in which the compound of formula (I) is such that G represents a G1 group; X represents an oxygen atom; R₁, R₃, R₄, R₅, R₆ and R₈ denote a hydrogen atom; R₂ and R₇ denote, independently of one another, a substituted or unsubstituted amino radical; R₉ denotes a substituted or unsubstituted aryl radical or a substituted or unsubstituted amino radical; A denotes a substituted or unsubstituted benzene ring.

4. Use according to Claim 1 or 2, in which the compound of formula (I) is such that G represents a G2 group; X represents an oxygen atom; R₁, R₃, R₄, R₅, R₆, R₈, R₁₀ and R₁₁ denote a hydrogen atom; R₂ and R₇ denote, independently of one another, a substituted or unsubstituted amino radical; A represents a substituted or unsubstituted benzene ring.

5. Use according to Claim 1 or 2, in which the compound of formula (I) is such that G represents a G3 group; R₁, R₃, R₄, R₅, R₆, R₈, R₁₀ and R₁₁ denote a hydrogen atom; R₂ or R₇ denote, independently of one another, a hydrogen atom or a substituted or unsubstituted amino group; R₉ denotes a hydrogen atom, a substituted or unsubstituted alkyl group or an alkoxy group; X represents a direct bond; W₁ represents a sulphur atom, a carbon atom substituted by a hydrogen atom, a C₁-C₄ alkyl radical, a phenyl radical, an amino group carrying two C₁-C₄ alkyl groups, the two radicals optionally forming, with the nitrogen atom carrying them, a saturated 5-membered heterocycle; Y₁ represents a carbon atom substituted by a hydrogen atom, a C₁-C₄ alkyl radical, a phenyl radical, an amino group carrying two C₁-C₄ alkyl groups, the two radicals optionally forming, with the nitrogen atom carrying them, a saturated 5-membered heterocycle, a nitrogen atom, a nitrogen atom substituted by a hydrogen atom, a C₁-C₂ alkyl radical, a phenyl radical, a phenylalkyl group, the alkyl group of which is a C₁-C₄ alkyl group; Z₁ represents a nitrogen atom, an NH group, a nitrogen atom substituted by a hydrogen atom, a C₁-C₂ alkyl radical, a phenyl radical, a phenylalkyl group, the alkyl group of which is a C₁-C₄ alkyl group.

6. Use according to Claim 1 or 2, in which the compound of formula (I) is such that G represents a G4 group; R₁, R₄, R₅, R₈ and R₁₁ denote a hydrogen atom; R₂ or R₇ denote, independently of one another, a hydrogen atom, a halogen atom, an alkoxy group, a substituted or unsubstituted amino group; R₃ and R₆ denote, independently of one another, a hydrogen atom, a halogen atom, a substituted or unsubstituted amino group; R₉ denotes a hydrogen atom, a substituted or unsubstituted alkyl group, an alkoxy group, an aryl group, a substituted or unsubstituted amino group; R₁₀ denotes a hydrogen atom or an alkyl radical; A represents a substituted or unsubstituted benzene ring or a substituted or unsubstituted pyridine ring; X represents an oxygen or sulphur atom, a direct bond or an SO₂ group; W₂ represents a carbon atom or a nitrogen atom; Y₂ represents a carbon atom or a nitrogen atom; Z₂ represents a CH group, a carbon atom substituted by a hydrogen atom, a C₁-C₄ alkyl radical, a phenyl radical, an amino group carrying two C₁-C₄ alkyl groups, the two radicals optionally forming, with the nitrogen atom carrying them, a saturated 5-membered heterocycle, a nitrogen atom, an NH group, a nitrogen atom substituted by a hydrogen atom, a C₁-C₂ alkyl radical, a phenyl radical, a phenylalkyl group, the alkyl group of which is a C₁-C₄ alkyl group.

7. Use according to Claim 1 or 2, in which the compound of formula (I) is such that G represents a G5 group, X represents an oxygen atom or a direct bond; R₁, R₄, R₅, R₈ and R₁₀ denote a hydrogen atom; R₂ and R₇ denote, independently of one another, an alkyl radical, an alkoxy radical, a substituted or unsubstituted amino radical; R₃ and R₆ denote, independently of one another, a hydrogen atom, an alkyl radical, a substituted or unsubstituted amino radical; R₉ denotes a hydrogen atom or a substituted or unsubstituted amino radical; R₁₂ denotes a substituted or unsubstituted alkyl radical, a substituted or unsubstituted aryl radical, a thienyl radical, a furanyl radical.

8. Use according to Claim 1 or 2, in which the compound of formula (I) is such that G represents a G6 group; X represents a direct bond or an oxygen atom; R₁, R₃, R₄, R₅, R₆, R₈ and R₁₀ denote a hydrogen atom, R₂, R₉ and R₇ denote, independently of one another, a substituted or unsubstituted amino group; R₁₂ denotes an alkyl radical.

9. Use according to any one of the preceding claims, in which the compound of formula (I) is chosen from the following compounds:

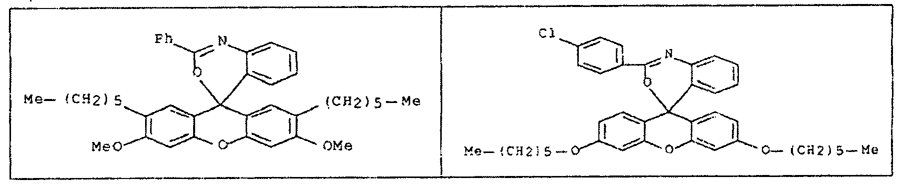

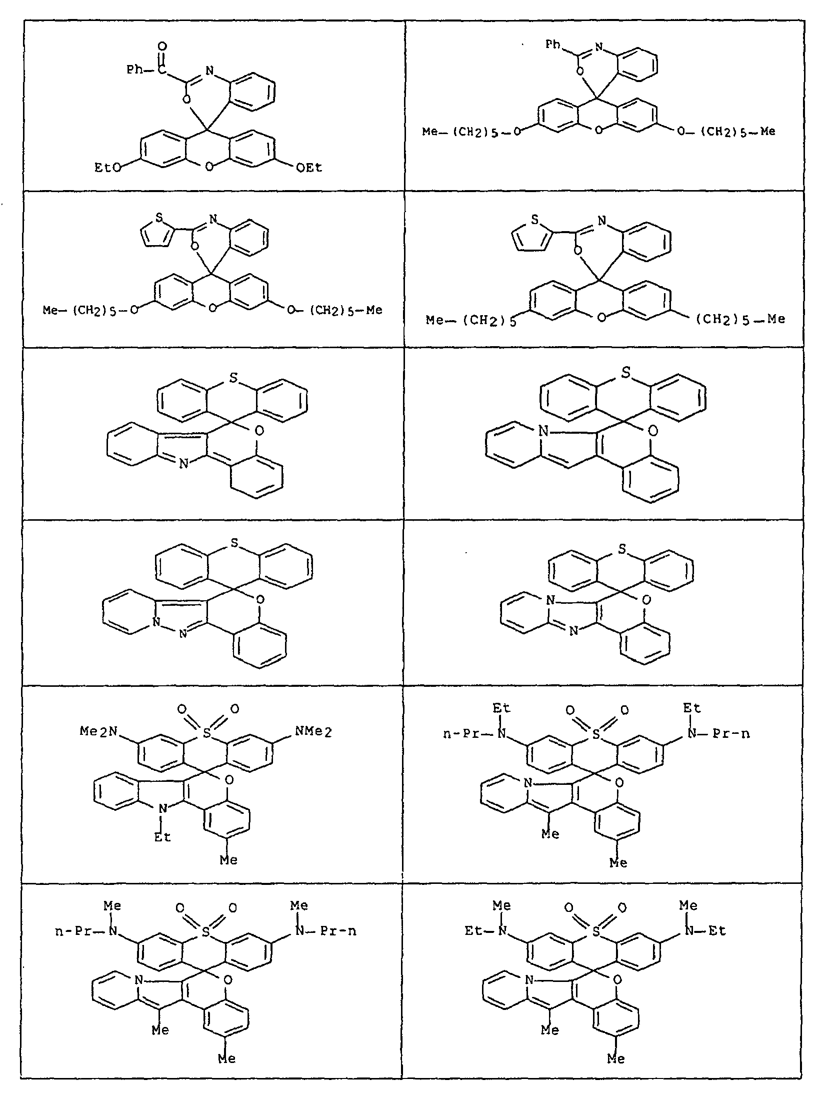

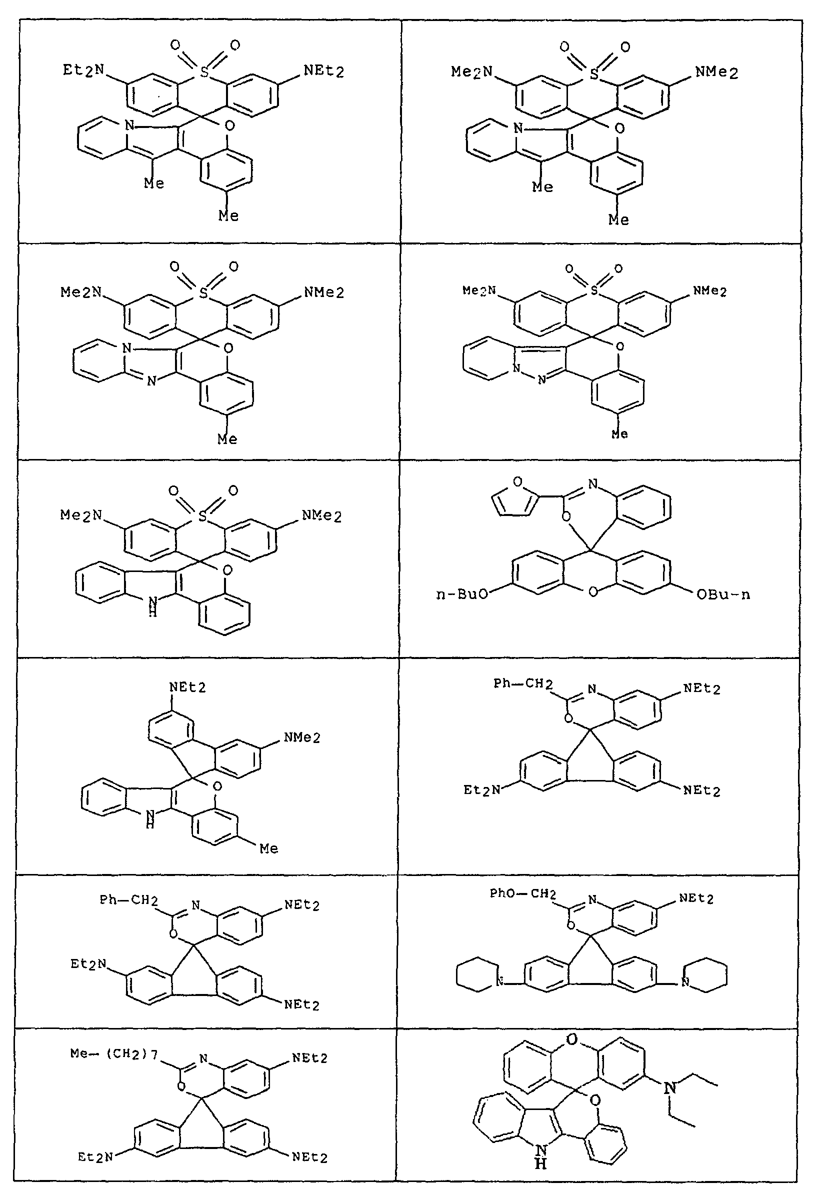

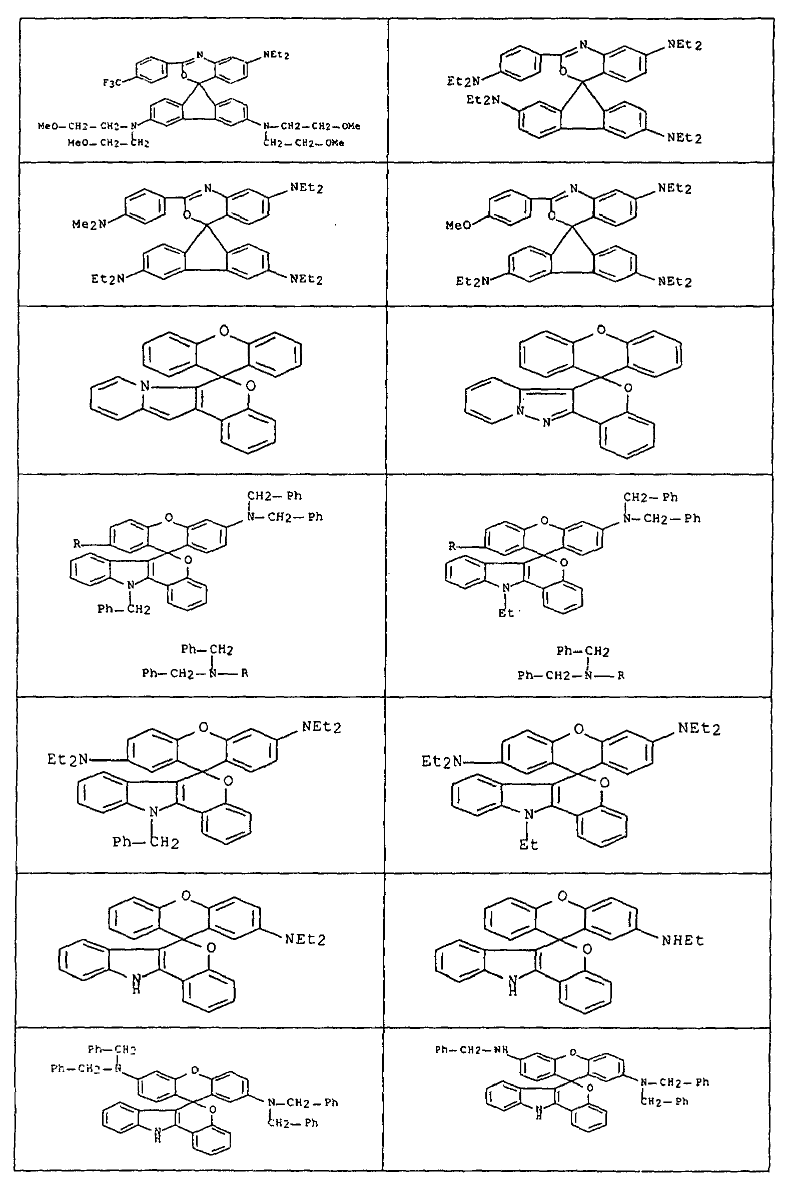

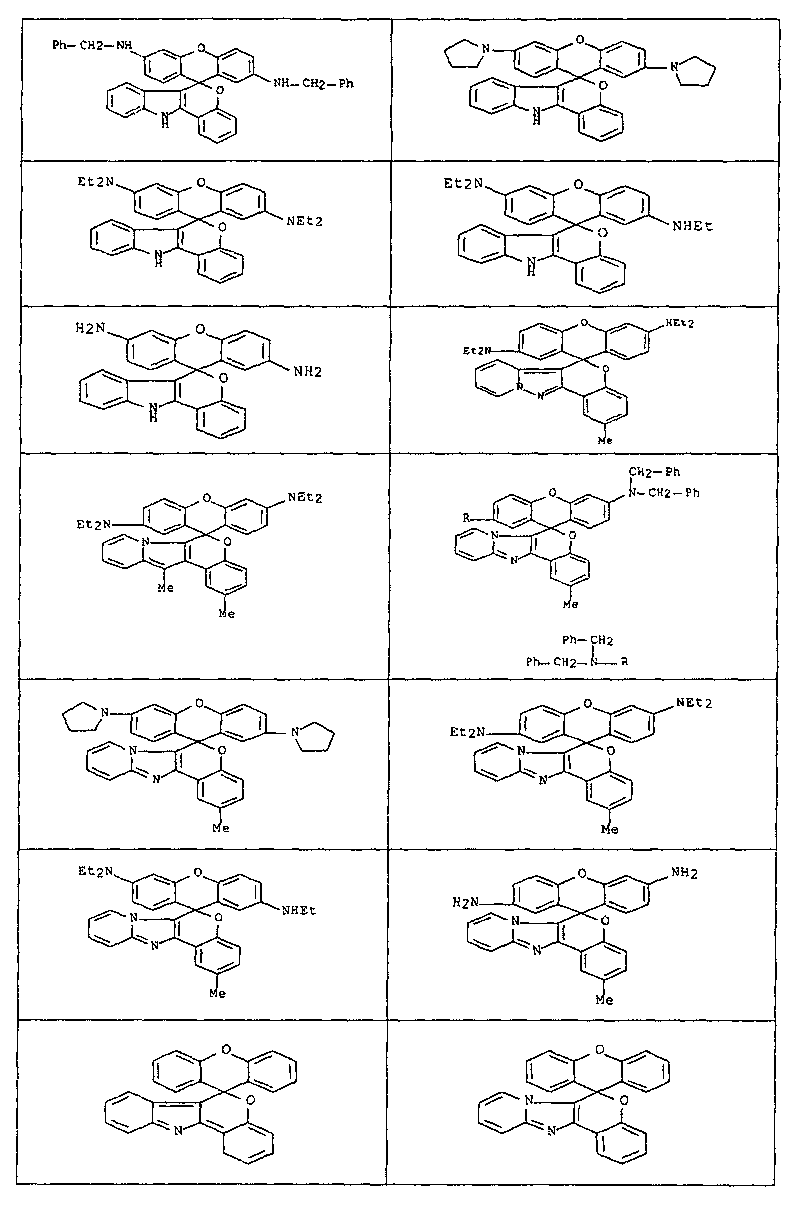

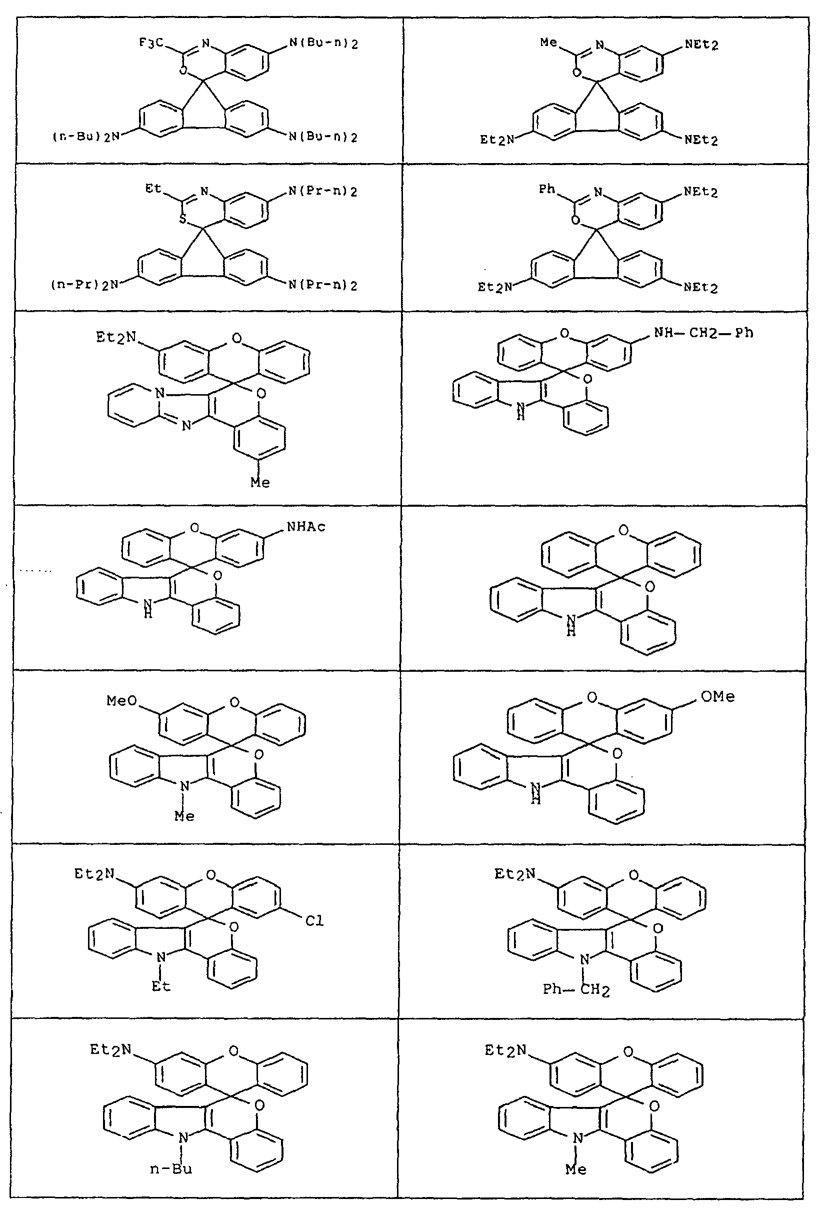

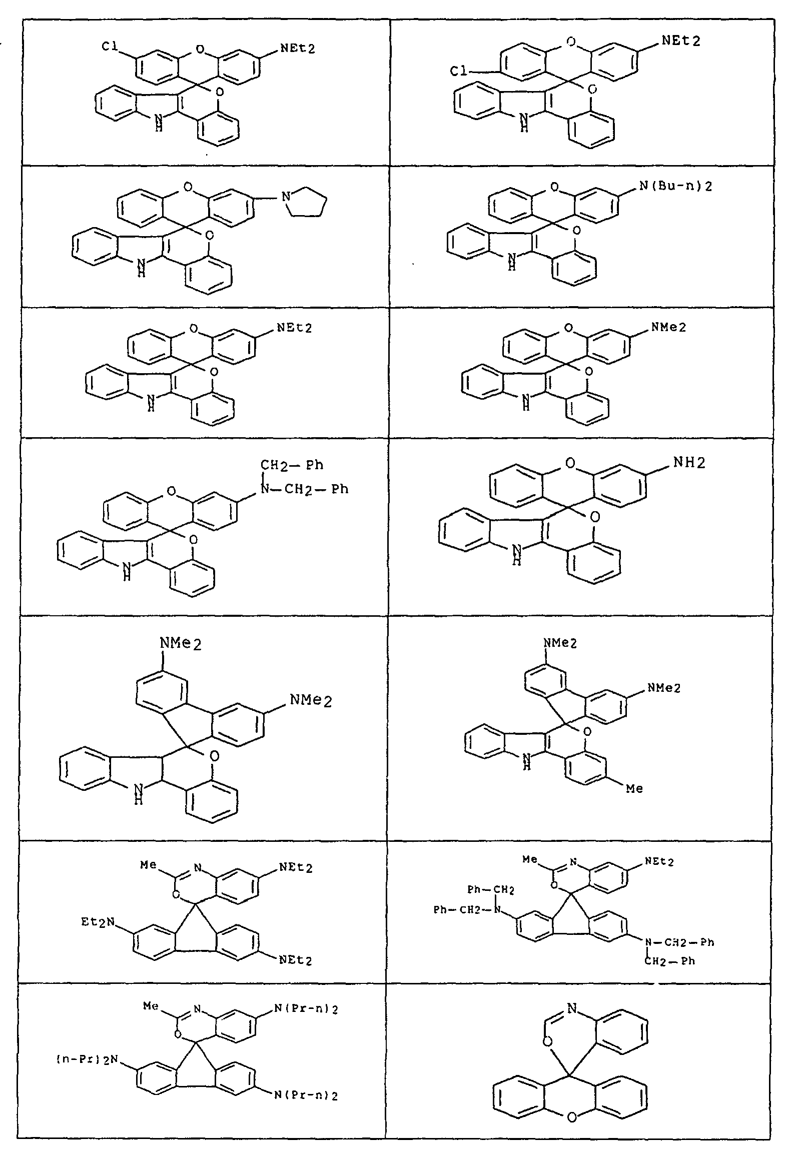

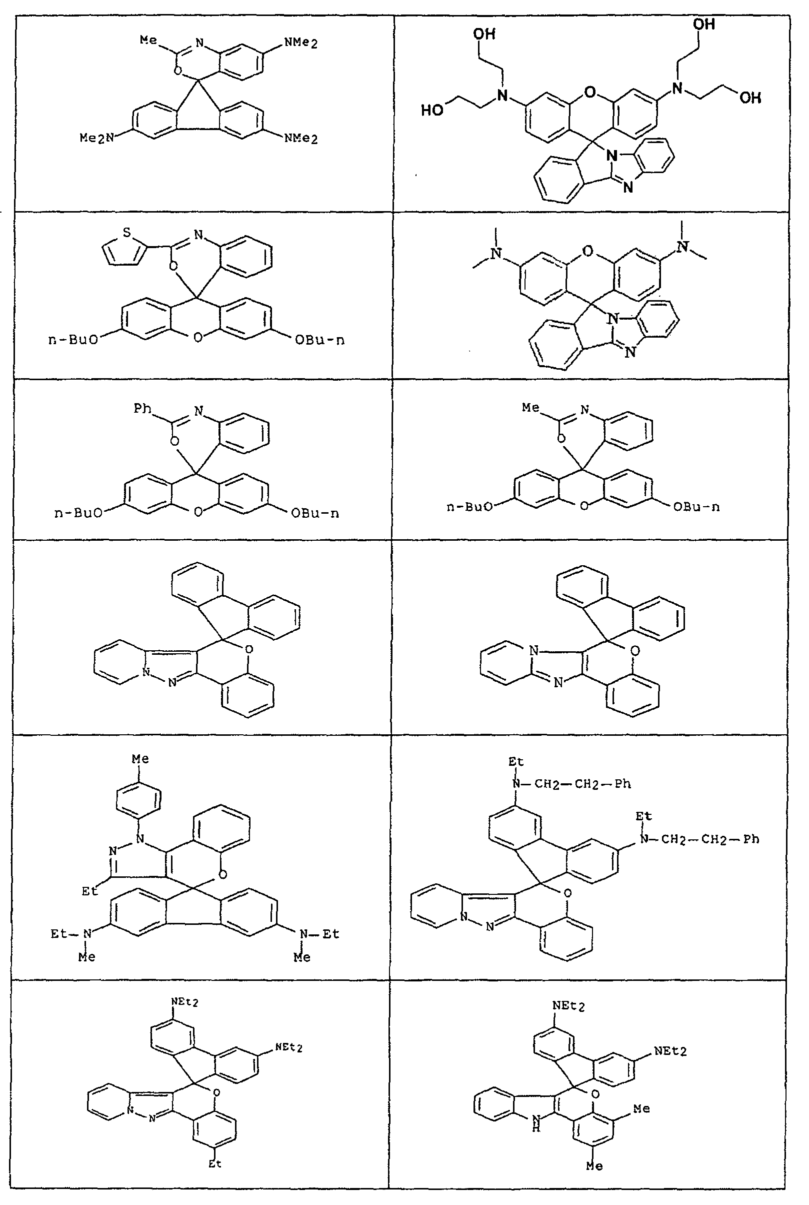

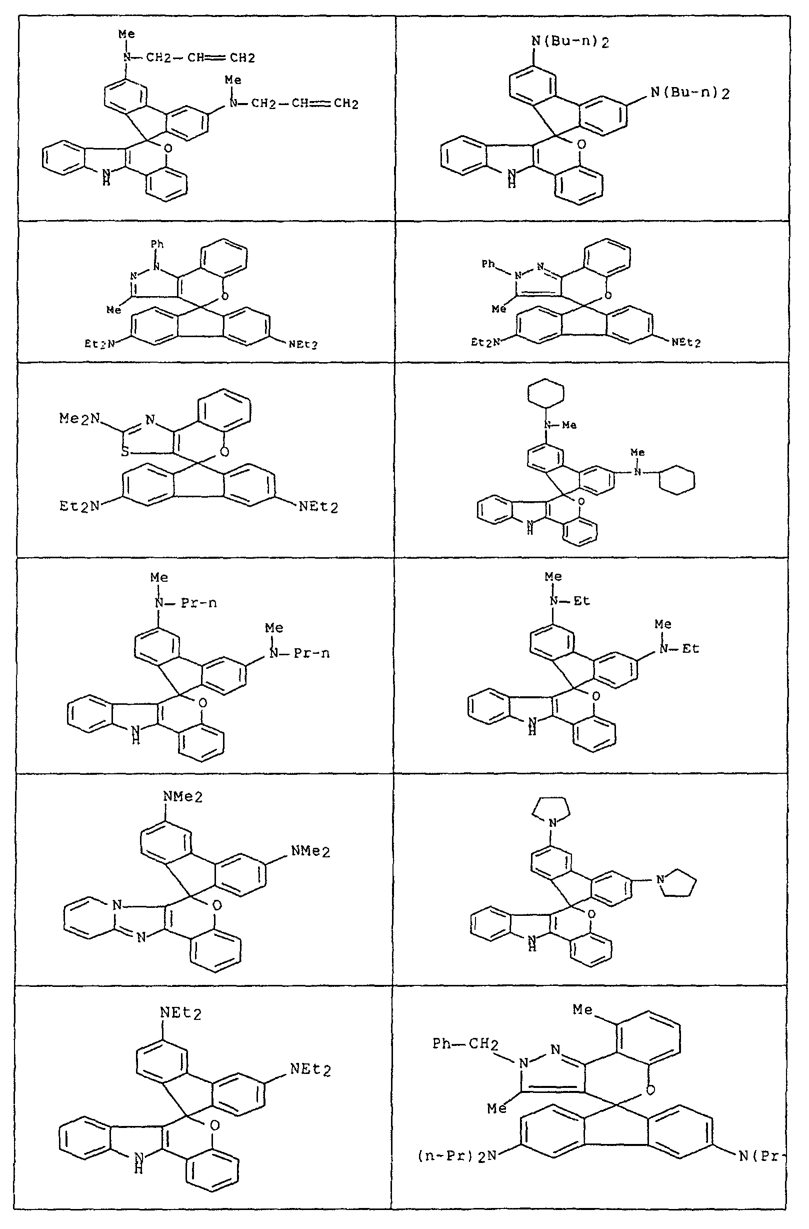

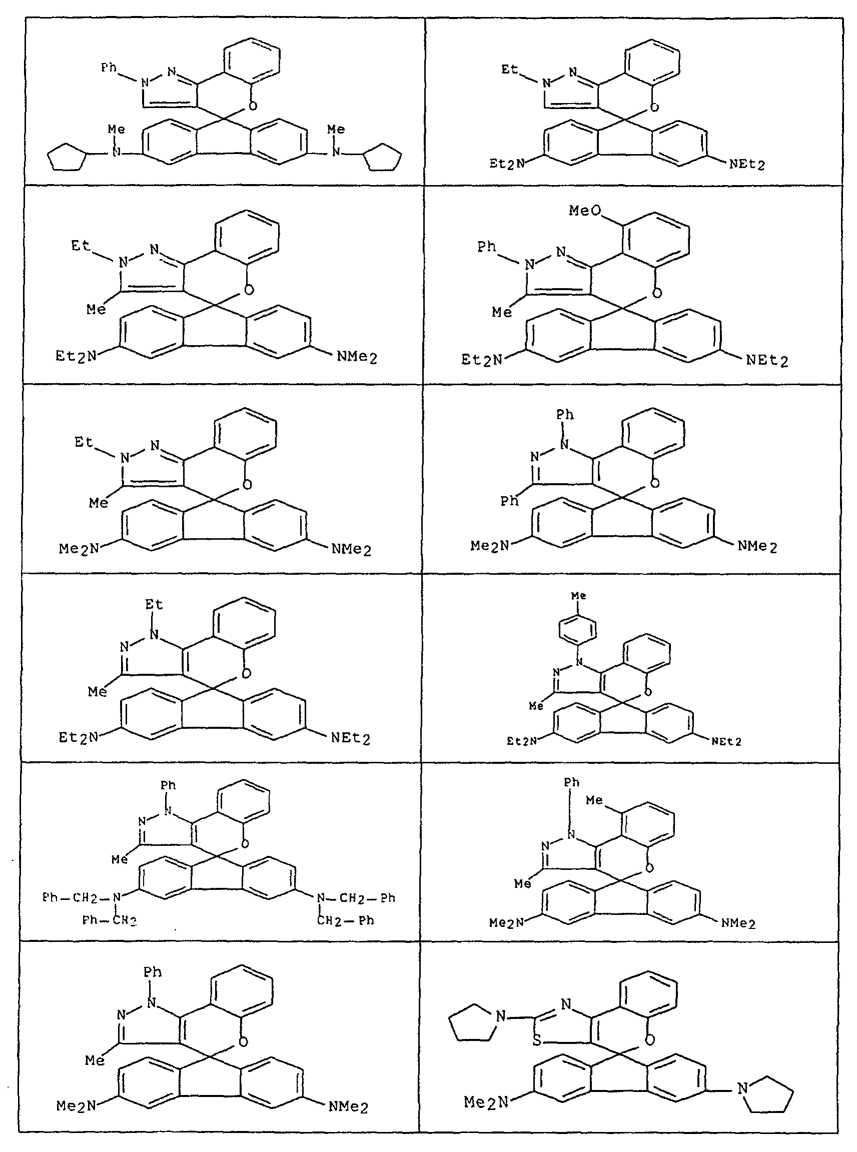

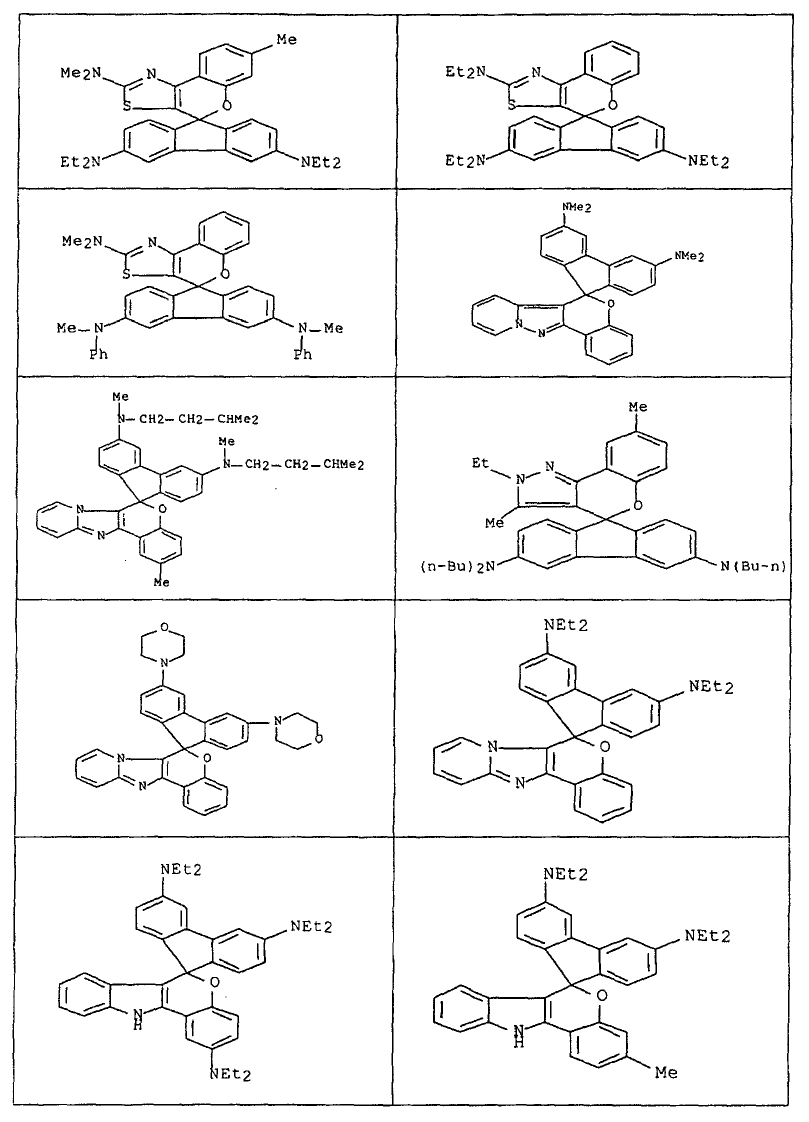

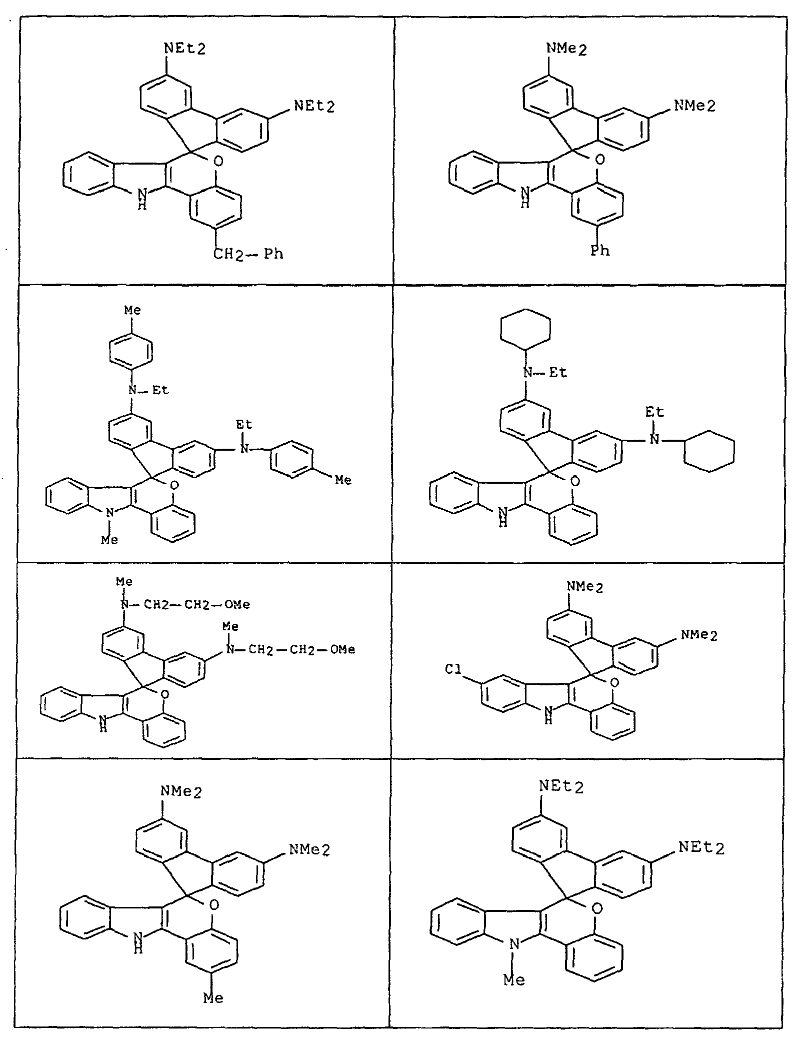

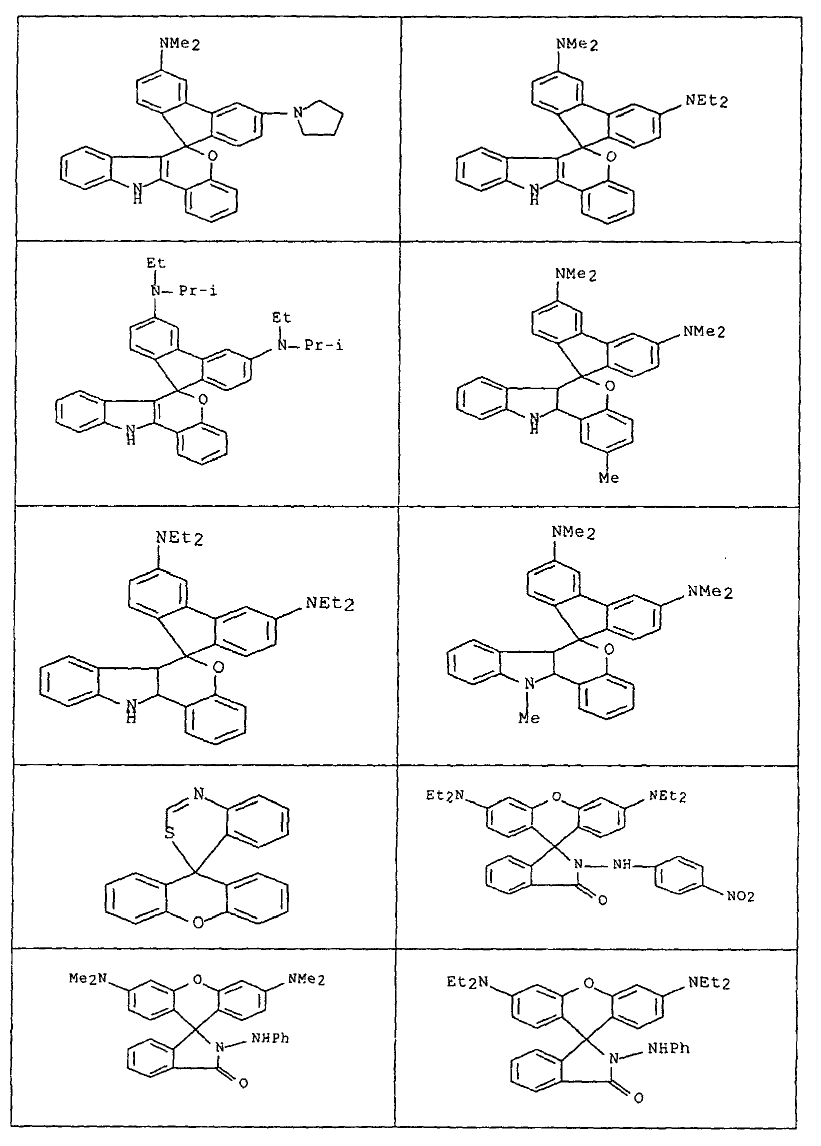

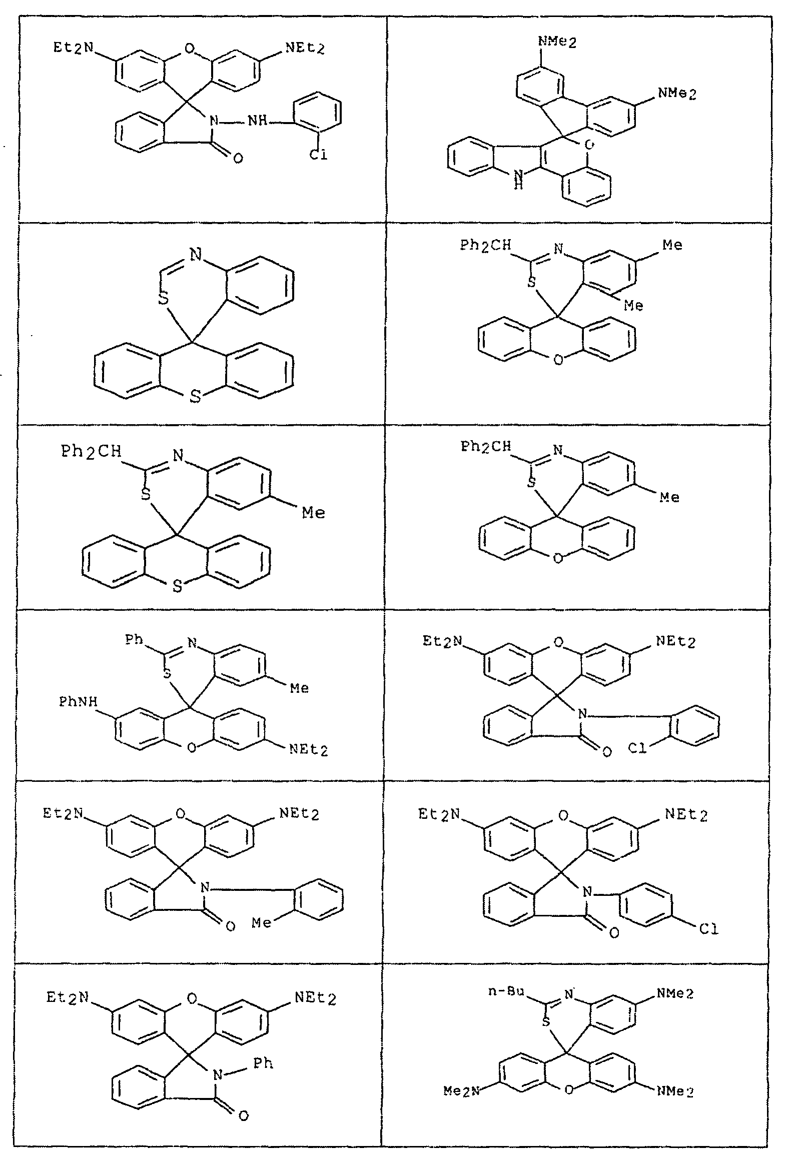

10. Use according to Claim 9, in which the compound of formula (I) is chosen from the following compounds:

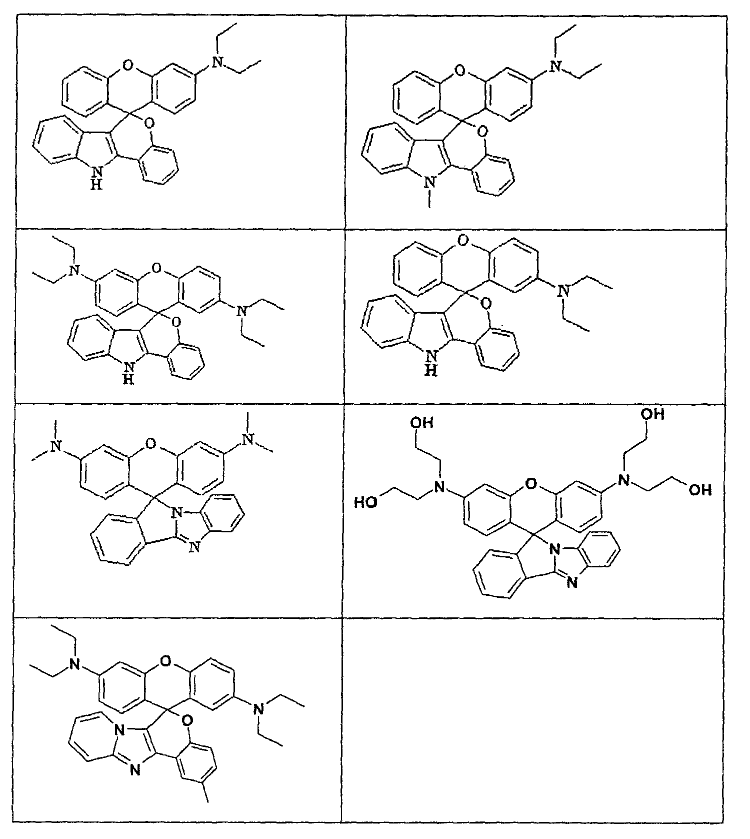

11. Use according to any one of the preceding claims, in which the compounds chosen from the compounds of formula (I), the dyes corresponding to the compounds of formula (I) in which the ring H is open and their addition salts represent from 0.001 to 10% by weight, with respect to the total weight of the composition.

12. Use according to any one of the preceding claims, in which the medium appropriate for dyeing is composed of water or of at least one organic solvent or of a mixture of water and of at least one organic solvent.

13. Use according to Claim 12, in which the medium appropriate for dyeing is composed of water or of a mixture of water and of at least one organic solvent and the pH is between 3 and 12.

14. Method for the treatment of keratinous fibres, in which the following stages are carried out:
a) application of a dyeing composition as defined in any one of Claims 1 to 13 for a sufficient development time, the pH being adjusted using an acidifying agent or a basifying agent according to the colouring desired;
b) optionally modification of the colouring of the keratinous fibres using an acidifying agent or a basifying agent applied to the keratinous fibres.

15. Method according to Claim 14, in which, in the first stage, the acidifying agent or the basifying agent is mixed with the dyeing composition before application to the keratinous fibres.

16. Method according to Claim 14, in which, in the first stage, the acidifying agent or the basifying agent is applied before or after the dyeing composition.

17. Method according to any one of Claims 14 to 16, in which the acidifying agent is chosen from inorganic acids, such as hydrochloric acid, nitric acid or sulphuric acid, or organic acids, such as compounds comprising at least one carboxylic acid functional group, one sulphonic acid functional group, one phosphonic acid functional group or one phosphoric acid functional group.

18. Method according to any one of Claims 14 to 17, in which the basifying agent is chosen from:
- basic amino acids;
- alkali metal or alkaline earth metal carbonates or bicarbonates;
- silicates or metasilicates;
- compounds of following formula (II):
X(OH)ₙ (II)
in which:
• X represents a potassium, lithium, sodium, ammonium or N⁺R₁₅R₁₆R₁₇R₁₈ ion with R₁₅, R₁₆, R₁₇ and R₁₈, which are identical or different, denoting a C₂-C₄ alkyl radical, when n is equal to 1;
• X represents a magnesium or calcium atom, when n is equal to 2;
- compounds of following formula (III): 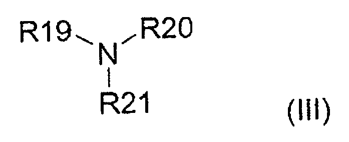 in which:
• R₁₉ represents a hydrogen atom, a C₁-C₆ alkyl radical, a C₁-C₆ monohydroxyalkyl radical or a C₂-C₆ polyhydroxyalkyl radical;
• R₂₀ and R₂₁, which are identical or different, represent a hydrogen atom, a C₁-C₆ alkyl radical, a C₁-C₆ monohydroxyalkyl radical or a C₂-C₆ polyhydroxyalkyl radical;
- compounds of following formula (IV): 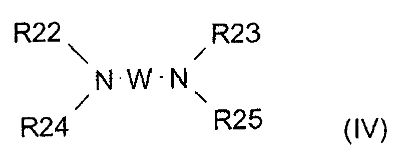 in which:
• W is a propylene residue optionally substituted by a hydroxyl group or a C₁-C₄ alkyl radical;
• R₂₂, R₂₃, R₂₄ and R₂₅, which are identical or different, represent a hydrogen atom, a C₁-C₄ alkyl radical or a C₁-C₄ hydroxyalkyl radical.

19. Multicompartment device comprising, in a first compartment, a composition as defined in any one of Claims 1 to 13 and, in a second compartment, an acidifying agent or a basifying agent.

20. Device according to Claim 19, comprising, in the second compartment, an acidifying agent, and in a third compartment, a basifying agent.

21. Composition as defined in any one of Claims 1 to 13, additionally comprising at least one surfactant and at least one polymer.

22. Composition according to Claim 21, in which the surfactant or surfactants are chosen from anionic, cationic, non-ionic, amphoteric or zwitterionic surfactants or their mixtures.

23. Composition according to Claim 21 or 22, in which the polymer or polymers are associative or non-associative thickening polymers.

## Patentansprüche

1. Verwendung einer Zusammensetzung, die in einem zum Färben geeigneten Medium mindestens eine Verbindung enthält, die unter den Verbindungen der Formel (I), die eine cyclische Gruppe G enthalten, welche einen Ring H umfasst, der zur Öffnung befähigt ist, und den Farbstoffen, die den Verbindungen der Formel (I) entsprechen, deren Ring H geöffnet ist, und ihren Additionssalzen ausgewählt ist, zum Färben von Keratinfasern: 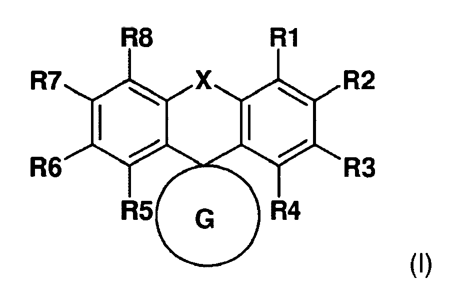 in der Formel:
· die Gruppen R₁, R₂, R₃, R₄, R₅, R₆, R₇ und R₈ bedeuten unabhängig voneinander:
- ein Wasserstoffatom;
- eine Halogenogruppe;
- eine Hydroxygruppe,
- eine Nitrogruppe;
- eine Aminogruppe;
- eine Carboxygruppe;
- eine Aminocarbonylgruppe;
- eine Cyanogruppe;
- eine Gruppe, die von einer geradkettigen oder verzweigten, acyclischen oder mono- oder polycyclischen, kondensierten oder nicht kondensierten, gesättigten oder ungesättigten, aromatischen oder nichtaromatischen Kohlenwasserstoffkette mit 1 bis 100 Kohlenstoffatomen abgeleitet ist, die durch ein oder mehrere Heteroatome, die unter Stickstoff, Sauerstoff und Schwefel ausgewählt sind, oder durch eine oder mehrere Carbonylgruppen unterbrochen sein kann, die durch eine Hydrogenocarbonylgruppe oder durch eine Gruppe abgeschlossen werden kann, die ein der mehrere Heteroatome enthält, die unter Stickstoff, Sauerstoff und Schwefel ausgewählt sind, die mit einer Carbonylgruppe oder mit einer Gruppe beginnen kann, die ein der mehrere Heteroatome enthält, die unter Stickstoff, Sauerstoff und Schwefel ausgewählt sind, und die mit einer oder mehreren Gruppen substituiert sein kann, die unter den Gruppen Hydroxy, Halogeno, Carboxy, Carboxyalkyl(C₁₋₉), Cyano, Amino und Alkoxy(C₁₋₆) ausgewählt sind;
• zwei der Gruppen R₁, R₂, R₃, R₄, R₅, R₆, R₇ und R₈, die von zwei angrenzenden Kohlenstoffatomen getragen werden, können gemeinsam und mit den Kohlenstoffatomen, an die sie gebunden sind, eine aromatische, kondensierte oder nicht kondensierte 5- bis 20-gliedrige, mono- oder polycarbocyclische Gruppe bilden, wobei ein oder mehrere Kohlenstoffatome durch ein Sauerstoffatom, Stickstoffatom, Schwefelatom oder Phosphoratom ersetzt sein können, wobei die mono- oder polycarbocyclische aromatische Gruppe unsubstituiert vorliegt oder mit einer oder mehreren Gruppen substituiert ist, die unter Halogeno, Hydroxy, Amino, Carboxy, Aryl(C₆₋₁₈) und Cyano ausgewählt sind; wobei die Aminogruppen unsubstituiert sind oder mit einer oder mit zwei gleichen oder voneinander verschiedenen Gruppen substituiert sind, die unter einer Alkyl(C₁₋₉)gruppe; einer Hydroxyalkyl(C₁₋₉)gruppe; einer Alkenyl(C₂₋₉)gruppe; einer Cycloalkyl(C₆₋₁₂)gruppe; einer Aryl(C₆₋₁₈)gruppe, die gegebenenfalls selbst mit einer oder mehreren Gruppen substituiert sein kann, die unter den Gruppen Halogeno und Alkyl(C₁₋₉) ausgewählt sind; einer Aryl(C₆₋₁₈)carbonylgruppe; einer Cycloalkyl(C₆₋₁₂)-alkyl(C₁₋₉)gruppe; einer Aryl(C₆₋₁₈)alkyl(C₁₋₉)gruppe; einer Alkyl(C₁₋₉)carbonylgruppe; einer Alkoxy(C₁₋₉)carbonylalkyl(C₁₋₉)-gruppe; einer α-Naphthylalkylgruppe; einer Halogenalkyl(C₁₋₉)-gruppe; einer Cyanoalkyl(C₁₋₉)gruppe; einer Acyl(C₂₋₁₅)gruppe; einer Alkoxy(C₁₋₉)carbonylgruppe; einer Aryloxy(C₆₋₁₈)-carbonylgruppe; einer Aryloxy(C₆₋₁₈)alkyl(C₁₋₉)carbonylgruppe; einer Aryl(C₆₋₁₈)alkoxy(C₁₋₉)carbonylgruppe; einer Alkoxy(C₁₋₉)-aryl(C₆₋₁₈)gruppe; einer Alkoxy(C₁₋₉)alkyl(C₁₋₉)carbonylgruppe; einer Dialkyl(C₁₋₉)aminocarbonylgruppe; einer Dialkyl(C₁₋₉)-aminosulfonylgruppe; einer Alkyl(C₁₋₉)aryl(C₆₋₁₈)sulfonylgruppe; einer Alkyl(C₁₋₉)sulfonylgruppe; einer Dialkyl(C₁₋₉)aminoalkyl(C₁₋₉) gruppe; einer Alkoxy(C₁₋₉)alkyl(C₁₋₉)gruppe ausgewählt sind; wenn die Aminogruppen mit zwei Gruppen substituiert sind, können diese mit dem Stickstoffatom der Aminogruppe einen 5- oder 6-gliedrigen Heterocyclus bilden, der gegebenenfalls ein oder mehrere weitere Heteroatome enthält;
• X bedeutet eine direkte Bindung oder ein zweiwertiges Atom, beispielsweise ein Schwefelatom oder ein Sauerstoffatom, oder eine Sulfongruppe SO₂ oder C(R₁₃)₂ oder NR₁₃;
• G bedeutet eine zweiwertige Gruppe einer der folgenden Formeln: 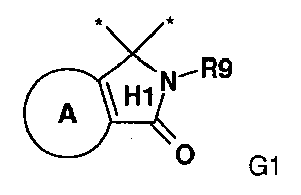 oder 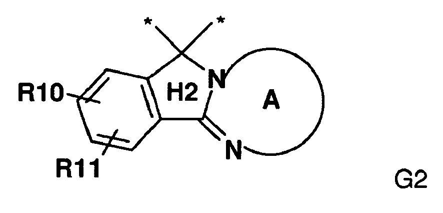 oder 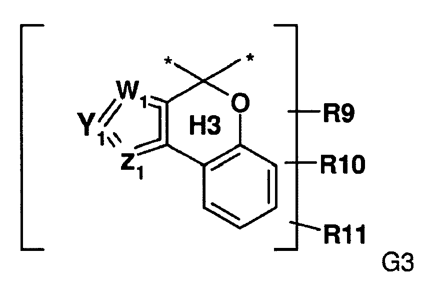 oder 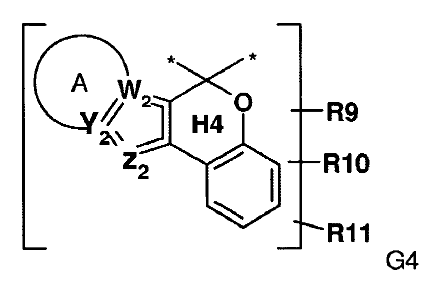 oder 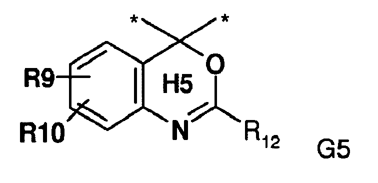 oder 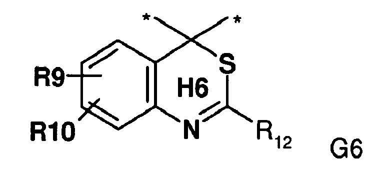 oder 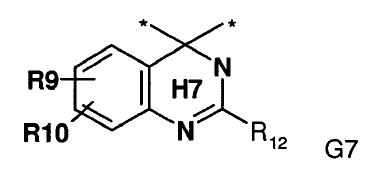
worin bedeuten:
Y₁, W₁ und Z₁ einerseits und Y₂, W₂ und Z₂ andererseits unabhängig voneinander ein Kohlenstoffatom, ein Stickstoffatom, ein Schwefelatom oder eine zweiwertige Gruppe CR₁₃ oder NR₁₃:
R₉, R₁₀, R₁₁ und R₁₃ die für R₁ angegebenen Bedeutungen;
R₁₂ bedeutet:
- ein Wasserstoffatom;
- eine Alkyl(C₁₋₉)gruppe;
- eine Aminogruppe;
- eine Alkoxy(C₁₋₉)gruppe;
- eine Aryl(C₆₋₁₈)gruppe, die unsubstituiert vorliegt oder mit einer oder mehreren Gruppen substituiert ist, die unter einem Wasserstoffatom, einer Hydroxygruppe, einer Alkyl(C₁₋₉)-gruppe, einer Aryl(C₆₋₁₈)gruppe, einer Aryloxy(C₆₋₁₈)gruppe, einer Alkoxy(C₁₋₉)gruppe, einer Halogenogruppe, einer Carboxygruppe, einer Cyanogruppe und einer substituierten oder unsubstituierten Aminogruppe ausgewählt sind;
- eine Furanylgruppe;
- eine Alkyl(C₁₋₉)thiogruppe;
- eine Thienylgruppe;
- eine Phenylcarbonylgruppe;
- eine Trifluoralkylgruppe;
- eine Diaryl(C₆₋₁₈)alkyl(C₁₋₉)gruppe;
A bedeutet eine Aryl(C₆₋₁₈)gruppe oder eine 5- bis 12-gliedrige heterocyclische Gruppe, die gesättigt oder ungesättigt, substituiert oder unsubstituiert ist.

2. Verwendung nach Anspruch 1, wobei die Verbindung der Formel (I) so vorliegt, dass bedeuten:
· R₁, R₄, R₅ und R₈ ein Wasserstoffatom;
· R₂ und R₇ unabhängig voneinander ein Wasserstoffatom; eine Halogenogruppe; eine Alkyl(C₁₋₉)gruppe; eine Alkoxy(C₁₋₉)gruppe; eine substituierte oder unsubstituierte Aminogruppe;
· R₃ und R₆ unabhängig voneinander ein Wasserstoffatom; eine Halogenogruppe; eine Alkyl(C₁₋₉)gruppe; eine substituierte oder unsubstituierte Aminogruppe;
· X eine direkte Bindung oder ein Schwefelatom oder ein Sauerstoffatom oder eine Gruppe SO₂;
· G eine zweiwertige Gruppe der Formel G₁, G₂, G₃, G₄, G₅ oder G₆.

3. Verwendung nach Anspruch 1 oder 2, wobei die Verbindung der Formel (I) so vorliegt, dass G eine Gruppe G₁ bedeutet; X ein Sauerstoffatom ist; R₁, R₃, R₄, R₅, R₆ und R₈ ein Wasserstoffatom bedeuten; R₂ und R₇ unabhängig voneinander eine substituierte oder unsubstituierte Aminogruppe bedeuten; R₉ eine substituierte oder unsubstituierte Arylgruppe, eine substituierte oder unsubstituierte Aminogruppe bedeutet; A ein substituierter oder unsubstituierter Benzolring ist.

4. Verwendung nach Anspruch 1 oder 2, wobei die Verbindung der Formel (I) so vorliegt, dass G eine Gruppe G₂ bedeutet; X ein Sauerstoffatom ist; R₁, R₃, R₄, R₅, R₆, R₈, R₁₀ und R₁₁ ein Wasserstoffatom bedeuten; R₂ und R₇ unabhängig voneinander eine substituierte oder unsubstituierte Aminogruppe bedeuten; A ein substituierter oder unsubstituierter Benzolring ist.

5. Verwendung nach Anspruch 1 oder 2, wobei die Verbindung der Formel (I) so vorliegt, dass G eine Gruppe G₃ bedeutet; R₁, R₃, R₄, R₅, R₆, R₈, R₁₀ und R₁₁ ein Wasserstoffatom bedeuten; R₂ oder R₇ unabhängig voneinander ein Wasserstoffatom, eine substituierte oder unsubstituierte Aminogruppe bedeuten; R₉ ein Wasserstoffatom, eine substituierte oder unsubstituierte Alkylgruppe, eine Alkoxygruppe bedeutet; X eine direkte Bindung ist; W₁ ein Schwefelatom, ein Kohlenstoffatom bedeutet, das mit einem Wasserstoffatom, einer C₁₋₄-Alkylgruppe, einer Phenylgruppe, einer Aminogruppe mit zwei C₁₋₄-Alkylgruppen substituiert ist, wobei die beiden Gruppen gegebenenfalls mit dem Stickstoffatom, an das sie gebunden sind, einen gesättigten 5-gliedrigen Heterocyclus bilden; Y₁ ein Kohlenstoffatom, das mit einem Wasserstoffatom, einer C₁₋₄-Alkylgruppe, einer Phenylgruppe, einer Aminogruppe mit zwei C₁₋₄-Alkylgruppen substituiert ist, wobei die beiden Gruppen gegebenenfalls mit dem Stickstoffatom, an das sie gebunden sind, einen gesättigten 5-gliedrigen Heterocyclus bilden, ein Stickstoffatom, ein Stickstoffatom bedeutet, das mit einem Wasserstoffatom, einer C₁₋₂-Alkylgruppe, einer Phenylgruppe, einer Phenylalkylgruppe, deren Alkylgruppe 1 bis 4 Kohlenstoffatome aufweist, substituiert ist; Z₁ ein Stickstoffatom, eine Gruppe NH, ein Stickstoffatom bedeutet, das mit einem Wasserstoffatom, einer C₁₋₂-Alkylgruppe, einer Phenylgruppe, einer Phenylalkylgruppe, deren Alkylgruppe 1 bis 4 Kohlenstoffatome aufweist, substituiert ist.

6. Verwendung nach Anspruch 1 oder 2, wobei die Verbindung der Formel (I) so vorliegt, dass G eine Gruppe G₄ bedeutet; R₁, R₄, R₅, R₈ und R₁₁ ein Wasserstoffatom bedeuten; R₂ oder R₇ unabhängig voneinander ein Wasserstoffatom, ein Halogenatom, eine Alkoxygruppe, eine substituierte oder unsubstituierte Aminogruppe bedeuten; R₃ und R₆ unabhängig voneinander ein Wasserstoffatom, ein Halogenatom, eine substituierte oder unsubstituierte Aminogruppe bedeuten; R₉ ein Wasserstoffatom, eine substituierte oder unsubstituierte Alkylgruppe, eine Alkoxygruppe, eine Arylgruppe, eine substituierte oder unsubstituierte Aminogruppe bedeutet; R₁₀ ein Wasserstoffatom oder eine Alkylgruppe bedeutet; A ein substituierter oder unsubstituierter Benzolring oder ein substituierter oder unsubstituierter Pyridinring ist; X ein Sauerstoffatom, ein Schwefelatom, eine direkte Bindung oder eine Gruppe SO₂ ist; W₂ ein Kohlenstoffatom oder ein Stickstoffatom bedeutet; Y₂ ein Kohlenstoffatom, ein Stickstoffatom bedeutet; Z₂ eine Gruppe CH, ein Kohlenstoffatom, das mit einem Wasserstoffatom, einer C₁₋₄-Alkylgruppe, einer Phenylgruppe, einer Aminogruppe mit zwei C₁₋₄-Alkylgruppen substituiert ist, wobei die beiden Gruppen gegebenenfalls mit dem Stickstoffatom, an das sie gebunden sind, einen gesättigten 5-gliedrigen Heterocyclus bilden, ein Stickstoffatom, eine Gruppe NH, ein Stickstoffatom bedeutet, das mit einem Wasserstoffatom, einer C₁₋₂-Alkylgruppe, einer Phenylgruppe, einer Phenylalkylgruppe, deren Alkylgruppe 1 bis 4 Kohlenstoffatome aufweist, substituiert ist.

7. Verwendung nach Anspruch 1 oder 2, wobei die Verbindung der Formel (I) so vorliegt, dass G eine Gruppe G₅ bedeutet; X ein Sauerstoffatom oder eine direkte Bindung ist; R₁, R₄, R₅, R₈ und R₁₀ ein Wasserstoffatom bedeuten; R₂ und R₇ unabhängig voneinander eine Alkylgruppe, eine Alkoxygruppe, eine substituierte oder unsubstituierte Aminogruppe bedeuten; R₃ und R₆ unabhängig voneinander ein Wasserstoffatom, eine Alkylgruppe, eine substituierte oder unsubstituierte Aminogruppe bedeuten; R₉ ein Wasserstoffatom, eine substituierte oder unsubstituierte Aminogruppe bedeutet; R₁₂ eine substituierte oder unsubstituierte Aminogruppe, eine substituierte oder unsubstituierte Arylgruppe, eine Thienylgruppe, eine Furanylgruppe bedeutet.

8. Verwendung nach Anspruch 1 oder 2, wobei die Verbindung der Formel (I) so vorliegt, dass G eine Gruppe G₆ bedeutet; X eine direkte Bindung oder ein Sauerstoffatom ist; R₁, R₃, R₄, R₅, R₆, R₈ und R₁₀ ein Wasserstoffatom bedeuten; R₂, R₉ und R₇ unabhängig voneinander eine substituierte oder unsubstituierte Aminogruppe bedeuten; R₁₂ eine Alkylgruppe ist.

9. Verwendung nach einem der vorhergehenden Ansprüche, wobei die Verbindung der Formel (I) unter den folgenden Verbindungen ausgewählt ist:

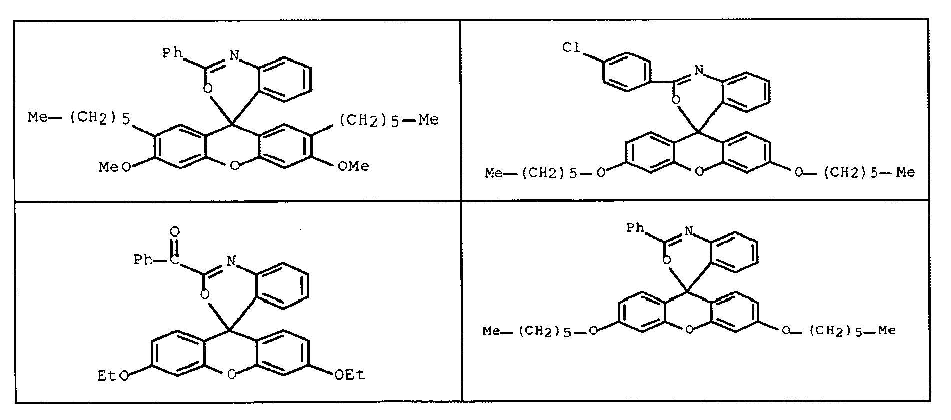

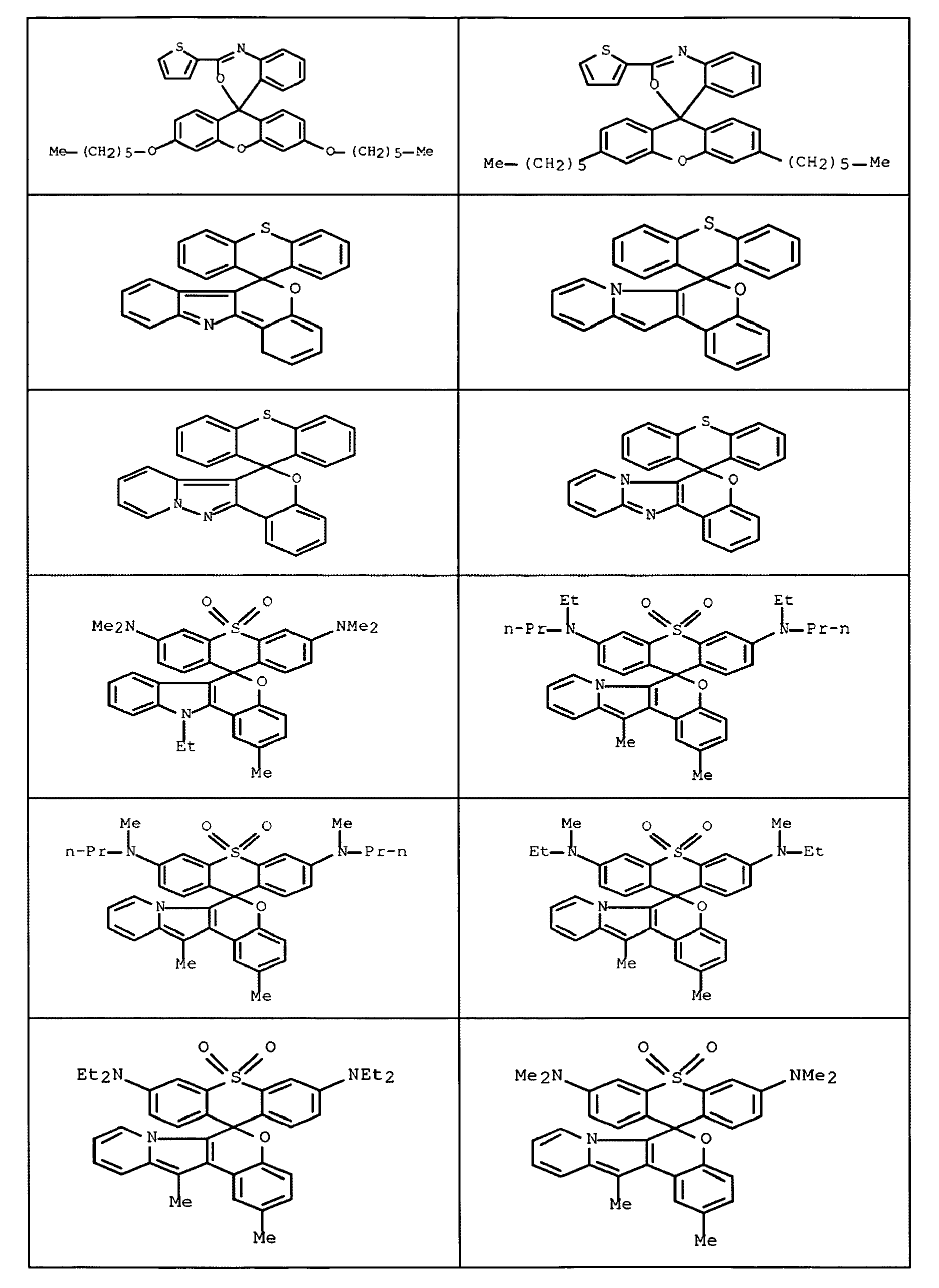

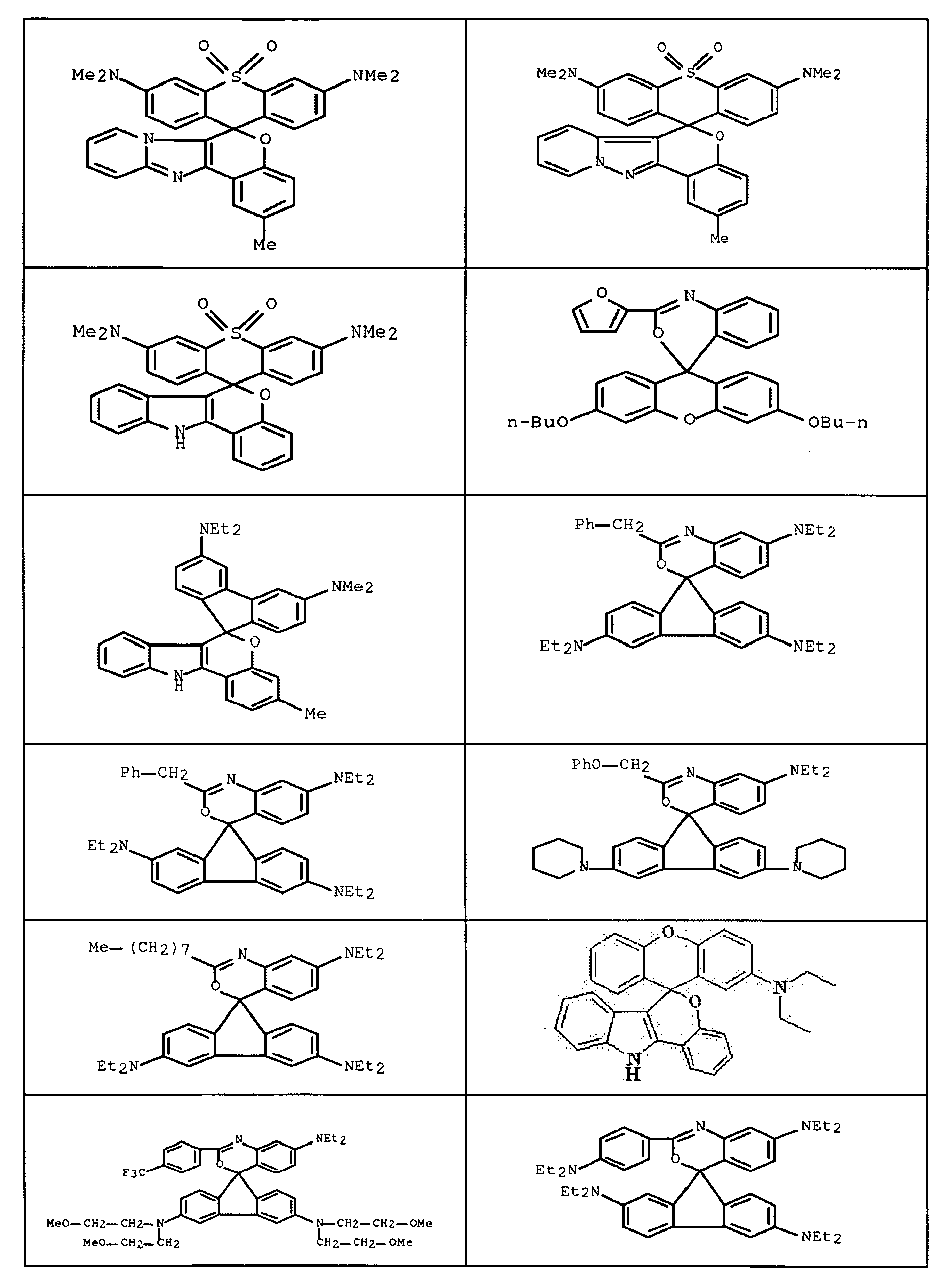

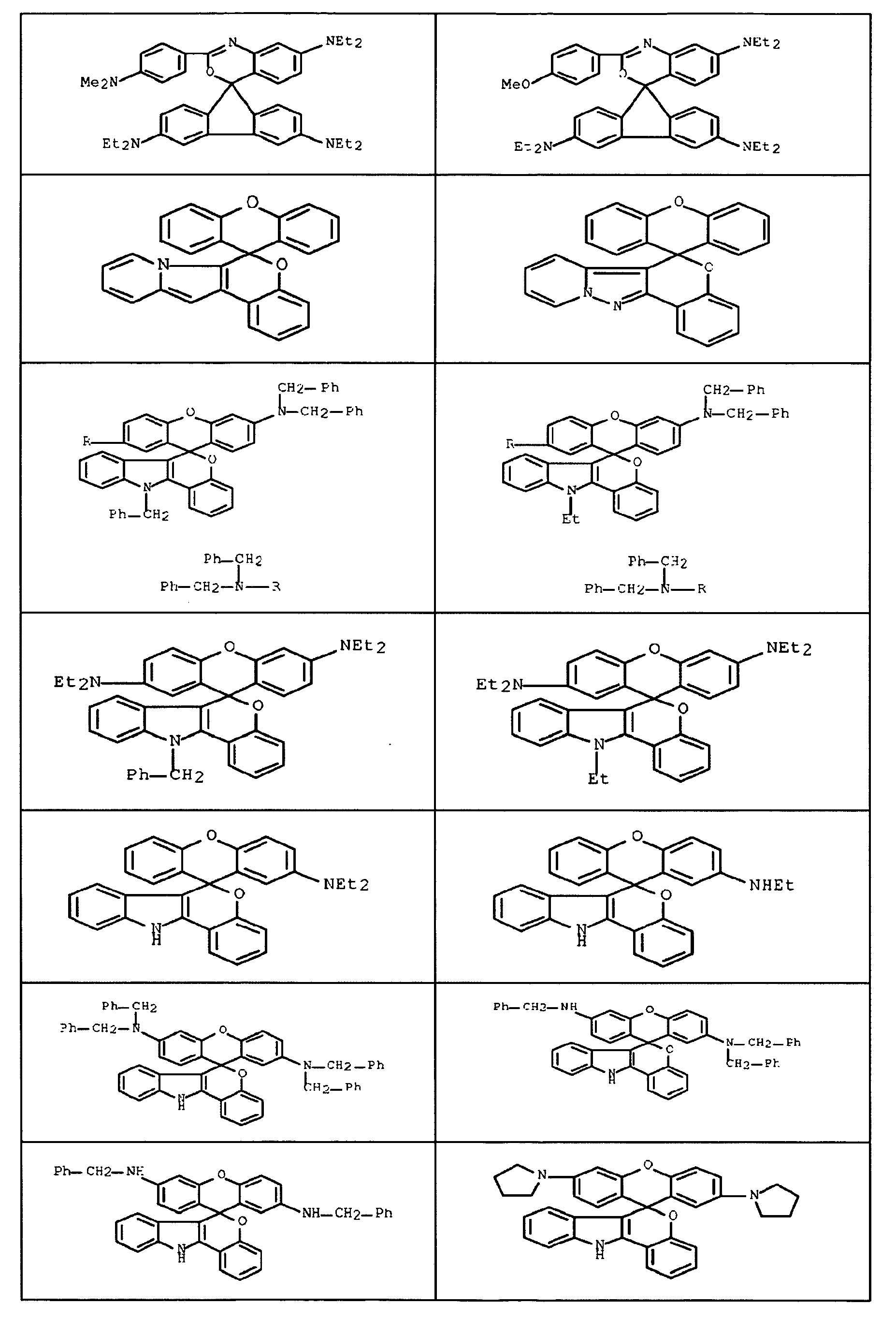

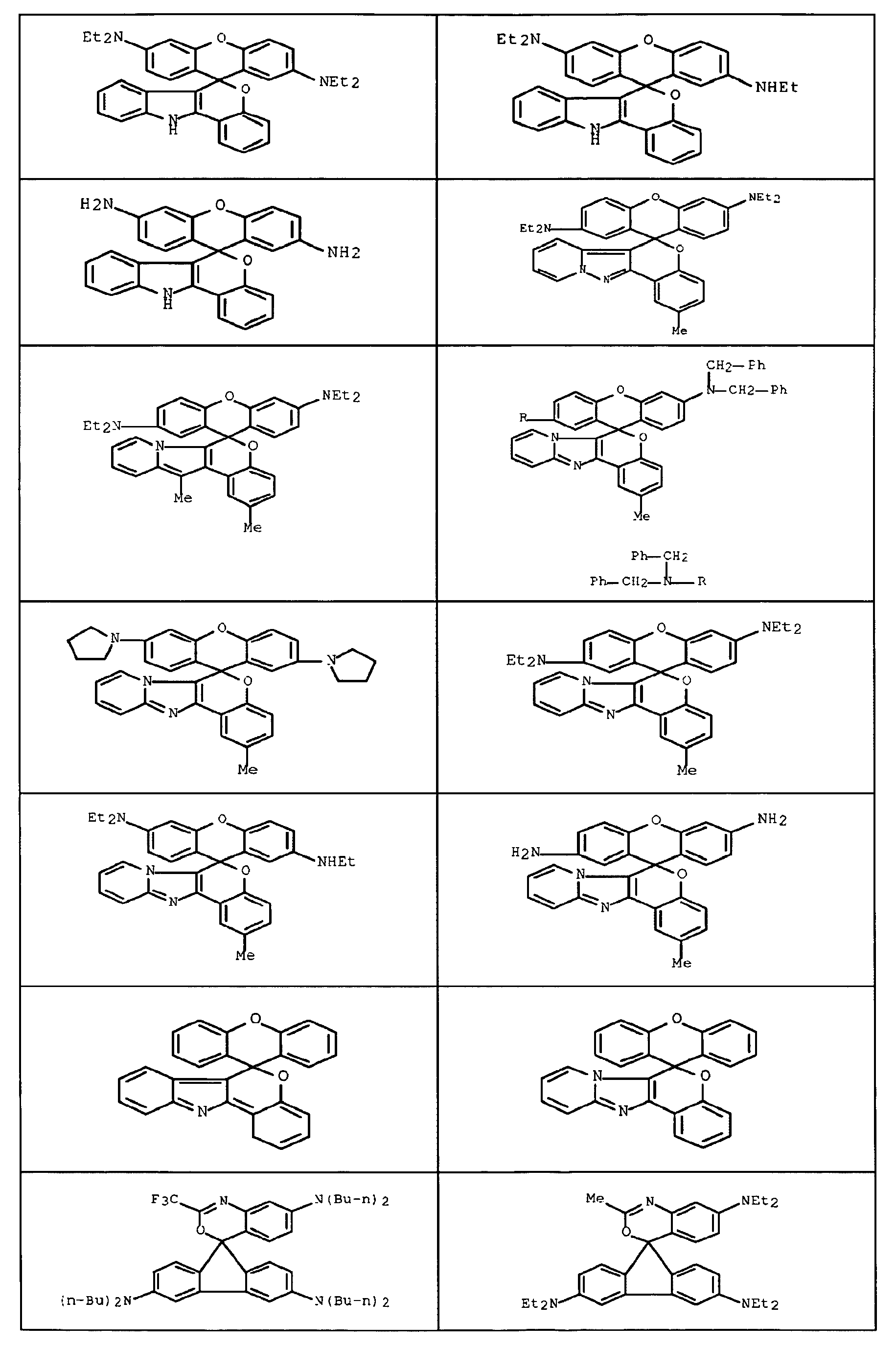

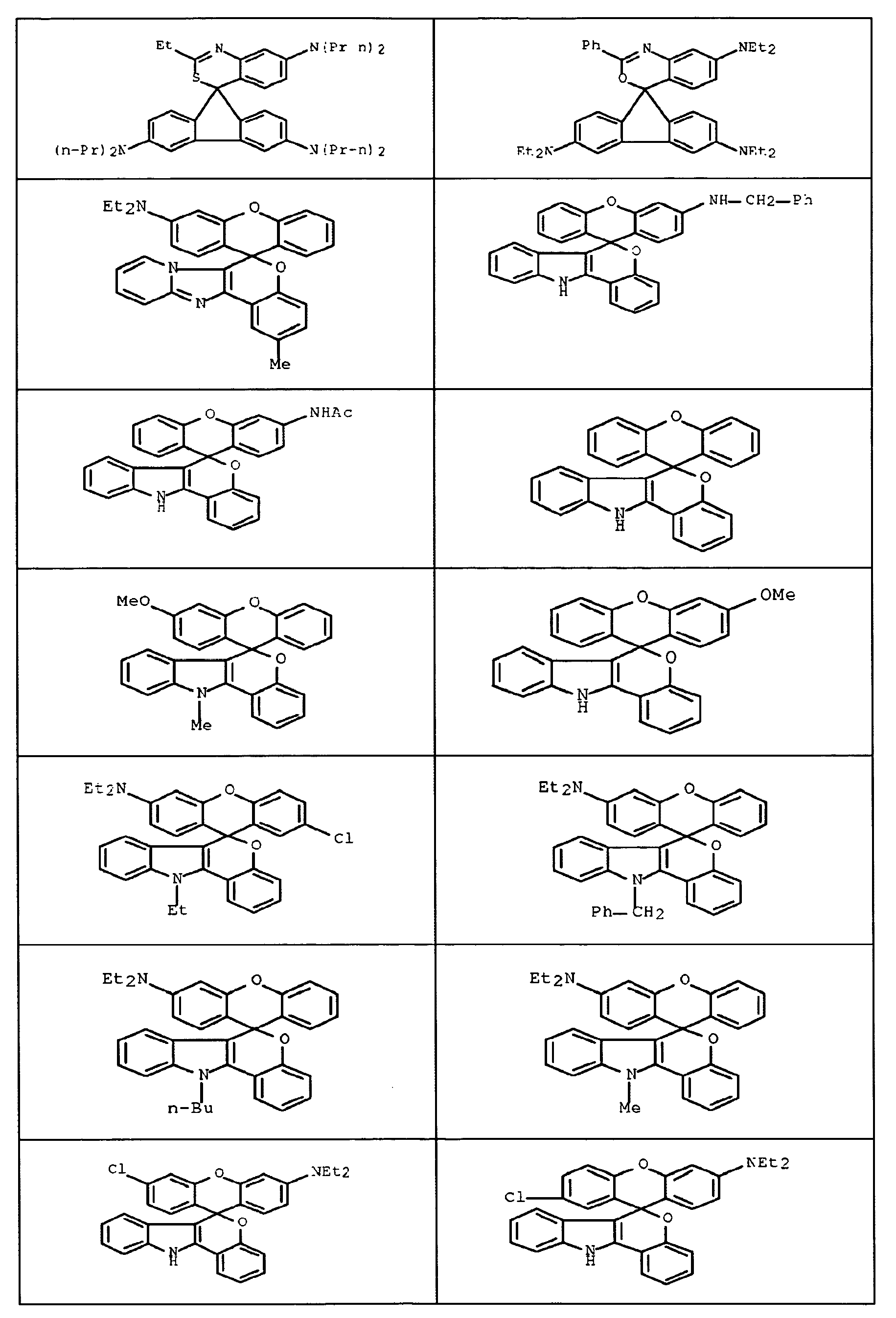

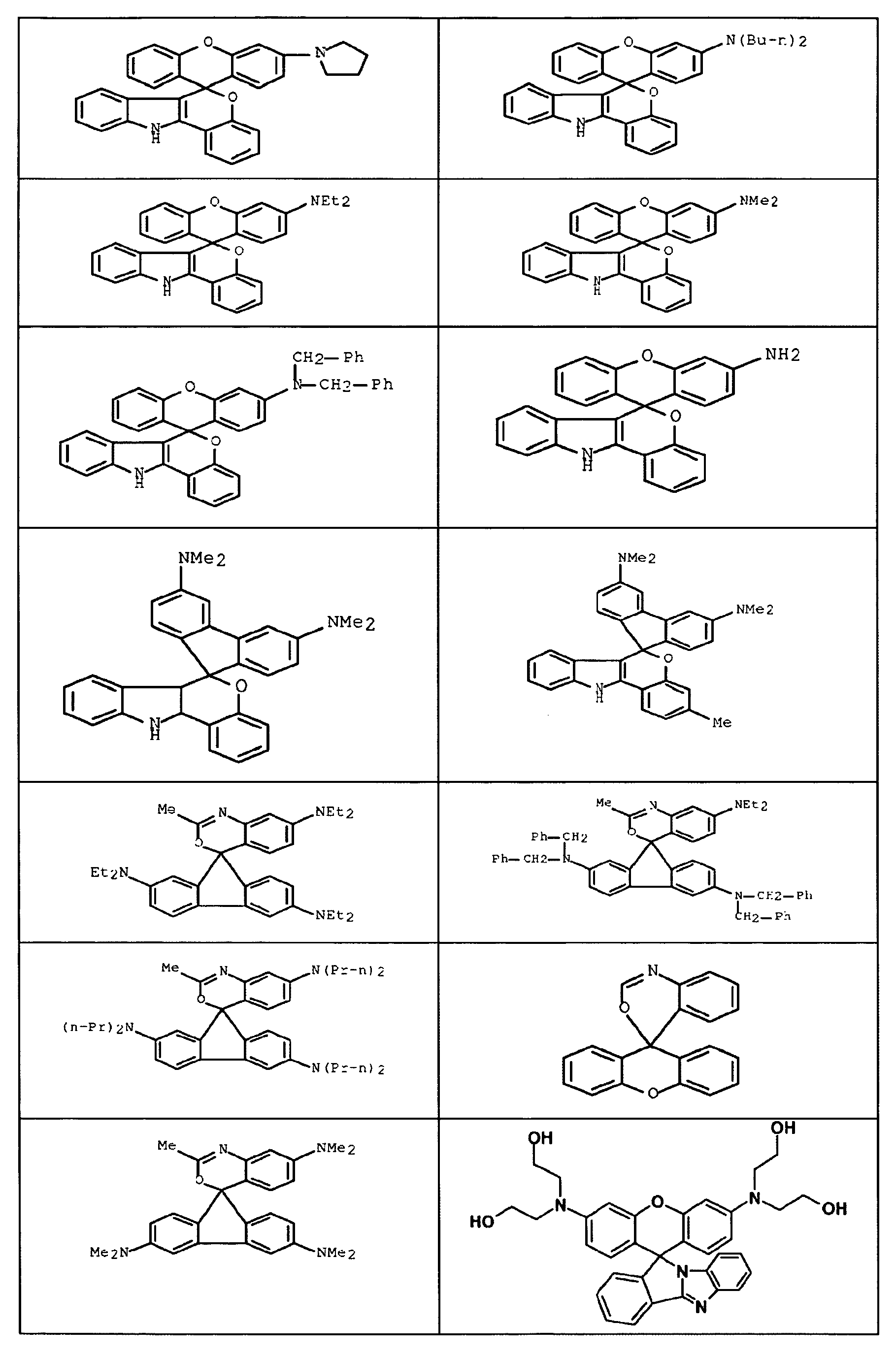

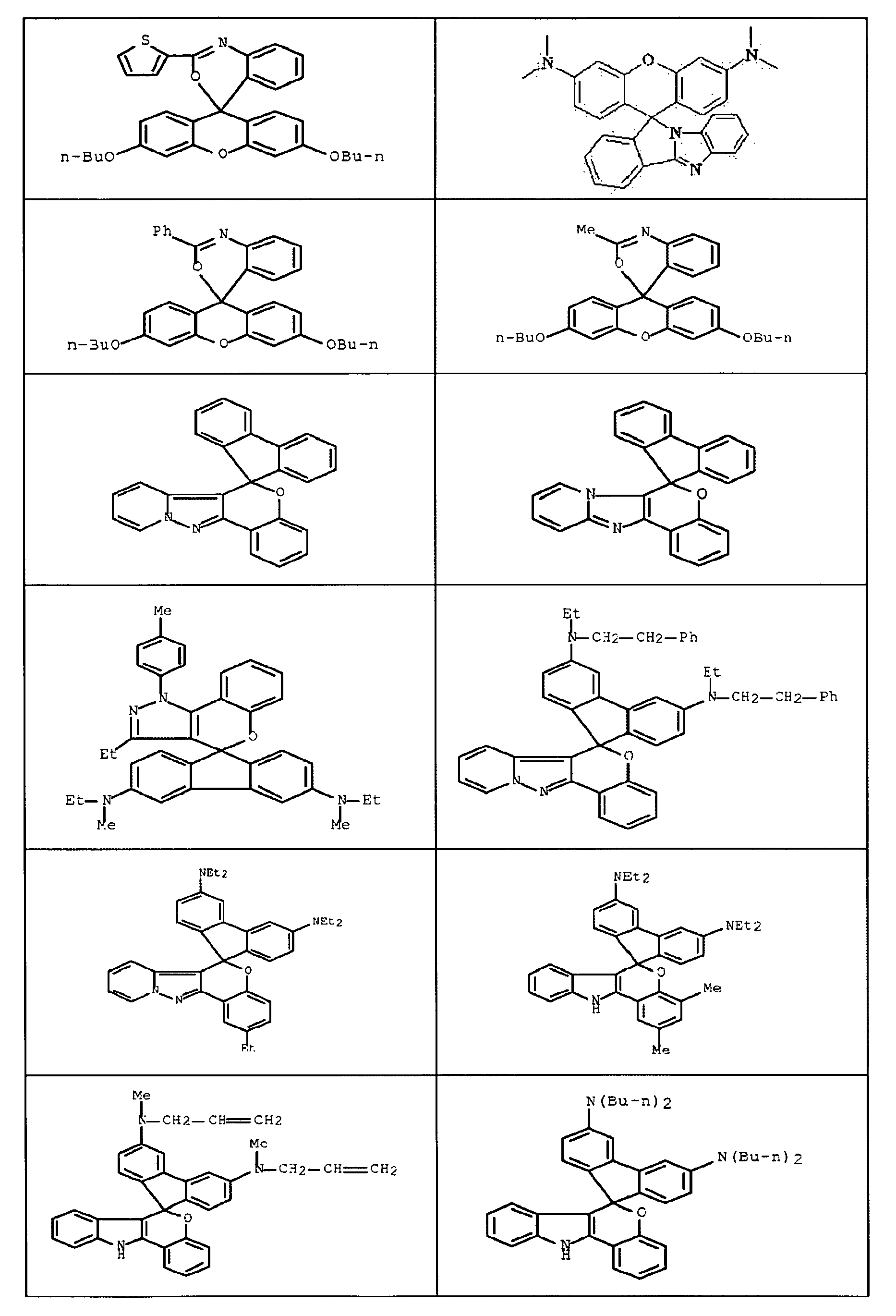

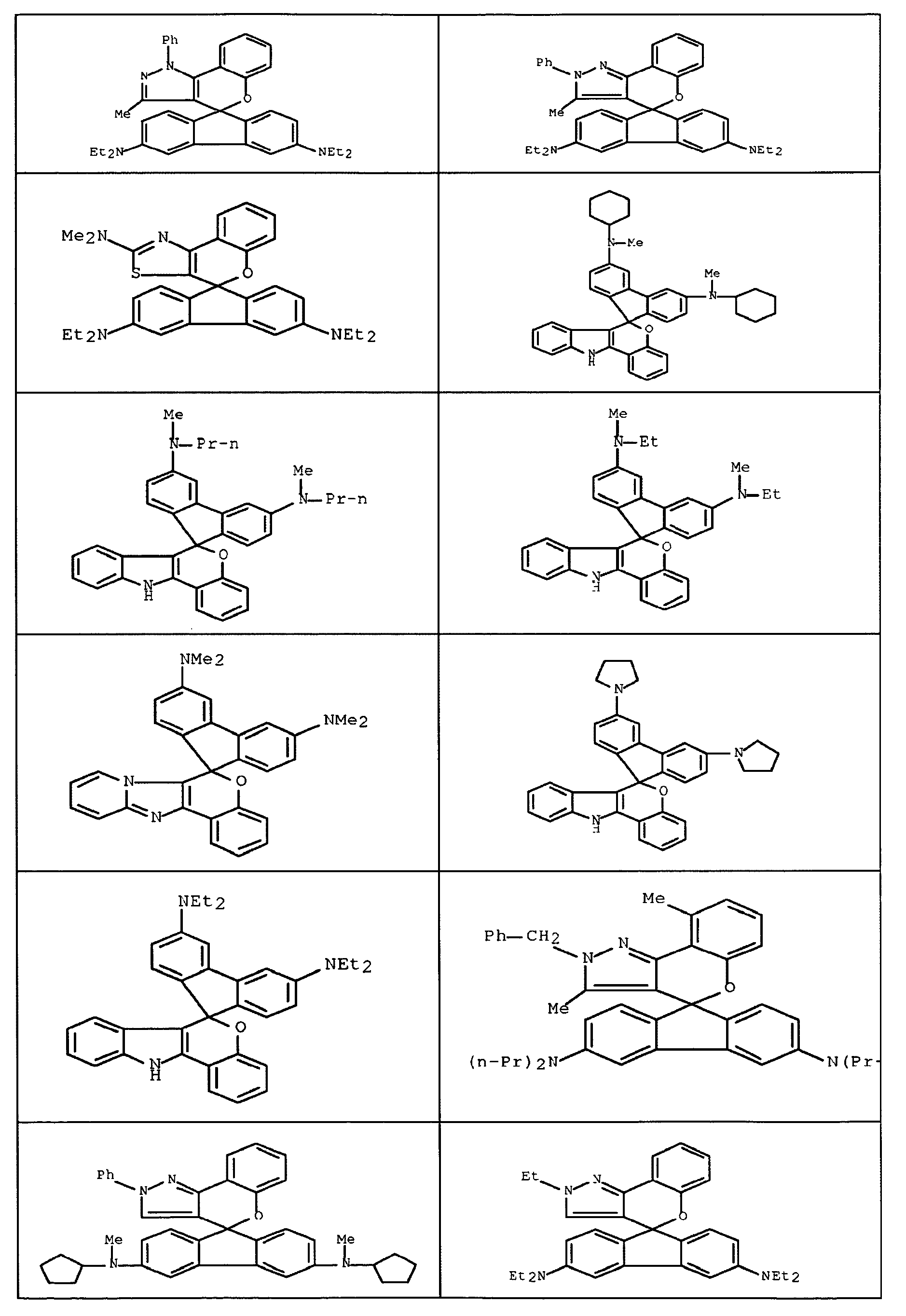

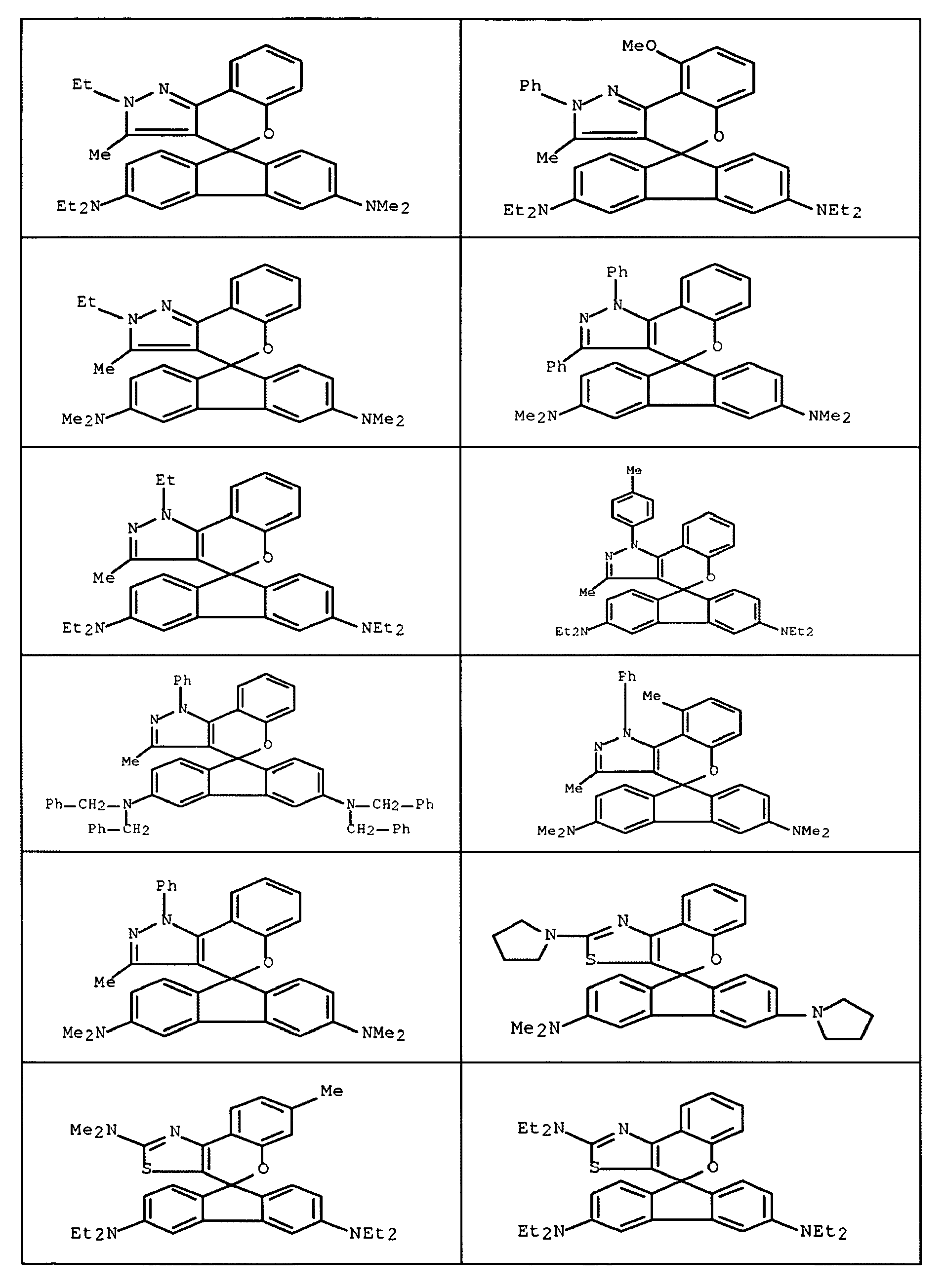

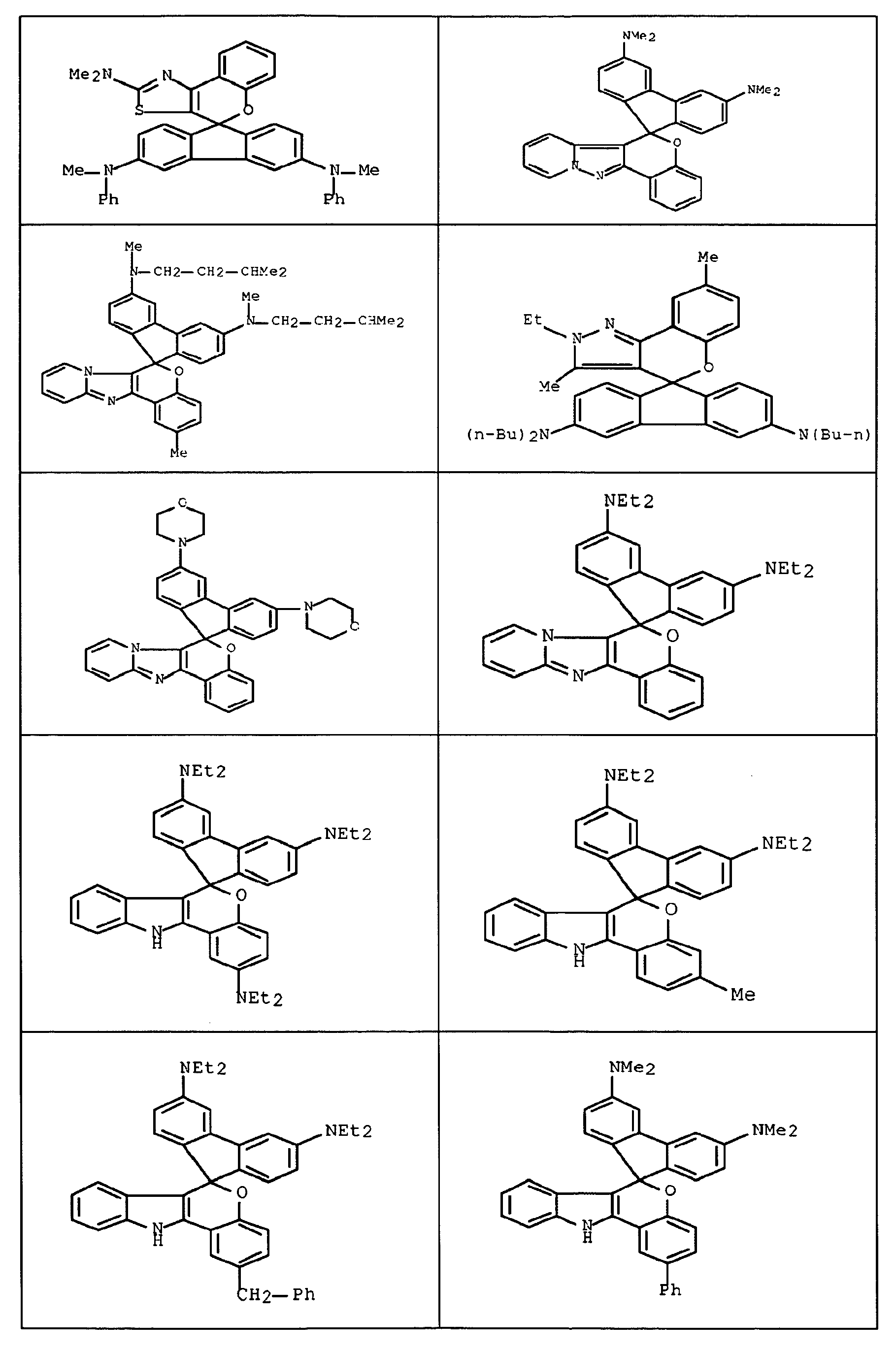

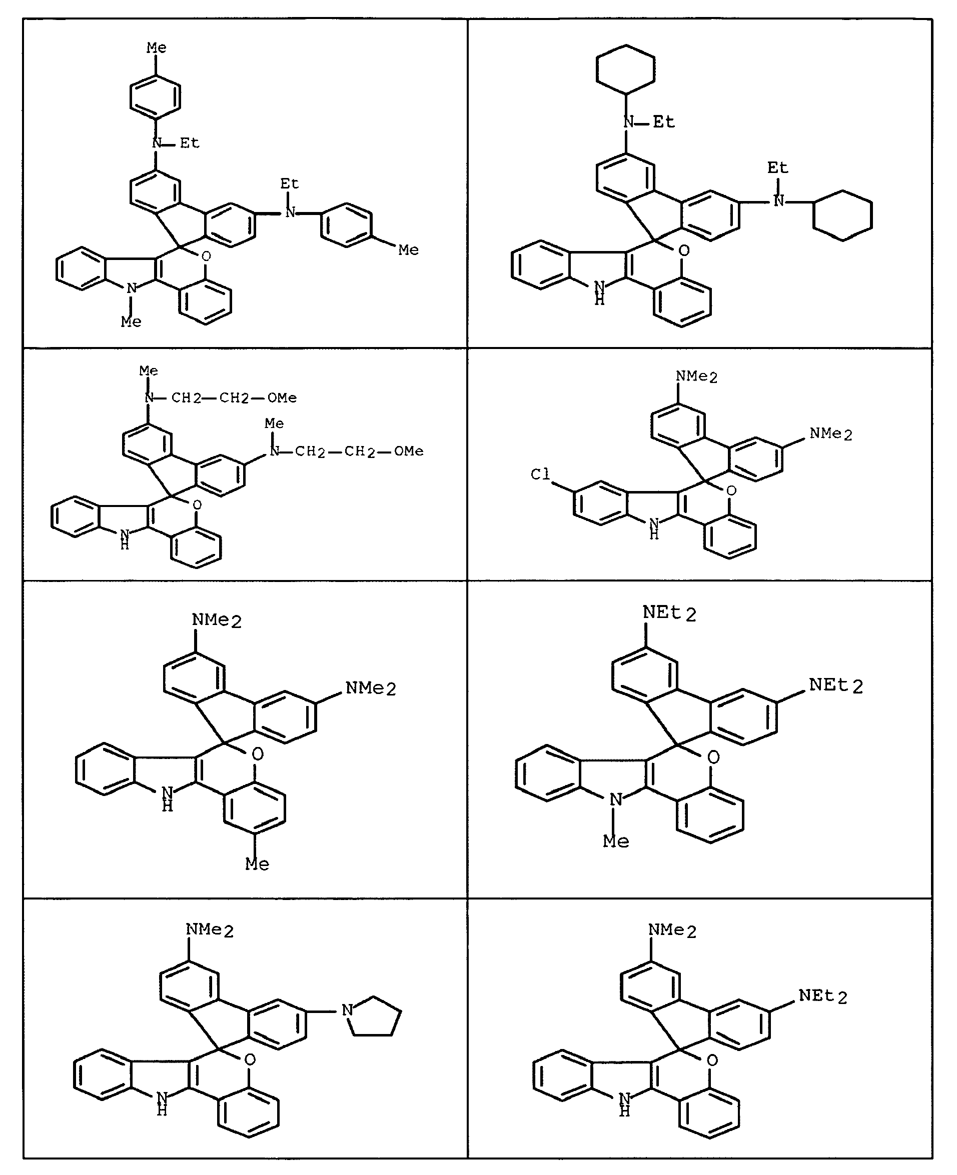

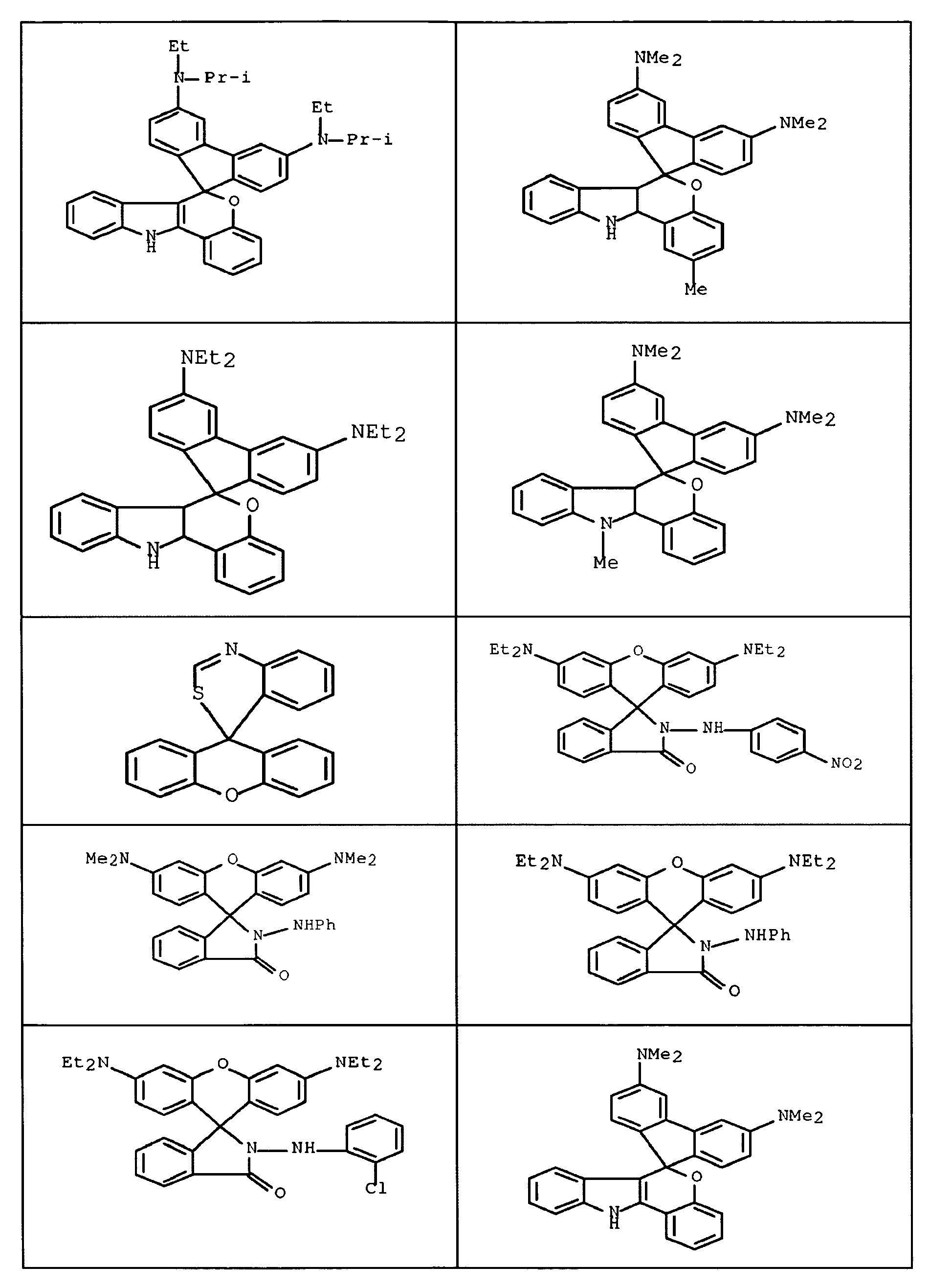

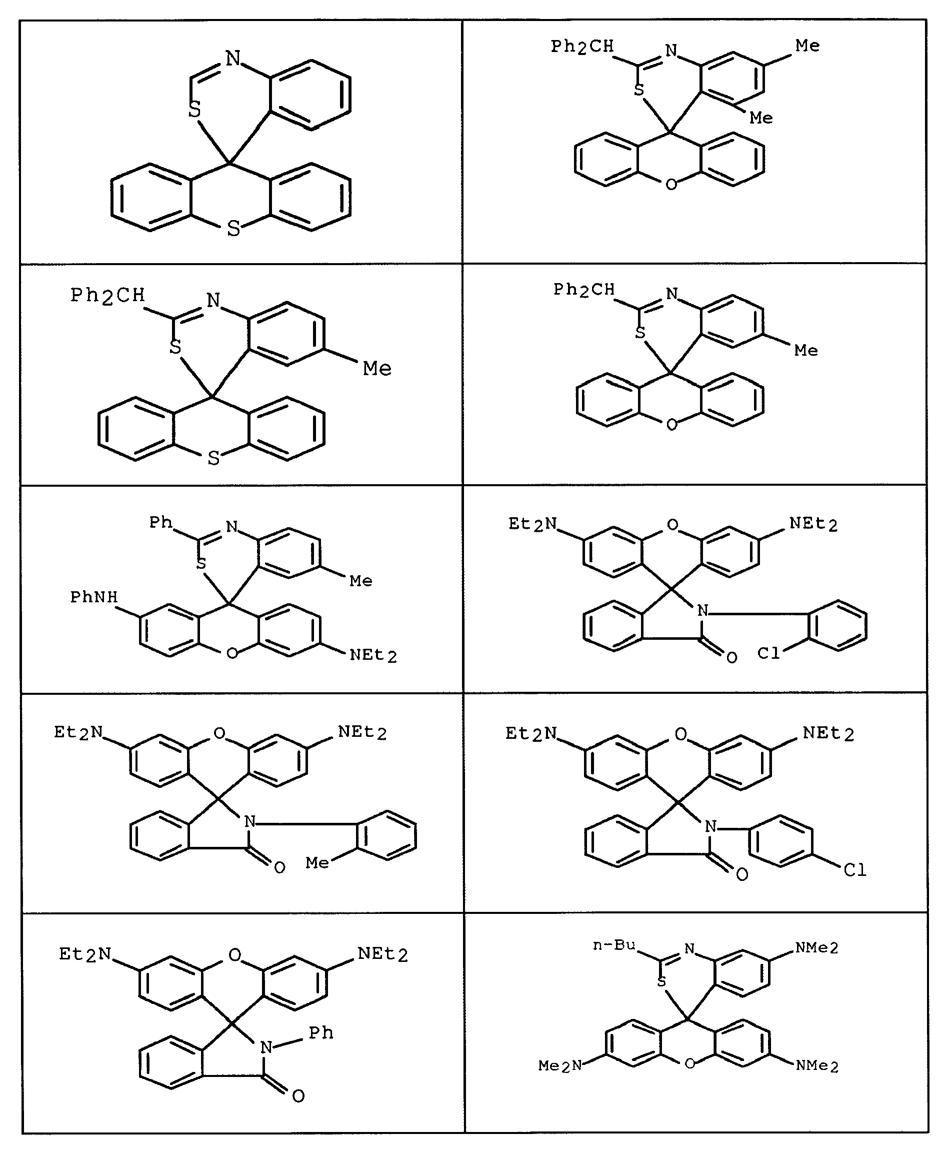

10. Verwendung nach Anspruch 9, wobei die Verbindung der Formel (I) unter den folgenden Verbindungen ausgewählt ist:

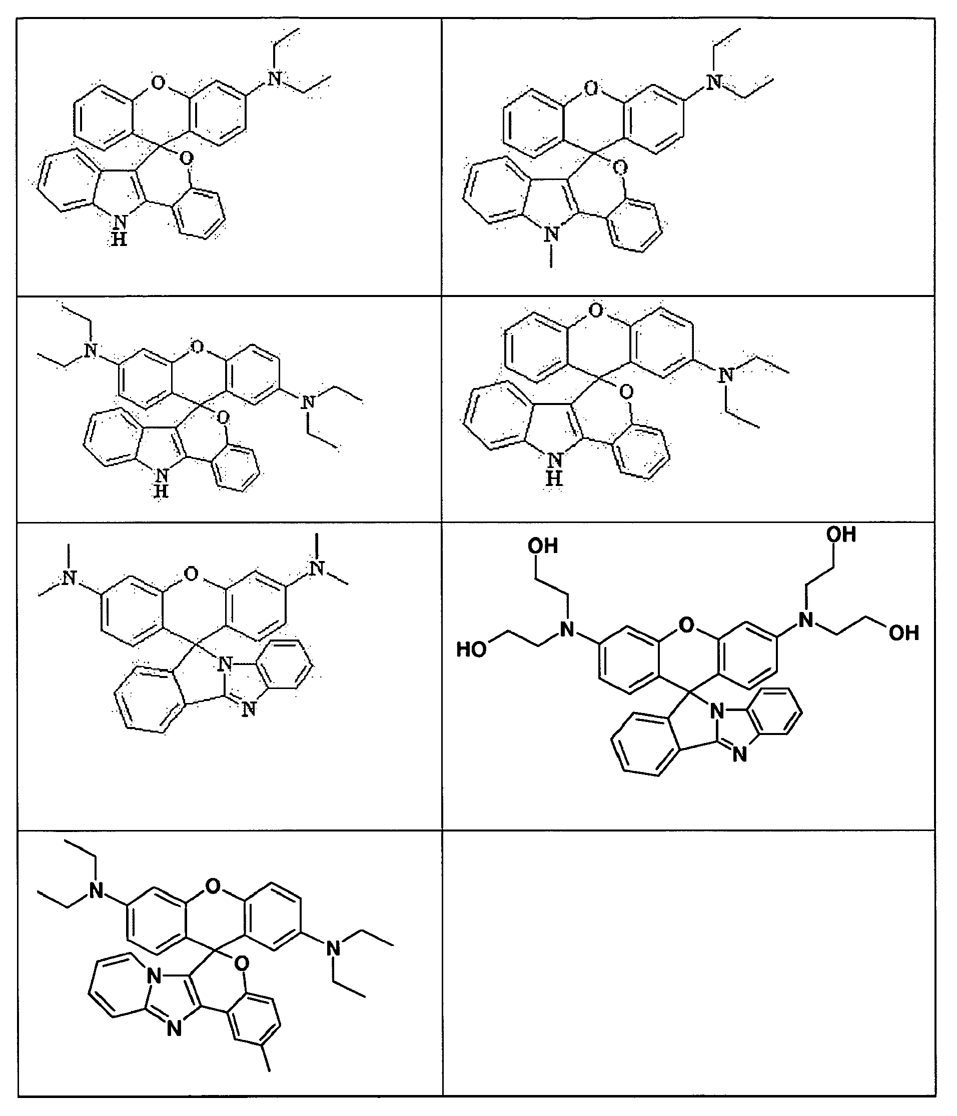

11. Verwendung nach einem der vorhergehenden Ansprüche, wobei die Verbindungen, die unter den Verbindungen der Formel (I), den Farbstoffen, die den Verbindungen der Formel (I) entsprechen, bei denen der Ring H geöffnet ist, und ihren Additionssalzen ausgewählt ist, 0,001 bis 10 Gew.-%, bezogen auf das Gesamtgewicht der Zusammensetzung, ausmachen.

12. Verwendung nach einem der vorhergehenden Ansprüche, wobei das zum Färben geeignete Medium aus Wasser oder mindestens einem organischen Lösungsmittel oder einem Gemisch aus Wasser und mindestens einem organischen Lösungsmittel besteht.

13. Verwendung nach Anspruch 12, wobei das zum Färben geeignete Medium aus Wasser oder einem Gemisch aus Wasser und mindestens einem organischen Lösungsmittel besteht und der pH-Wert im Bereich von 3 bis 12 liegt.

14. Verfahren zur Behandlung von Keratinfasern, bei dem die folgenden Schritte durchgeführt werden:
a) Aufbringen einer Farbmittelzusammensetzung, wie sie in einem der Ansprüche 1 bis 13 definiert ist, während einer ausreichenden Einwirkzeit, wobei der pH-Wert in Abhängigkeit von der gewünschten Färbung mit Hilfe eines Ansäuerungsmittel oder einer Alkalisierungsmittels eingestellt wird;
b) gegebenenfalls Modifikation der Färbung der Keratinfasern mit Hilfe eines Ansäuerungsmittel oder einer Alkalisierungsmittels, das auf die Keratinfasern aufgebracht wird.

15. Verfahren nach Anspruch 14, bei dem das Ansäuerungsmittel oder Alkalisierungsmittel in dem ersten Schritt vor dem Auftragen auf die Keratinfasern mit der Farbmittelzusammensetzung vermischt wird.

16. Verfahren nach Anspruch 14, bei dem das Ansäuerungsmittel oder Alkalisierungsmittel in dem ersten Schritt vor oder nach der Farbmittelzusammensetzung auf die Keratinfasern aufgetragen wird.

17. Verfahren nach einem der Ansprüche 14 bis 16, bei dem das Ansäuerungsmittel unter den anorganischen Säuren, wie Salzsäure, Salpetersäure oder Schwefelsäure, oder den organischen Säuren ausgewählt ist, wie den Verbindungen, die mindestens eine Carboxyfunktion, Sulfonsäurefunktion, Phosphonsäurefunktion oder Phosphorsäurefunktion aufweisen.

18. Verfahren nach einem der Ansprüche 14 bis 17, bei dem das Alkalisierungsmittel ausgewählt ist unter:
- basischen Aminosäuren;
- Carbonaten oder Bicarbonaten von Alkalimetallen oder Erdalkalimetallen;
- Silicaten oder Metasilicaten;
- Verbindungen der folgenden Formel (II):
X(OH)ₙ (II)
worin bedeuten:
· X ein Kaliumion, Lithiumion, Natriumion oder Ammoniumion N+R₁₅R₁₆R₁₇R₁₈, wobei die Gruppen R₁₅, R₁₆, R₁₇ und R₁₈, die gleich oder verschieden sind, eine C₂₋₄-Alkylgruppe bedeuten, wenn n 1 ist;
· X ein Magnesiumion oder Calciumion, wenn n 2 ist;
- Verbindungen der folgenden Formel (III): 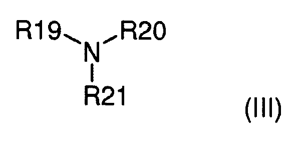 worin bedeuten:
· R₁₉ ein Wasserstoffatom; eine Alkyl(C₁₋₆)gruppe; eine Monohydroxyalkyl(C₁₋₆)gruppe; eine Polyhydroxyalkyl(C₂₋₆)gruppe;
· R₂₀ und R₂₁, die gleich oder verschieden sind, ein Wasserstoffatom; eine Alkyl(C₁₋₆)gruppe; eine Monohydroxyalkyl(C₁₋₆)-gruppe; eine Polyhydroxyalkyl(C₂₋₆)gruppe;
- Verbindungen der folgenden Formel (IV): 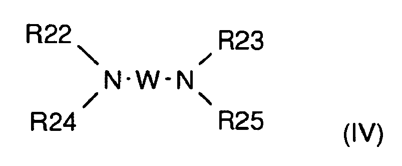 worin bedeuten:
· W einen Propylenrest, der gegebenenfalls mit einer Hydroxygruppe oder einer Alkyl(C₁₋₄)gruppe substituiert ist;
· R₂₂, R₂₃, R₂₄ und R₂₅, die gleich oder voneinander verschieden sind, ein Wasserstoffatom; eine Alkyl(C₁₋₄)gruppe; eine Hydroxyalkyl(C₁₋₄)gruppe.

19. Vorrichtung mit mehreren Abteilungen, die in einer ersten Abteilung eine Zusammensetzung, wie sie in einem der Ansprüche 1 bis 13 definiert ist, und in einer zweiten Abteilung ein Ansäuerungsmittel oder ein Alkalisierungsmittel enthält.

20. Vorrichtung nach Anspruch 19, die in der zweiten Abteilung ein Ansäuerungsmittel und in einer dritten Abteilung ein Alkalisierungsmittel enthält.

21. Zusammensetzung, wie sie in einem der Ansprüche 1 bis 13 definiert ist, die ferner mindestens einen grenzflächenaktiven Stoff und mindestens ein Polymer enthält.

22. Zusammensetzung nach Anspruch 21, wobei der oder die grenzflächenaktiven Stoffe unter den anionischen, kationischen, nichtionischen, amphoteren oder zwitterionischen grenzflächenaktiven Stoffen oder deren Gemischen ausgewählt sind.

23. Zusammensetzung nach Anspruch 21 oder 22, wobei das oder die Polymere assoziative oder nicht assoziative, verdickende Polymere sind.
